# EUROPEAN PATENT APPLICATION

(11) **EP 2 468 856 A1**
(43) Date of publication of application: **27.06.2012**
(21) Application number: 10196890.7
(22) Date of filing: 23.12.2010
(51) Int. Cl.: C12N 9/36, C12N 15/62, A61K 38/00

(54) **Antimicrobial Agents**

(71) Applicant: Lysando Aktiengesellschaft, 9497 Triesenberg (LI); Katholieke Universiteit Leuven, K.U. Leuven R&D, 3000 Leuven (BE)
(72) Inventor: Briers, Yves, 5032 Rohr AG (CH); Lavigne, Rob, 2170 Merksem (BE); Walmagh, Maarten, 3540 Herk-de-Stad (BE); Miller, Stefan, 93055 Regensburg (DE)
(74) Representative: Dehmel, Albrecht

(57) **Abstract**

The present invention relates to a fusion protein composed of an enzyme having the activity of degrading the cell wall of Gram-negative bacteria and/or Gram-positive bacteria and at least two peptide stretches fused to the enzyme at the N- and/or C-terminus, wherein the peptide stretches are distinct and selected from the group of synthetic amphiphatic peptide, synthetic cationic peptide, synthetic polycationic peptide, synthetic hydrophobic peptide, or naturally occurring antimicrobial peptide, like sushi peptide and defensin. Moreover, the present invention relates to nucleic acid molecules encoding said fusion protein, vectors comprising said nucleic acid molecules and host cells comprising either said nucleic acid molecules or said vectors. In addition, the present invention relates to said fusion protein for use as a medicament, diagnostic means, cosmetic substance, a disinfectant or a food additive. The present invention also relates to the treatment or prevention of Gram-postive and/or Gram-negative bacterial contamination of foodstuff, of food processing equipment, of food processing plants, of surfaces coming into contact with foodstuff, of medical devices, of surfaces in hospitals and surgeries.

SEQUENCE LISTING

<110>  Lysando Holding AG and Katholieke Universiteit Leuven

<120>  ANTIMICROBIAL AGENTS

<130>  LYS-007 EP

<140>  not yet known
<141>  2010-12-23

<160>  135

<170>  PatentIn version 3.3

<210>  1
<211>  339
<212>  PRT
<213>  Streptococcus pneumoniae Phage Cpl-1

<400>  1

Met Val Lys Lys Asn Asp Leu Phe Val Asp Val Ser Ser His Asn Gly
1               5                   10                  15

Tyr Asp Ile Thr Gly Ile Leu Glu Gln Met Gly Thr Thr Asn Thr Ile
            20                  25                  30

Ile Lys Ile Ser Glu Ser Thr Thr Tyr Leu Asn Pro Cys Leu Ser Ala
        35                  40                  45

Gln Val Glu Gln Ser Asn Pro Ile Gly Phe Tyr His Phe Ala Arg Phe
    50                  55                  60

Gly Gly Asp Val Ala Glu Ala Glu Arg Glu Ala Gln Phe Phe Leu Asp
65                  70                  75                  80

Asn Val Pro Met Gln Val Lys Tyr Leu Val Leu Asp Tyr Glu Asp Asp
                85                  90                  95

Pro Ser Gly Asp Ala Gln Ala Asn Thr Asn Ala Cys Leu Arg Phe Met
            100                 105                 110

Gln Met Ile Ala Asp Ala Gly Tyr Lys Pro Ile Tyr Tyr Ser Tyr Lys
        115                 120                 125

Pro Phe Thr His Asp Asn Val Asp Tyr Gln Gln Ile Leu Ala Gln Phe
    130                 135                 140

Pro Asn Ser Leu Trp Ile Ala Gly Tyr Gly Leu Asn Asp Gly Thr Ala
145                 150                 155                 160

Asn Phe Glu Tyr Phe Pro Ser Met Asp Gly Ile Arg Trp Trp Gln Tyr
                165                 170                 175

Ser Ser Asn Pro Phe Asp Lys Asn Ile Val Leu Leu Asp Asp Glu Glu
            180                 185                 190

Asp Asp Lys Pro Lys Thr Ala Gly Thr Trp Lys Gln Asp Ser Lys Gly
        195                 200                 205

Trp Trp Phe Arg Arg Asn Asn Gly Ser Phe Pro Tyr Asn Lys Trp Glu
    210                 215                 220

Lys Ile Gly Gly Val Trp Tyr Tyr Phe Asp Ser Lys Gly Tyr Cys Leu
225                 230                 235                 240

Thr Ser Glu Trp Leu Lys Asp Asn Glu Lys Trp Tyr Tyr Leu Lys Asp
                245                 250                 255

Asn Gly Ala Met Ala Thr Gly Trp Val Leu Val Gly Ser Glu Trp Tyr
            260                 265                 270

Tyr Met Asp Asp Ser Gly Ala Met Val Thr Gly Trp Val Lys Tyr Lys
        275                 280                 285

Asn Asn Trp Tyr Tyr Met Thr Asn Glu Arg Gly Asn Met Val Ser Asn
    290                 295                 300

Glu Phe Ile Lys Ser Gly Lys Gly Trp Tyr Phe Met Asn Thr Asn Gly
305                 310                 315                 320

Glu Leu Ala Asp Asn Pro Ser Phe Thr Lys Glu Pro Asp Gly Leu Ile
                325                 330                 335

Thr Val Ala

<210>  2
<211>  341
<212>  PRT
<213>  Listeria monocytogenes Phage A511

<400>  2

Met Val Lys Tyr Thr Val Glu Asn Lys Ile Ile Ala Gly Leu Pro Lys
1               5                   10                  15

Gly Lys Leu Lys Gly Ala Asn Phe Val Ile Ala His Glu Thr Ala Asn
            20                  25                  30

Ser Lys Ser Thr Ile Asp Asn Glu Val Ser Tyr Met Thr Arg Asn Trp
        35                  40                  45

Lys Asn Ala Phe Val Thr His Phe Val Gly Gly Gly Gly Arg Val Val
    50                  55                  60

Gln Val Ala Asn Val Asn Tyr Val Ser Trp Gly Ala Gly Gln Tyr Ala
65                  70                  75                  80

Asn Ser Tyr Ser Tyr Ala Gln Val Glu Leu Cys Arg Thr Ser Asn Ala
                85                  90                  95

Thr Thr Phe Lys Lys Asp Tyr Glu Val Tyr Cys Gln Leu Leu Val Asp
            100                 105                 110

Leu Ala Lys Lys Ala Gly Ile Pro Ile Thr Leu Asp Ser Gly Ser Lys
        115                 120                 125

Thr Ser Asp Lys Gly Ile Lys Ser His Lys Trp Val Ala Asp Lys Leu
    130                 135                 140

Gly Gly Thr Thr His Gln Asp Pro Tyr Ala Tyr Leu Ser Ser Trp Gly
145                 150                 155                 160

Ile Ser Lys Ala Gln Phe Ala Ser Asp Leu Ala Lys Val Ser Gly Gly
                165                 170                 175

Gly Asn Thr Gly Thr Ala Pro Ala Lys Pro Ser Thr Pro Ala Pro Lys
            180                 185                 190

Pro Ser Thr Pro Ser Thr Asn Leu Asp Lys Leu Gly Leu Val Asp Tyr
        195                 200                 205

Met Asn Ala Lys Lys Met Asp Ser Ser Tyr Ser Asn Arg Asp Lys Leu
    210                 215                 220

Ala Lys Gln Tyr Gly Ile Ala Asn Tyr Ser Gly Thr Ala Ser Gln Asn
225                 230                 235                 240

Thr Thr Leu Leu Ser Lys Ile Lys Gly Gly Ala Pro Lys Pro Ser Thr
                245                 250                 255

Pro Ala Pro Lys Pro Ser Thr Ser Thr Ala Lys Lys Ile Tyr Phe Pro
            260                 265                 270

Pro Asn Lys Gly Asn Trp Ser Val Tyr Pro Thr Asn Lys Ala Pro Val
        275                 280                 285

Lys Ala Asn Ala Ile Gly Ala Ile Asn Pro Thr Lys Phe Gly Gly Leu
    290                 295                 300

Thr Tyr Thr Ile Gln Lys Asp Arg Gly Asn Gly Val Tyr Glu Ile Gln
305                 310                 315                 320

Thr Asp Gln Phe Gly Arg Val Gln Val Tyr Gly Ala Pro Ser Thr Gly
                325                 330                 335

Ala Val Ile Lys Lys
            340

<210> 3
<211> 495
<212> PRT
<213> Staphylococcus aureus Phage K

<400> 3

Met Ala Lys Thr Gln Ala Glu Ile Asn Lys Arg Leu Asp Ala Tyr Ala
1               5                   10                  15

Lys Gly Thr Val Asp Ser Pro Tyr Arg Val Lys Lys Ala Thr Ser Tyr
            20                  25                  30

Asp Pro Ser Phe Gly Val Met Glu Ala Gly Ala Ile Asp Ala Asp Gly
        35                  40                  45

Tyr Tyr His Ala Gln Cys Gln Asp Leu Ile Thr Asp Tyr Val Leu Trp
    50                  55                  60

Leu Thr Asp Asn Lys Val Arg Thr Trp Gly Asn Ala Lys Asp Gln Ile
65                  70                  75                  80

Lys Gln Ser Tyr Gly Thr Gly Phe Lys Ile His Glu Asn Lys Pro Ser
                85                  90                  95

Thr Val Pro Lys Lys Gly Trp Ile Ala Val Phe Thr Ser Gly Ser Tyr
            100                 105                 110

Glu Gln Trp Gly His Ile Gly Ile Val Tyr Asp Gly Gly Asn Thr Ser
        115                 120                 125

Thr Phe Thr Ile Leu Glu Gln Asn Trp Asn Gly Tyr Ala Asn Lys Lys
    130                 135                 140

Pro Thr Lys Arg Val Asp Asn Tyr Tyr Gly Leu Thr His Phe Ile Glu
145                 150                 155                 160

Ile Pro Val Lys Ala Gly Thr Thr Val Lys Lys Glu Thr Ala Lys Lys
                165                 170                 175

Ser Ala Ser Lys Thr Pro Ala Pro Lys Lys Lys Ala Thr Leu Lys Val
            180                 185                 190

Ser Lys Asn His Ile Asn Tyr Thr Met Asp Lys Arg Gly Lys Lys Pro
        195                 200                 205

Glu Gly Met Val Ile His Asn Asp Ala Gly Arg Ser Ser Gly Gln Gln
    210                 215                 220

Tyr Glu Asn Ser Leu Ala Asn Ala Gly Tyr Ala Arg Tyr Ala Asn Gly
225                 230                 235                 240

Ile Ala His Tyr Tyr Gly Ser Glu Gly Tyr Val Trp Glu Ala Ile Asp
                245                 250                 255

Ala Lys Asn Gln Ile Ala Trp His Thr Gly Asp Gly Thr Gly Ala Asn
            260                 265                 270

Ser Gly Asn Phe Arg Phe Ala Gly Ile Glu Val Cys Gln Ser Met Ser
        275                 280                 285

Ala Ser Asp Ala Gln Phe Leu Lys Asn Glu Gln Ala Val Phe Gln Phe
    290                 295                 300

Thr Ala Glu Lys Phe Lys Glu Trp Gly Leu Thr Pro Asn Arg Lys Thr
305                 310                 315                 320

Val Arg Leu His Met Glu Phe Val Pro Thr Ala Cys Pro His Arg Ser
                325                 330                 335

Met Val Leu His Thr Gly Phe Asn Pro Val Thr Gln Gly Arg Pro Ser
            340                 345                 350

Gln Ala Ile Met Asn Lys Leu Lys Asp Tyr Phe Ile Lys Gln Ile Lys
        355                 360                 365

Asn Tyr Met Asp Lys Gly Thr Ser Ser Ser Thr Val Val Lys Asp Gly
    370                 375                 380

Lys Thr Ser Ser Ala Ser Thr Pro Ala Thr Arg Pro Val Thr Gly Ser
385                 390                 395                 400

Trp Lys Lys Asn Gln Tyr Gly Thr Trp Tyr Lys Pro Glu Asn Ala Thr
                405                 410                 415

Phe Val Asn Gly Asn Gln Pro Ile Val Thr Arg Ile Gly Ser Pro Phe
            420                 425                 430

Leu Asn Ala Pro Val Gly Gly Asn Leu Pro Ala Gly Ala Thr Ile Val
        435                 440                 445

Tyr Asp Glu Val Cys Ile Gln Ala Gly His Ile Trp Ile Gly Tyr Asn
    450                 455                 460

Ala Tyr Asn Gly Asn Arg Val Tyr Cys Pro Val Arg Thr Cys Gln Gly
465                 470                 475                 480

Val Pro Pro Asn Gln Ile Pro Gly Val Ala Trp Gly Val Phe Lys
                485                 490                 495

<210>  4
<211>  245
<212>  PRT
<213>  Staphylococcus simulans

<400> 4

Met Ala His Glu His Ser Ala Gln Trp Leu Asn Asn Tyr Lys Lys Gly
1               5                   10                  15

Tyr Gly Tyr Gly Pro Tyr Pro Leu Gly Ile Asn Gly Gly Met His Tyr
            20                  25                  30

Gly Val Asp Phe Phe Met Asn Ile Gly Thr Pro Val Lys Ala Ile Ser
        35                  40                  45

Ser Gly Lys Ile Val Glu Ala Gly Trp Ser Asn Tyr Gly Gly Gly Asn
    50                  55                  60

Gln Ile Gly Leu Ile Glu Asn Asp Gly Val His Arg Gln Trp Tyr Met
65                  70                  75                  80

His Leu Ser Lys Tyr Asn Val Lys Val Gly Asp Tyr Val Lys Ala Gly
                85                  90                  95

Gln Ile Ile Gly Trp Ser Gly Ser Thr Gly Tyr Ser Thr Ala Pro His
            100                 105                 110

Leu His Phe Gln Arg Met Val Asn Ser Phe Ser Asn Ser Thr Ala Gln
        115                 120                 125

Asp Pro Met Pro Phe Leu Lys Ser Ala Gly Tyr Gly Lys Ala Gly Gly
    130                 135                 140

Thr Val Thr Pro Thr Pro Asn Thr Gly Trp Lys Thr Asn Lys Tyr Gly
145                 150                 155                 160

Thr Leu Tyr Lys Ser Glu Ser Ala Ser Phe Thr Pro Asn Thr Asp Ile
                165                 170                 175

Ile Thr Arg Thr Thr Gly Pro Phe Arg Ser Met Pro Gln Ser Gly Val
            180                 185                 190

Leu Lys Ala Gly Gln Thr Ile His Tyr Asp Glu Val Met Lys Gln Asp
        195                 200                 205

Gly His Val Trp Val Gly Tyr Thr Gly Asn Ser Gly Gln Arg Ile Tyr
    210                 215                 220

Leu Pro Val Arg Thr Trp Asn Lys Ser Thr Asn Thr Leu Gly Val Leu
225                 230                 235                 240

Trp Gly Thr Ile Lys
                245

<210> 5

<211>  286
<212>  PRT
<213>  Propionibacterium acnes

<400>  5

Met Val Arg Tyr Ile Pro Ala Ala His His Ser Ala Gly Ser Asn Asn
1               5                   10                  15

Pro Val Asn Arg Val Val Ile His Ala Thr Cys Pro Asp Val Gly Phe
            20                  25                  30

Pro Ser Ala Ser Arg Lys Gly Arg Ala Val Ser Thr Ala Asn Tyr Phe
        35                  40                  45

Ala Ser Pro Ser Ser Gly Gly Ser Ala His Tyr Val Cys Asp Ile Gly
    50                  55                  60

Glu Thr Val Gln Cys Leu Ser Glu Ser Thr Ile Gly Trp His Ala Pro
65                  70                  75                  80

Pro Asn Pro His Ser Leu Gly Ile Glu Ile Cys Ala Asp Gly Gly Ser
                85                  90                  95

His Ala Ser Phe Arg Val Pro Gly His Ala Tyr Thr Arg Glu Gln Trp
            100                 105                 110

Leu Asp Pro Gln Val Trp Pro Ala Val Glu Arg Ala Ala Val Leu Cys
        115                 120                 125

Arg Arg Leu Cys Asp Lys Tyr Asn Val Pro Lys Arg Lys Leu Ser Ala
    130                 135                 140

Ala Asp Leu Lys Ala Gly Arg Arg Gly Val Cys Gly His Val Asp Val
145                 150                 155                 160

Thr Asp Ala Trp His Gln Ser Asp His Asp Asp Pro Gly Pro Trp Phe
                165                 170                 175

Pro Trp Asp Lys Phe Met Ala Val Val Asn Gly Gly Ser Gly Asp Ser
            180                 185                 190

Gly Glu Leu Thr Val Ala Asp Val Lys Ala Leu His Asp Gln Ile Lys
        195                 200                 205

Gln Leu Ser Ala Gln Leu Thr Gly Ser Val Asn Lys Leu His His Asp
    210                 215                 220

Val Gly Val Val Gln Val Gln Asn Gly Asp Leu Gly Lys Arg Val Asp
225                 230                 235                 240

Ala Leu Ser Trp Val Lys Asn Pro Val Thr Gly Lys Leu Trp Arg Thr
                245                 250                 255

Lys Asp Ala Leu Trp Ser Val Trp Tyr Tyr Val Leu Glu Cys Arg Ser
            260                 265                 270

Arg Leu Asp Arg Leu Glu Ser Ala Val Asn Asp Leu Lys Lys
        275                 280                 285

<210>  6
<211>  260
<212>  PRT
<213>  Pseudomonas aeruginosa phage KZ

<400>  6

Met Lys Val Leu Arg Lys Gly Asp Arg Gly Asp Glu Val Cys Gln Leu
1               5                   10                  15

Gln Thr Leu Leu Asn Leu Cys Gly Tyr Asp Val Gly Lys Pro Asp Gly
            20                  25                  30

Ile Phe Gly Asn Asn Thr Phe Asn Gln Val Val Lys Phe Gln Lys Asp
        35                  40                  45

Asn Cys Leu Asp Ser Asp Gly Ile Val Gly Lys Asn Thr Trp Ala Glu
    50                  55                  60

Leu Phe Ser Lys Tyr Ser Pro Pro Ile Pro Tyr Lys Thr Ile Pro Met
65                  70                  75                  80

Pro Thr Ala Asn Lys Ser Arg Ala Ala Ala Thr Pro Val Met Asn Ala
                85                  90                  95

Val Glu Asn Ala Thr Gly Val Arg Ser Gln Leu Leu Leu Thr Phe Ala
            100                 105                 110

Ser Ile Glu Ser Ala Phe Asp Tyr Glu Ile Lys Ala Lys Thr Ser Ser
        115                 120                 125

Ala Thr Gly Trp Phe Gln Phe Leu Thr Gly Thr Trp Lys Thr Met Ile
    130                 135                 140

Glu Asn Tyr Gly Met Lys Tyr Gly Val Leu Thr Asp Pro Thr Gly Ala
145                 150                 155                 160

Leu Arg Lys Asp Pro Arg Ile Ser Ala Leu Met Gly Ala Glu Leu Ile
                165                 170                 175

Lys Glu Asn Met Asn Ile Leu Arg Pro Val Leu Lys Arg Glu Pro Thr
            180                 185                 190

Asp Thr Asp Leu Tyr Leu Ala His Phe Phe Gly Pro Gly Ala Ala Arg
        195                 200                 205

Arg Phe Leu Thr Thr Gly Gln Asn Glu Leu Ala Ala Thr His Phe Pro
    210                 215                 220

Lys Glu Ala Gln Ala Asn Pro Ser Ile Phe Tyr Asn Lys Asp Gly Ser
225                 230                 235                 240

Pro Lys Thr Ile Gln Glu Val Tyr Asn Leu Met Asp Gly Lys Val Ala
                245                 250                 255

Ala His Arg Lys
            260

<210>  7
<211>  292
<212>  PRT
<213>  Pseudomonas aeruginosa phage EL

<400>  7

Met Asn Phe Arg Thr Lys Asn Gly Tyr Arg Asp Leu Gln Ala Leu Val
1               5                   10                  15

Lys Glu Leu Gly Leu Tyr Thr Gly Gln Ile Asp Gly Val Trp Gly Lys
            20                  25                  30

Gly Thr Ser Ser Ser Thr Glu Thr Leu Leu Arg Gly Tyr Ala Glu Val
        35                  40                  45

Val Gly Lys Asn Thr Gly Gly Ile Gly Leu Pro Thr Thr Ser Asp Ala
    50                  55                  60

Ser Gly Tyr Asn Val Ile Thr Ala Leu Gln Arg Asn Leu Ala Phe Leu
65                  70                  75                  80

Gly Leu Tyr Ser Leu Thr Val Asp Gly Ile Trp Gly Asn Gly Thr Leu
                85                  90                  95

Ser Gly Leu Asp Lys Ala Phe Glu Val Tyr Lys Glu Arg Tyr Arg Thr
            100                 105                 110

Pro Thr Tyr Asp Ile Ala Trp Ser Gly Lys Val Ser Pro Ala Phe Thr
        115                 120                 125

Ala Lys Val Lys Asp Trp Cys Gly Val His Val Pro Asn His Arg Ala
    130                 135                 140

Pro His Trp Leu Met Ala Cys Met Ala Phe Glu Thr Gly Gln Thr Phe
145                 150                 155                 160

Ser Pro Ser Ile Lys Asn Ala Ala Gly Ser Glu Ala Tyr Gly Leu Ile
                165                 170                 175

Gln Phe Met Ser Pro Ala Ala Asn Asp Leu Asn Val Pro Leu Ser Val
            180                 185                 190

Ile Arg Ser Met Asp Gln Leu Thr Gln Leu Asp Leu Val Phe Lys Tyr
        195                 200                 205

Phe Glu Met Trp Met Lys Arg Gly Lys Arg Tyr Thr Gln Leu Glu Asp
    210                 215                 220

Phe Tyr Leu Thr Ile Phe His Pro Ala Ser Val Gly Lys Lys Ala Asp
225                 230                 235                 240

Glu Val Leu Phe Leu Gln Gly Ser Lys Ala Tyr Leu Gln Asn Lys Gly
                245                 250                 255

Phe Asp Val Asp Lys Asp Gly Lys Ile Thr Leu Gly Glu Ile Ser Ser
            260                 265                 270

Thr Leu Tyr Thr Thr Tyr Tyr Lys Gly Leu Leu Pro Glu Asn Arg His
        275                 280                 285

Val Ile Ser Tyr
    290

<210>  8
<211>  181
<212>  PRT
<213>  Salmonella

<400>  8

Met Lys Pro Lys Asp Glu Ile Phe Asp Glu Ile Leu Gly Lys Glu Gly
1               5                   10                  15

Gly Tyr Val Asn His Pro Asp Asp Lys Gly Gly Pro Thr Lys Trp Gly
            20                  25                  30

Ile Thr Glu Lys Val Ala Arg Ala His Gly Tyr Arg Gly Asp Met Arg
        35                  40                  45

Asn Leu Thr Arg Gly Gln Ala Leu Glu Ile Leu Glu Thr Asp Tyr Trp
    50                  55                  60

Tyr Gly Pro Arg Phe Asp Arg Val Ala Lys Ala Ser Pro Asp Val Ala
65                  70                  75                  80

Ala Glu Leu Cys Asp Thr Gly Val Asn Met Gly Pro Ser Val Ala Ala
                85                  90                  95

Lys Met Leu Gln Arg Trp Leu Asn Val Phe Asn Gln Gly Gly Arg Leu
            100                 105                 110

Tyr Pro Asp Met Asp Thr Asp Gly Arg Ile Gly Pro Arg Thr Leu Asn

115                 120                 125

Ala Leu Arg Val Tyr Leu Glu Lys Arg Gly Lys Asp Gly Glu Arg Val
    130                 135                 140

Leu Leu Val Ala Leu Asn Cys Thr Gln Gly Glu Arg Tyr Leu Glu Leu
145                 150                 155                 160

Ala Glu Lys Arg Glu Ala Asp Glu Ser Phe Val Tyr Gly Trp Met Lys
                165                 170                 175

Glu Arg Val Leu Ile
            180

<210>  9
<211>  163
<212>  PRT
<213>  Enterobacteria phage T4

<400>  9

Met Asn Ile Phe Glu Met Leu Arg Ile Asp Glu Gly Leu Arg Leu Lys
1               5                   10                  15

Ile Tyr Lys Asp Thr Glu Gly Tyr Tyr Thr Ile Gly Ile Gly His Leu
            20                  25                  30

Leu Thr Lys Ser Pro Ser Leu Asn Ala Ala Lys Ser Glu Leu Asp Lys
        35                  40                  45

Ala Ile Gly Arg Asn Cys Asn Gly Val Ile Thr Lys Asp Glu Ala Glu
    50                  55                  60

Lys Leu Phe Asn Gln Asp Val Asp Ala Ala Val Arg Gly Ile Leu Arg
65                  70                  75                  80

Asn Ala Lys Leu Lys Pro Val Tyr Asp Ser Leu Asp Ala Val Arg Arg
                85                  90                  95

Cys Ala Leu Ile Asn Met Val Phe Gln Met Gly Glu Thr Gly Val Ala
            100                 105                 110

Gly Phe Thr Asn Ser Leu Arg Met Leu Gln Gln Lys Arg Trp Asp Glu
        115                 120                 125

Ala Ala Val Asn Leu Ala Lys Ser Arg Trp Tyr Asn Gln Thr Pro Asn
    130                 135                 140

Arg Ala Lys Arg Val Ile Thr Thr Phe Arg Thr Gly Thr Trp Asp Ala
145                 150                 155                 160

Tyr Lys Asn

<210>  10
<211>  280
<212>  PRT
<213>  Acinetobacter baumannii phage

<400>  10

Met Glu Tyr Asp Met Ile Leu Lys Phe Gly Ser Lys Gly Asp Ala Val
1               5                   10                  15

Ala Thr Leu Gln Lys Gln Leu Ala Lys Met Gly Tyr Lys Gly Val Lys
            20                  25                  30

Asp Lys Pro Leu Ser Val Asp Gly His Phe Gly Glu Ser Thr Glu Phe
        35                  40                  45

Ala Val Ile Gln Leu Gln Arg Lys Phe Gly Leu Val Ala Asp Gly Lys
    50                  55                  60

Val Gly Asp Lys Thr Arg Gln Ala Leu Ala Gly Asp Ser Val Ser Lys
65                  70                  75                  80

Phe Leu Lys Asp Glu Asp Tyr Lys Lys Ala Ala Ile Arg Leu Lys Val
                85                  90                  95

Pro Glu Leu Val Ile Arg Val Phe Gly Ala Val Glu Gly Leu Gly Val
            100                 105                 110

Gly Phe Leu Pro Asn Gly Lys Ala Lys Ile Leu Phe Glu Arg His Arg
        115                 120                 125

Met Tyr Phe Tyr Leu Cys Gln Ala Leu Gly Lys Thr Phe Ala Asn Ser
    130                 135                 140

Gln Val Lys Ile Thr Pro Asn Ile Val Asn Thr Leu Thr Gly Gly Tyr
145                 150                 155                 160

Lys Gly Asp Ala Ala Glu Tyr Thr Arg Leu Ser Met Ala Ile Asn Ile
                165                 170                 175

His Lys Glu Ser Ala Leu Met Ser Thr Ser Trp Gly Gln Phe Gln Ile
            180                 185                 190

Met Gly Glu Asn Trp Lys Asp Leu Gly Tyr Ser Ser Val Gln Glu Phe
        195                 200                 205

Val Asp Gln Gln Gln Leu Asn Glu Gly Asn Gln Leu Glu Ala Phe Ile
    210                 215                 220

Arg Phe Ile Glu Trp Lys Pro Gly Leu Leu Glu Ala Leu Arg Lys Gln
225                 230                 235                 240

Asp Trp Asp Thr Val Phe Thr Leu Tyr Asn Gly Lys Asn Tyr Lys Lys
                245                 250                 255

Leu Gly Tyr Gln Ala Lys Phe Gln Lys Glu Trp Asp His Leu Glu Pro
            260                 265                 270

Ile Tyr Arg Glu Lys Thr Ala Ala
        275                 280

<210>  11
<211>  152
<212>  PRT
<213>  E. coli phage K1F

<400>  11

Met Val Ser Lys Val Gln Phe Asn Pro Arg Ser Arg Thr Asp Ala Ile
1               5                   10                  15

Phe Val His Cys Ser Ala Thr Lys Pro Glu Met Asp Ile Gly Val Glu
            20                  25                  30

Thr Ile Arg Met Trp His Lys Gln Gln Ala Trp Leu Asp Val Gly Tyr
        35                  40                  45

His Phe Ile Ile Lys Arg Asp Gly Thr Val Glu Glu Gly Arg Pro Val
    50                  55                  60

Asn Val Val Gly Ser His Val Lys Asp Trp Asn Ser Arg Ser Val Gly
65                  70                  75                  80

Val Cys Leu Val Gly Gly Ile Asn Ala Lys Gly Gln Phe Glu Ala Asn
                85                  90                  95

Phe Thr Pro Ala Gln Met Asn Ser Leu Arg Asn Lys Leu Asp Asp Leu
            100                 105                 110

Lys Val Met Tyr Pro Gln Ala Glu Ile Arg Ala His His Asp Val Ala
        115                 120                 125

Pro Lys Ala Cys Pro Ser Phe Asp Leu Gln Arg Trp Leu Ser Thr Asn
    130                 135                 140

Glu Leu Val Thr Ser Asp Arg Gly
145                 150

<210>  12
<211>  328
<212>  PRT
<213>  Pseudomonas putida phage

<400>  12

Met Lys Asn Ser Glu Lys Asn Ala Ser Ile Ile Met Ser Ile Gln Arg

1               5                   10                  15

Thr Leu Ala Ser Leu Ser Leu Tyr Gly Gly Arg Ile Asp Gly Leu Phe
            20                  25                  30

Gly Glu Lys Cys Arg Gly Ala Ile Ile Leu Met Leu Asn Lys Val Tyr
        35                  40                  45

Pro Asn Phe Ser Thr Asn Lys Leu Pro Ser Asn Thr Tyr Glu Ala Glu
    50                  55                  60

Ser Val Phe Thr Phe Leu Gln Thr Ala Leu Ala Gly Val Gly Leu Tyr
65                  70                  75                  80

Thr Ile Thr Ile Asp Gly Lys Trp Gly Gly Thr Ser Gln Gly Ala Ile
                85                  90                  95

Asp Ala Leu Val Lys Ser Tyr Arg Gln Ile Thr Glu Ala Glu Arg Ala
            100                 105                 110

Gly Ser Thr Leu Pro Leu Gly Leu Ala Thr Val Met Ser Lys His Met
        115                 120                 125

Ser Ile Glu Gln Leu Arg Ala Met Leu Pro Thr Asp Arg Gln Gly Tyr
    130                 135                 140

Ala Glu Val Tyr Ile Asp Pro Leu Asn Glu Thr Met Asp Ile Phe Glu
145                 150                 155                 160

Ile Asn Thr Pro Leu Arg Ile Ala His Phe Met Ala Gln Ile Leu His
                165                 170                 175

Glu Thr Ala Cys Phe Lys Tyr Thr Glu Glu Leu Ala Ser Gly Lys Ala
            180                 185                 190

Tyr Glu Gly Arg Ala Asp Leu Gly Asn Thr Arg Pro Gly Asp Gly Pro
        195                 200                 205

Leu Phe Lys Gly Arg Gly Leu Leu Gln Ile Thr Gly Arg Leu Asn Tyr
    210                 215                 220

Val Lys Cys Gln Val Tyr Leu Arg Glu Lys Leu Lys Asp Pro Thr Phe
225                 230                 235                 240

Asp Ile Thr Ser Ser Val Thr Cys Ala Gln Gln Leu Ser Glu Ser Pro
                245                 250                 255

Leu Leu Ala Ala Leu Ala Ser Gly Tyr Phe Trp Arg Phe Ile Lys Pro
            260                 265                 270

Lys Leu Asn Glu Thr Ala Asp Lys Asp Asp Ile Tyr Trp Val Ser Val

275                 280                 285

Tyr Val Asn Gly Tyr Ala Lys Gln Ala Asn Pro Tyr Tyr Pro Asn Arg
    290                 295                 300

Asp Lys Glu Pro Asn His Met Lys Glu Arg Val Gln Met Leu Ala Val
305                 310                 315                 320

Thr Lys Lys Ala His Gly Ile Val
                325

<210>  13
<211>  165
<212>  PRT
<213>  Salmonella

<400>  13

Met Pro Val Ile Asn Thr His Gln Asn Ile Ala Ala Phe Leu Asp Met
1               5                   10                  15

Leu Ala Tyr Ser Glu Gly Thr Ala Asn His Pro Leu Thr Lys Asn Arg
            20                  25                  30

Gly Tyr Asp Val Ile Val Thr Gly Phe Asp Gly Ser Pro Glu Ile Phe
        35                  40                  45

Thr Asp Tyr Ser Asp His Pro Phe Ala His Gly Arg Pro Pro Lys Val
    50                  55                  60

Phe Asn Arg Arg Gly Glu Lys Ser Thr Ala Ser Gly Arg Tyr Gln Gln
65                  70                  75                  80

Leu Tyr Ile Phe Trp Pro His Tyr Lys Lys Gln Leu Ala Leu Pro Asp
                85                  90                  95

Phe Ser Pro Leu Ser Gln Asp Lys Leu Ala Ile Gln Leu Ile Arg Glu
            100                 105                 110

Arg Gly Ala Ile Asp Asp Ile Arg Ala Gly Arg Ile Glu Arg Ala Val
        115                 120                 125

Ser Arg Cys Arg Asn Ile Trp Ala Ser Leu Pro Gly Ala Gly Tyr Gly
    130                 135                 140

Gln Arg Glu His Ser Leu Glu Lys Leu Val Thr Val Trp Arg Thr Ala
145                 150                 155                 160

Gly Gly Val Met Ala
                165

<210>  14
<211>  165

<212>  PRT
<213>  E.coli Phage P2

<400>  14

Met Pro Val Ile Asn Thr His Gln Asn Ile Ala Ala Phe Leu Asp Met
1               5                   10                  15

Leu Ala Val Ser Glu Gly Thr Ala Asn His Pro Leu Thr Lys Asn Arg
            20                  25                  30

Gly Tyr Asp Val Ile Val Thr Gly Leu Asp Gly Lys Pro Glu Ile Phe
        35                  40                  45

Thr Asp Tyr Ser Asp His Pro Phe Ala His Gly Arg Pro Ala Lys Val
    50                  55                  60

Phe Asn Arg Arg Gly Glu Lys Ser Thr Ala Ser Gly Arg Tyr Gln Gln
65                  70                  75                  80

Leu Tyr Leu Phe Trp Pro His Tyr Arg Lys Gln Leu Ala Leu Pro Asp
                85                  90                  95

Phe Ser Pro Leu Ser Gln Asp Arg Leu Ala Ile Gln Leu Ile Arg Glu
            100                 105                 110

Arg Gly Ala Leu Asp Asp Ile Arg Ala Gly Arg Ile Glu Arg Ala Ile
        115                 120                 125

Ser Arg Cys Arg Asn Ile Trp Ala Ser Leu Pro Gly Ala Gly Tyr Gly
    130                 135                 140

Gln Arg Glu His Ser Leu Glu Lys Leu Val Thr Val Trp Arg Thr Ala
145                 150                 155                 160

Gly Gly Val Pro Ala
                165


<210>  15
<211>  177
<212>  PRT
<213>  Salmonella typhimurium phage

<400>  15

Met Asn Pro Ile Ile Asp Gly Ile Ile Ala Leu Glu Gly Gly Tyr Val
1               5                   10                  15

Phe Asn Pro Lys Asp Lys Gly Gly Ala Thr His Trp Gly Ile Thr Glu
            20                  25                  30

Ala Thr Ala Arg Ala His Gly Tyr Ala Gly Asp Met Arg Asp Leu Thr
        35                  40                  45

His Ala Glu Ala Tyr Ala Ile Leu Glu Glu Asp Tyr Trp Ile Lys Pro
    50                  55                  60

Gly Phe Asp Val Ile Ser Thr Leu Ser Trp Pro Val Ser Phe Glu Leu
65                  70                  75                  80

Cys Asp Ala Ala Val Asn Ile Gly Ala Tyr His Pro Ser Ala Trp Leu
                85                  90                  95

Gln Arg Trp Leu Asn Val Phe Asn His Glu Gly Lys Arg Tyr Pro Asp
            100                 105                 110

Ile His Val Asp Gly Asn Ile Gly Pro Arg Thr Leu Ala Ala Leu Glu
        115                 120                 125

His Tyr Leu Ala Trp Arg Gly Gln Glu Gly Glu Ala Val Leu Val Lys
    130                 135                 140

Ala Leu Asn Cys Ser Gln Gly Thr Tyr Tyr Leu Asn Val Ala Glu Lys
145                 150                 155                 160

Asn His Asn Asn Glu Gln Phe Ile Tyr Gly Trp Ile Lys Asn Arg Val
                165                 170                 175

Thr

<210>  16
<211>  208
<212>  PRT
<213>  E. coli Phage N4

<400>  16

Met Ala Ile Ser Lys Lys Lys Val Gly Gly Val Gly Gly Val Ile Ala
1               5                   10                  15

Ala Ile Ile Ala Ala Val Phe Ala Val Glu Gly Gly Tyr Val Asn Asp
            20                  25                  30

Pro Lys Asp Pro Gly Gly Glu Thr Asn His Gly Val Thr Ile Gln Val
        35                  40                  45

Ala Gln Lys His Lys Gln Glu Leu Glu Ser Met Tyr Asn Trp Asp Gly
    50                  55                  60

Ser Met Lys Asn Leu Thr Gln Glu Met Ala Ser Ser Ile Tyr Tyr Asn
65                  70                  75                  80

Asp Tyr Ile Leu Lys Pro Gly Phe Val Lys Phe Ala Asp Val Ser Pro
                85                  90                  95

Ala Val Thr Glu Lys Leu Val Asp Ala Gly Val Asn Thr Gly Pro Ala

            100                 105                 110

Arg Pro Ser Arg Trp Leu Gln Glu Ser Leu Asn Ala Phe Ser Arg Asn
        115                 120                 125

Gly Lys Asp Tyr Pro Lys Ile Gln Val Asp Gly Lys Val Gly Ser Gly
    130                 135                 140

Thr Leu Ser Ala Tyr Lys Ser Leu Gln Asn Lys Arg Gly Lys Val Glu
145                 150                 155                 160

Ala Cys Lys Leu Ile Leu Lys Ser Leu Asp Gly Lys Gln Leu Asn Tyr
                165                 170                 175

Tyr Leu Ser Leu Asn Met Pro Glu Tyr Thr Thr Gly Trp Ile Ala Asn
            180                 185                 190

Arg Ile Gly Asn Val Pro Leu Glu Arg Cys Asn Glu Asp Ile Val Asn
        195                 200                 205

<210> 17
<211> 185
<212> PRT
<213> N4-gp61 trunc.

<400> 17

Met Val Glu Gly Gly Tyr Val Asn Asp Pro Lys Asp Pro Gly Gly Glu
1               5                   10                  15

Thr Asn His Gly Val Thr Ile Gln Val Ala Gln Lys His Lys Gln Glu
            20                  25                  30

Leu Glu Ser Met Tyr Asn Trp Asp Gly Ser Met Lys Asn Leu Thr Gln
        35                  40                  45

Glu Met Ala Ser Ser Ile Tyr Tyr Asn Asp Tyr Ile Leu Lys Pro Gly
    50                  55                  60

Phe Val Lys Phe Ala Asp Val Ser Pro Ala Val Thr Glu Lys Leu Val
65                  70                  75                  80

Asp Ala Gly Val Asn Thr Gly Pro Ala Arg Pro Ser Arg Trp Leu Gln
                85                  90                  95

Glu Ser Leu Asn Ala Phe Ser Arg Asn Gly Lys Asp Tyr Pro Lys Ile
            100                 105                 110

Gln Val Asp Gly Lys Val Gly Ser Gly Thr Leu Ser Ala Tyr Lys Ser
        115                 120                 125

Leu Gln Asn Lys Arg Gly Lys Val Glu Ala Cys Lys Leu Ile Leu Lys
    130                 135                 140

Ser Leu Asp Gly Lys Gln Leu Asn Tyr Tyr Leu Ser Leu Asn Met Pro
145                 150                 155                 160

Glu Tyr Thr Thr Gly Trp Ile Ala Asn Arg Ile Gly Asn Val Pro Leu
                165                 170                 175

Glu Arg Cys Asn Glu Asp Ile Val Asn
            180                 185

<210>  18
<211>  259
<212>  PRT
<213>  Pseudomonas aeruginosa phage KZ

<400>  18

Lys Val Leu Arg Lys Gly Asp Arg Gly Asp Glu Val Cys Gln Leu Gln
1               5                   10                  15

Thr Leu Leu Asn Leu Cys Gly Tyr Asp Val Gly Lys Pro Asp Gly Ile
            20                  25                  30

Phe Gly Asn Asn Thr Phe Asn Gln Val Val Lys Phe Gln Lys Asp Asn
        35                  40                  45

Cys Leu Asp Ser Asp Gly Ile Val Gly Lys Asn Thr Trp Ala Glu Leu
    50                  55                  60

Phe Ser Lys Tyr Ser Pro Pro Ile Pro Tyr Lys Thr Ile Pro Met Pro
65                  70                  75                  80

Thr Ala Asn Lys Ser Arg Ala Ala Ala Thr Pro Val Met Asn Ala Val
                85                  90                  95

Glu Asn Ala Thr Gly Val Arg Ser Gln Leu Leu Leu Thr Phe Ala Ser
            100                 105                 110

Ile Glu Ser Ala Phe Asp Tyr Glu Ile Lys Ala Lys Thr Ser Ser Ala
        115                 120                 125

Thr Gly Trp Phe Gln Phe Leu Thr Gly Thr Trp Lys Thr Met Ile Glu
    130                 135                 140

Asn Tyr Gly Met Lys Tyr Gly Val Leu Thr Asp Pro Thr Gly Ala Leu
145                 150                 155                 160

Arg Lys Asp Pro Arg Ile Ser Ala Leu Met Gly Ala Glu Leu Ile Lys
                165                 170                 175

Glu Asn Met Asn Ile Leu Arg Pro Val Leu Lys Arg Glu Pro Thr Asp
            180                 185                 190

Thr Asp Leu Tyr Leu Ala His Phe Phe Gly Pro Gly Ala Ala Arg Arg
        195                 200                 205

Phe Leu Thr Thr Gly Gln Asn Glu Leu Ala Ala Thr His Phe Pro Lys
    210                 215                 220

Glu Ala Gln Ala Asn Pro Ser Ile Phe Tyr Asn Lys Asp Gly Ser Pro
225                 230                 235                 240

Lys Thr Ile Gln Glu Val Tyr Asn Leu Met Asp Gly Lys Val Ala Ala
                245                 250                 255

His Arg Lys

<210>  19
<211>  6
<212>  PRT
<213>  artificial

<220>
<223>  synthetic sequence

<400>  19

Lys Arg Lys Lys Arg Lys
1               5

<210>  20
<211>  5
<212>  PRT
<213>  artificial

<220>
<223>  synethtic sequence

<220>
<221>  misc_feature
<222>  (3)..(3)
<223>  Xaa can be any naturally occurring amino acid

<400>  20

Lys Arg Xaa Lys Arg
1               5

<210>  21
<211>  5
<212>  PRT
<213>  artificial

<220>
<223>  synthetic sequence

<400>  21

Lys Arg Ser Lys Arg
1               5

<210>  22
<211>  5
<212>  PRT
<213>  artificial

<220>
<223>  synthetic sequence

<400>  22

Lys Arg Gly Ser Gly
1               5


<210>  23
<211>  9
<212>  PRT
<213>  artificial

<220>
<223>  synthetic sequence

<400>  23

Lys Arg Lys Lys Arg Lys Lys Arg Lys
1               5


<210>  24
<211>  9
<212>  PRT
<213>  artificial

<220>
<223>  synthetic sequence

<400>  24

Arg Arg Arg Arg Arg Arg Arg Arg Arg
1               5


<210>  25
<211>  8
<212>  PRT
<213>  artificial

<220>
<223>  synthetic sequence

<400>  25

Lys Lys Lys Lys Lys Lys Lys Lys
1               5


<210>  26
<211>  10
<212>  PRT
<213>  artificial

<220>
<223>  synthetic sequence

<400>  26

Lys Arg Lys Lys Arg Lys Lys Arg Lys Lys

1                   5                   10


<210>  27
<211>  12
<212>  PRT
<213>  artificial

<220>
<223>  synthetic sequence

<400>  27

Lys Arg Lys Lys Arg Lys Lys Arg Lys Lys Arg Lys
1               5                   10


<210>  28
<211>  14
<212>  PRT
<213>  artificial

<220>
<223>  synthetic sequence

<400>  28

Lys Arg Lys Lys Arg Lys Lys Arg Lys Lys Arg Lys Lys Arg
1               5                   10


<210>  29
<211>  16
<212>  PRT
<213>  artificial

<220>
<223>  synthetic sequence

<400>  29

Lys Lys Lys Lys Lys Lys Lys Lys Lys Lys Lys Lys Lys Lys Lys Lys
1               5                   10                  15


<210>  30
<211>  18
<212>  PRT
<213>  artificial

<220>
<223>  synthetic sequence

<400>  30

Lys Arg Lys Lys Arg Lys Lys Arg Lys Lys Arg Lys Lys Arg Lys Lys
1               5                   10                  15


Arg Lys



<210>  31
<211>  19
<212>  PRT
<213>  artificial

<220>
<223>  synthetic sequence

<400>  31

Lys Arg Lys Lys Arg Lys Lys Arg Lys Lys Arg Lys Lys Arg Lys Lys
1               5                   10                  15

Arg Lys Lys

<210>  32
<211>  19
<212>  PRT
<213>  artificial

<220>
<223>  synthetic sequence

<400>  32

Arg Arg Arg Arg Arg Arg Arg Arg Arg Arg Arg Arg Arg Arg Arg Arg
1               5                   10                  15

Arg Arg Arg

<210>  33
<211>  19
<212>  PRT
<213>  artificial

<220>
<223>  synthetic sequence

<400>  33

Lys Lys Lys Lys Lys Lys Lys Lys Lys Lys Lys Lys Lys Lys Lys Lys
1               5                   10                  15

Lys Lys Lys

<210>  34
<211>  20
<212>  PRT
<213>  artificial

<220>
<223>  synthetic sequence

<400>  34

Lys Arg Lys Lys Arg Lys Lys Arg Lys Arg Ser Lys Arg Lys Lys Arg
1               5                   10                  15

Lys Lys Arg Lys
            20

<210>  35

<211>  21
<212>  PRT
<213>  artificial

<220>
<223>  synthetic sequence

<400>  35

Lys Arg Lys Lys Arg Lys Lys Arg Lys Arg Ser Lys Arg Lys Lys Arg
1               5                   10                  15


Lys Lys Arg Lys Lys
            20


<210>  36
<211>  21
<212>  PRT
<213>  artificial

<220>
<223>  synthetic sequence

<400>  36

Lys Arg Lys Lys Arg Lys Lys Arg Lys Lys Arg Lys Lys Arg Lys Lys
1               5                   10                  15


Arg Lys Lys Arg Lys
            20


<210>  37
<211>  22
<212>  PRT
<213>  artificial

<220>
<223>  synthetic sequence

<400>  37

Lys Arg Lys Lys Arg Lys Lys Arg Lys Arg Gly Ser Gly Lys Arg Lys
1               5                   10                  15


Lys Arg Lys Lys Arg Lys
            20


<210>  38
<211>  24
<212>  PRT
<213>  artificial

<220>
<223>  synthetic sequence

<400>  38

Lys Arg Lys Lys Arg Lys Lys Arg Lys Arg Gly Ser Gly Ser Gly Lys
1               5                   10                  15


Arg Lys Lys Arg Lys Lys Arg Lys

            20

<210>  39
<211>  25
<212>  PRT
<213>  artificial

<220>
<223>  synthetic sequence

<400>  39

Lys Arg Lys Lys Arg Lys Lys Arg Lys Lys Arg Lys Lys Arg Lys Lys
1               5                   10                  15

Arg Lys Lys Arg Lys Lys Arg Lys Lys
            20                  25

<210>  40
<211>  31
<212>  PRT
<213>  artificial

<220>
<223>  synthetic sequence

<400>  40

Lys Arg Lys Lys Arg Lys Lys Arg Lys Arg Ser Lys Arg Lys Lys Arg
1               5                   10                  15

Lys Lys Arg Lys Arg Ser Lys Arg Lys Lys Arg Lys Lys Arg Lys
            20                  25                  30

<210>  41
<211>  38
<212>  PRT
<213>  artificial

<220>
<223>  synthetic sequence

<400>  41

Lys Arg Lys Lys Arg Lys Lys Arg Lys Arg Gly Ser Gly Ser Gly Lys
1               5                   10                  15

Arg Lys Lys Arg Lys Lys Arg Lys Gly Ser Gly Ser Gly Lys Arg Lys
            20                  25                  30

Lys Arg Lys Lys Arg Lys
        35

<210>  42
<211>  39
<212>  PRT
<213>  artificial

<220>
<223>  synthetic sequence

<400>  42

Lys Arg Lys Lys Arg Lys Lys Arg Lys Lys Arg Lys Lys Arg Lys Lys
1               5                   10                  15

Arg Lys Lys Arg Lys Lys Arg Lys Lys Arg Lys Lys Arg Lys Lys Arg
            20                  25                  30

Lys Lys Arg Lys Lys Arg Lys
        35

<210>  43
<211>  42
<212>  PRT
<213>  artificial

<220>
<223>  synthetic sequence

<400>  43

Lys Arg Lys Lys Arg Lys Lys Arg Lys Arg Ser Lys Arg Lys Lys Arg
1               5                   10                  15

Lys Lys Arg Lys Arg Ser Lys Arg Lys Lys Arg Lys Lys Arg Lys Arg
            20                  25                  30

Ser Lys Arg Lys Lys Arg Lys Lys Arg Lys
        35                  40

<210>  44
<211>  37
<212>  PRT
<213>  LL-37 human

<400>  44

Leu Leu Gly Asp Phe Phe Arg Lys Ser Lys Glu Lys Ile Gly Lys Glu
1               5                   10                  15

Phe Lys Arg Ile Val Gln Arg Ile Lys Asp Phe Leu Arg Asn Leu Val
            20                  25                  30

Pro Arg Thr Glu Ser
        35

<210>  45
<211>  29
<212>  PRT
<213>  SMAP-29 sheep

<400>  45

Arg Gly Leu Arg Arg Leu Gly Arg Lys Ile Ala His Gly Val Lys Lys
1               5                   10                  15

Tyr Gly Pro Thr Val Leu Arg Ile Ile Arg Ile Ala Gly

20                  25

<210>  46
<211>  13
<212>  PRT
<213>  Indolicidin Bovine

<400>  46

Ile Leu Pro Trp Lys Trp Pro Trp Trp Pro Trp Arg Arg
1               5                   10

<210>  47
<211>  18
<212>  PRT
<213>  Protegrin Porcine

<400>  47

Arg Gly Gly Arg Leu Cys Tyr Cys Arg Arg Arg Phe Cys Val Cys Val
1               5                   10                  15

Gly Arg

<210>  48
<211>  31
<212>  PRT
<213>  Cecropin P1 Mammal (pig)

<400>  48

Ser Trp Leu Ser Lys Thr Ala Lys Lys Leu Glu Asn Ser Ala Lys Lys
1               5                   10                  15

Arg Ile Ser Glu Gly Ile Ala Ile Ala Ile Gln Gly Gly Pro Arg
            20                  25                  30

<210>  49
<211>  23
<212>  PRT
<213>  Magainin frog

<400>  49

Gly Ile Gly Lys Phe Leu His Ser Ala Lys Lys Phe Gly Lys Ala Phe
1               5                   10                  15

Val Gly Glu Ile Met Asn Ser
            20

<210>  50
<211>  25
<212>  PRT
<213>  Pleurocidin fish

<400>  50

Gly Trp Gly Ser Phe Phe Lys Lys Ala Ala His Val Gly Lys His Val
1               5                   10                  15

Gly Lys Ala Ala Leu Thr His Tyr Leu
            20                  25


<210>  51
<211>  36
<212>  PRT
<213>  Cecropin A (Aedes.aegypti)

<400>  51

Gly Gly Leu Lys Lys Leu Gly Lys Lys Leu Glu Gly Ala Gly Lys Arg
1               5                   10                  15


Val Phe Asn Ala Ala Glu Lys Ala Leu Pro Val Val Ala Gly Ala Lys
            20                  25                  30


Ala Leu Arg Lys
        35


<210>  52
<211>  40
<212>  PRT
<213>  Cecropin A (Drosophila. melanogaster)

<400>  52

Gly Trp Leu Lys Lys Ile Gly Lys Lys Ile Glu Arg Val Gly Gln His
1               5                   10                  15


Thr Arg Asp Ala Thr Ile Gln Gly Leu Gly Ile Pro Gln Gln Ala Ala
            20                  25                  30


Asn Val Ala Ala Thr Ala Arg Gly
        35                  40


<210>  53
<211>  21
<212>  PRT
<213>  Buforin II vertebrate

<400>  53

Thr Arg Ser Ser Arg Ala Gly Leu Gln Phe Pro Val Gly Arg Val His
1               5                   10                  15


Arg Leu Leu Arg Lys
            20


<210>  54
<211>  39
<212>  PRT
<213>  Sarcotoxin IA Fly

<400>  54

Gly Trp Leu Lys Lys Ile Gly Lys Lys Ile Glu Arg Val Gly Gln His
1               5                   10                  15

Thr Arg Asp Ala Thr Ile Gln Gly Leu Gly Ile Ala Gln Gln Ala Ala
            20                  25                  30

Asn Val Ala Ala Thr Ala Arg
        35

<210>  55
<211>  17
<212>  PRT
<213>  Apidaecin Honey bee

<400>  55

Ala Asn Arg Pro Val Tyr Ile Pro Pro Pro Arg Pro Pro His Pro Arg
1               5                   10                  15

Leu

<210>  56
<211>  24
<212>  PRT
<213>  Ascaphine 5 Frog

<400>  56

Gly Ile Lys Asp Trp Ile Lys Gly Ala Ala Lys Lys Leu Ile Lys Thr
1               5                   10                  15

Val Ala Ser His Ile Ala Asn Gln
            20

<210>  57
<211>  22
<212>  PRT
<213>  Nigrocine 2 Frog

<400>  57

Gly Leu Leu Ser Lys Val Leu Gly Val Gly Lys Lys Val Leu Cys Gly
1               5                   10                  15

Val Ser Gly Leu Val Cys
            20

<210>  58
<211>  24
<212>  PRT
<213>  Pseudin 1 Rana Frog

<400>  58

Gly Leu Asn Thr Leu Lys Lys Val Phe Gln Gly Leu His Glu Ala Ile
1               5                   10                  15

Lys Leu Ile Asn Asn His Val Gln
            20

<210>  59
<211>  18
<212>  PRT
<213>  Ranalexin Frog

<400>  59

Phe Leu Gly Gly Leu Ile Val Pro Ala Met Ile Cys Ala Val Thr Lys
1               5                   10                  15

Lys Cys

<210>  60
<211>  26
<212>  PRT
<213>  Melittin bee

<400>  60

Gly Ile Gly Ala Val Leu Lys Val Leu Thr Thr Gly Leu Pro Ala Leu
1               5                   10                  15

Ile Ser Trp Ile Lys Arg Lys Arg Gln Gln
            20                  25

<210>  61
<211>  25
<212>  PRT
<213>  Lycotoxin 1 Spider

<400>  61

Ile Trp Leu Thr Ala Leu Lys Phe Leu Gly Lys His Ala Ala Lys Lys
1               5                   10                  15

Leu Ala Lys Gln Gln Leu Ser Lys Leu
            20                  25

<210>  62
<211>  19
<212>  PRT
<213>  Parasin 1 Fish

<400>  62

Lys Gly Arg Gly Lys Gln Gly Gly Lys Val Arg Ala Lys Ala Lys Thr
1               5                   10                  15

Arg Ser Ser

<210>  63
<211>  34
<212>  PRT
<213>  horseshoe crab

<400>  63

Gly Phe Lys Leu Lys Gly Met Ala Arg Ile Ser Cys Leu Pro Asn Gly
1               5                   10                  15

Gln Trp Ser Asn Phe Pro Pro Lys Cys Ile Arg Glu Cys Ala Met Val
            20                  25                  30

Ser Ser

<210>  64
<211>  27
<212>  PRT
<213>  artificial

<220>
<223>  synthetic sequence

<400>  64

Lys Arg Trp Val Lys Arg Val Lys Arg Val Lys Arg Trp Val Lys Arg
1               5                   10                  15

Val Val Arg Val Val Lys Arg Trp Val Lys Arg
            20                  25

<210>  65
<211>  18
<212>  PRT
<213>  artificial

<220>
<223>  synthetic sequence

<400>  65

Gly Phe Phe Ile Pro Ala Val Ile Leu Pro Ser Ile Ala Phe Leu Ile
1               5                   10                  15

Val Pro

<210>  66
<211>  5
<212>  PRT
<213>  artificial

<220>
<223>  synthetic sequence

<400>  66

Phe Phe Val Ala Pro
1               5

<210>  67
<211>  39
<212>  PRT
<213>  toad

<400>  67

Ala Gly Arg Gly Lys Gln Gly Gly Lys Val Arg Ala Lys Ala Lys Thr
1               5                   10                  15

Arg Ser Ser Arg Ala Gly Leu Gln Phe Pro Val Gly Arg Val His Arg
            20                  25                  30

Leu Leu Arg Lys Gly Asn Tyr
        35

<210>  68
<211>  13
<212>  PRT
<213>  bacteriophage

<400>  68

Pro Asn Arg Ala Lys Arg Val Ile Thr Thr Phe Arg Thr
1               5                   10

<210>  69
<211>  25
<212>  PRT
<213>  artificial

<220>
<223>  synthetic sequence

<400>  69

Gly Lys Pro Gly Trp Leu Ile Lys Lys Ala Leu Val Phe Lys Lys Leu
1               5                   10                  15

Ile Arg Arg Pro Leu Lys Arg Leu Ala
            20                  25

<210>  70
<211>  341
<212>  PRT
<213>  artificial

<220>
<223>  synthetic sequence

<400>  70

Phe Phe Val Ala Pro Lys Arg Lys Lys Arg Lys Lys Arg Lys Lys Asn
1               5                   10                  15

Ser Glu Lys Asn Ala Ser Ile Ile Met Ser Ile Gln Arg Thr Leu Ala
            20                  25                  30

Ser Leu Ser Leu Tyr Gly Gly Arg Ile Asp Gly Leu Phe Gly Glu Lys
        35                  40                  45

Cys Arg Gly Ala Ile Ile Leu Met Leu Asn Lys Val Tyr Pro Asn Phe
    50                  55                  60

Ser Thr Asn Lys Leu Pro Ser Asn Thr Tyr Glu Ala Glu Ser Val Phe
65                  70                  75                  80

Thr Phe Leu Gln Thr Ala Leu Ala Gly Val Gly Leu Tyr Thr Ile Thr
                85                  90                  95

Ile Asp Gly Lys Trp Gly Gly Thr Ser Gln Gly Ala Ile Asp Ala Leu
            100                 105                 110

Val Lys Ser Tyr Arg Gln Ile Thr Glu Ala Glu Arg Ala Gly Ser Thr
        115                 120                 125

Leu Pro Leu Gly Leu Ala Thr Val Met Ser Lys His Met Ser Ile Glu
    130                 135                 140

Gln Leu Arg Ala Met Leu Pro Thr Asp Arg Gln Gly Tyr Ala Glu Val
145                 150                 155                 160

Tyr Ile Asp Pro Leu Asn Glu Thr Met Asp Ile Phe Glu Ile Asn Thr
                165                 170                 175

Pro Leu Arg Ile Ala His Phe Met Ala Gln Ile Leu His Glu Thr Ala
            180                 185                 190

Cys Phe Lys Tyr Thr Glu Glu Leu Ala Ser Gly Lys Ala Tyr Glu Gly
        195                 200                 205

Arg Ala Asp Leu Gly Asn Thr Arg Pro Gly Asp Gly Pro Leu Phe Lys
    210                 215                 220

Gly Arg Gly Leu Leu Gln Ile Thr Gly Arg Leu Asn Tyr Val Lys Cys
225                 230                 235                 240

Gln Val Tyr Leu Arg Glu Lys Leu Lys Asp Pro Thr Phe Asp Ile Thr
                245                 250                 255

Ser Ser Val Thr Cys Ala Gln Gln Leu Ser Glu Ser Pro Leu Leu Ala
            260                 265                 270

Ala Leu Ala Ser Gly Tyr Phe Trp Arg Phe Ile Lys Pro Lys Leu Asn
        275                 280                 285

Glu Thr Ala Asp Lys Asp Asp Ile Tyr Trp Val Ser Val Tyr Val Asn
    290                 295                 300

Gly Tyr Ala Lys Gln Ala Asn Pro Tyr Tyr Pro Asn Arg Asp Lys Glu
305                 310                 315                 320

Pro Asn His Met Lys Glu Arg Val Gln Met Leu Ala Val Thr Lys Lys
                325                 330                 335

Ala His Gly Ile Val
            340

<210>  71
<211>  349
<212>  PRT
<213>  artificial

<220>
<223>  synthetic sequence

<400>  71

Pro Asn Arg Ala Lys Arg Val Ile Thr Thr Phe Arg Thr Lys Arg Lys
1               5                   10                  15

Lys Arg Lys Lys Arg Lys Lys Asn Ser Glu Lys Asn Ala Ser Ile Ile
            20                  25                  30

Met Ser Ile Gln Arg Thr Leu Ala Ser Leu Ser Leu Tyr Gly Gly Arg
        35                  40                  45

Ile Asp Gly Leu Phe Gly Glu Lys Cys Arg Gly Ala Ile Ile Leu Met
    50                  55                  60

Leu Asn Lys Val Tyr Pro Asn Phe Ser Thr Asn Lys Leu Pro Ser Asn
65                  70                  75                  80

Thr Tyr Glu Ala Glu Ser Val Phe Thr Phe Leu Gln Thr Ala Leu Ala
                85                  90                  95

Gly Val Gly Leu Tyr Thr Ile Thr Ile Asp Gly Lys Trp Gly Gly Thr
            100                 105                 110

Ser Gln Gly Ala Ile Asp Ala Leu Val Lys Ser Tyr Arg Gln Ile Thr
        115                 120                 125

Glu Ala Glu Arg Ala Gly Ser Thr Leu Pro Leu Gly Leu Ala Thr Val
    130                 135                 140

Met Ser Lys His Met Ser Ile Glu Gln Leu Arg Ala Met Leu Pro Thr
145                 150                 155                 160

Asp Arg Gln Gly Tyr Ala Glu Val Tyr Ile Asp Pro Leu Asn Glu Thr
                165                 170                 175

Met Asp Ile Phe Glu Ile Asn Thr Pro Leu Arg Ile Ala His Phe Met
            180                 185                 190

Ala Gln Ile Leu His Glu Thr Ala Cys Phe Lys Tyr Thr Glu Glu Leu
        195                 200                 205

Ala Ser Gly Lys Ala Tyr Glu Gly Arg Ala Asp Leu Gly Asn Thr Arg

210                 215                 220

Pro Gly Asp Gly Pro Leu Phe Lys Gly Arg Gly Leu Leu Gln Ile Thr
225                 230                 235                 240

Gly Arg Leu Asn Tyr Val Lys Cys Gln Val Tyr Leu Arg Glu Lys Leu
                245                 250                 255

Lys Asp Pro Thr Phe Asp Ile Thr Ser Ser Val Thr Cys Ala Gln Gln
            260                 265                 270

Leu Ser Glu Ser Pro Leu Leu Ala Ala Leu Ala Ser Gly Tyr Phe Trp
        275                 280                 285

Arg Phe Ile Lys Pro Lys Leu Asn Glu Thr Ala Asp Lys Asp Asp Ile
    290                 295                 300

Tyr Trp Val Ser Val Tyr Val Asn Gly Tyr Ala Lys Gln Ala Asn Pro
305                 310                 315                 320

Tyr Tyr Pro Asn Arg Asp Lys Glu Pro Asn His Met Lys Glu Arg Val
                325                 330                 335

Gln Met Leu Ala Val Thr Lys Lys Ala His Gly Ile Val
            340                 345

<210>  72
<211>  354
<212>  PRT
<213>  artificial

<220>
<223>  synthetic sequence

<400>  72

Gly Phe Phe Ile Pro Ala Val Ile Leu Pro Ser Ile Ala Phe Leu Ile
1               5                   10                  15

Val Pro Lys Arg Lys Lys Arg Lys Lys Arg Lys Lys Asn Ser Glu Lys
            20                  25                  30

Asn Ala Ser Ile Ile Met Ser Ile Gln Arg Thr Leu Ala Ser Leu Ser
        35                  40                  45

Leu Tyr Gly Gly Arg Ile Asp Gly Leu Phe Gly Glu Lys Cys Arg Gly
    50                  55                  60

Ala Ile Ile Leu Met Leu Asn Lys Val Tyr Pro Asn Phe Ser Thr Asn
65                  70                  75                  80

Lys Leu Pro Ser Asn Thr Tyr Glu Ala Glu Ser Val Phe Thr Phe Leu
                85                  90                  95

Gln Thr Ala Leu Ala Gly Val Gly Leu Tyr Thr Ile Thr Ile Asp Gly
            100                 105                 110

Lys Trp Gly Gly Thr Ser Gln Gly Ala Ile Asp Ala Leu Val Lys Ser
        115                 120                 125

Tyr Arg Gln Ile Thr Glu Ala Glu Arg Ala Gly Ser Thr Leu Pro Leu
    130                 135                 140

Gly Leu Ala Thr Val Met Ser Lys His Met Ser Ile Glu Gln Leu Arg
145                 150                 155                 160

Ala Met Leu Pro Thr Asp Arg Gln Gly Tyr Ala Glu Val Tyr Ile Asp
                165                 170                 175

Pro Leu Asn Glu Thr Met Asp Ile Phe Glu Ile Asn Thr Pro Leu Arg
            180                 185                 190

Ile Ala His Phe Met Ala Gln Ile Leu His Glu Thr Ala Cys Phe Lys
        195                 200                 205

Tyr Thr Glu Glu Leu Ala Ser Gly Lys Ala Tyr Glu Gly Arg Ala Asp
    210                 215                 220

Leu Gly Asn Thr Arg Pro Gly Asp Gly Pro Leu Phe Lys Gly Arg Gly
225                 230                 235                 240

Leu Leu Gln Ile Thr Gly Arg Leu Asn Tyr Val Lys Cys Gln Val Tyr
                245                 250                 255

Leu Arg Glu Lys Leu Lys Asp Pro Thr Phe Asp Ile Thr Ser Ser Val
            260                 265                 270

Thr Cys Ala Gln Gln Leu Ser Glu Ser Pro Leu Leu Ala Ala Leu Ala
        275                 280                 285

Ser Gly Tyr Phe Trp Arg Phe Ile Lys Pro Lys Leu Asn Glu Thr Ala
    290                 295                 300

Asp Lys Asp Asp Ile Tyr Trp Val Ser Val Tyr Val Asn Gly Tyr Ala
305                 310                 315                 320

Lys Gln Ala Asn Pro Tyr Tyr Pro Asn Arg Asp Lys Glu Pro Asn His
                325                 330                 335

Met Lys Glu Arg Val Gln Met Leu Ala Val Thr Lys Lys Ala His Gly
            340                 345                 350

Ile Val

<210>  73
<211>  361
<212>  PRT
<213>  artificial

<220>
<223>  synthetic sequence

<400>  73

Gly Lys Pro Gly Trp Leu Ile Lys Lys Ala Leu Val Phe Lys Lys Leu
1               5                   10                  15

Ile Arg Arg Pro Leu Lys Arg Leu Ala Lys Arg Lys Lys Arg Lys Lys
            20                  25                  30

Arg Lys Lys Asn Ser Glu Lys Asn Ala Ser Ile Ile Met Ser Ile Gln
        35                  40                  45

Arg Thr Leu Ala Ser Leu Ser Leu Tyr Gly Gly Arg Ile Asp Gly Leu
    50                  55                  60

Phe Gly Glu Lys Cys Arg Gly Ala Ile Ile Leu Met Leu Asn Lys Val
65                  70                  75                  80

Tyr Pro Asn Phe Ser Thr Asn Lys Leu Pro Ser Asn Thr Tyr Glu Ala
                85                  90                  95

Glu Ser Val Phe Thr Phe Leu Gln Thr Ala Leu Ala Gly Val Gly Leu
            100                 105                 110

Tyr Thr Ile Thr Ile Asp Gly Lys Trp Gly Gly Thr Ser Gln Gly Ala
        115                 120                 125

Ile Asp Ala Leu Val Lys Ser Tyr Arg Gln Ile Thr Glu Ala Glu Arg
    130                 135                 140

Ala Gly Ser Thr Leu Pro Leu Gly Leu Ala Thr Val Met Ser Lys His
145                 150                 155                 160

Met Ser Ile Glu Gln Leu Arg Ala Met Leu Pro Thr Asp Arg Gln Gly
                165                 170                 175

Tyr Ala Glu Val Tyr Ile Asp Pro Leu Asn Glu Thr Met Asp Ile Phe
            180                 185                 190

Glu Ile Asn Thr Pro Leu Arg Ile Ala His Phe Met Ala Gln Ile Leu
        195                 200                 205

His Glu Thr Ala Cys Phe Lys Tyr Thr Glu Glu Leu Ala Ser Gly Lys
    210                 215                 220

Ala Tyr Glu Gly Arg Ala Asp Leu Gly Asn Thr Arg Pro Gly Asp Gly

225                 230                 235                 240

Pro Leu Phe Lys Gly Arg Gly Leu Leu Gln Ile Thr Gly Arg Leu Asn
                245                 250                 255

Tyr Val Lys Cys Gln Val Tyr Leu Arg Glu Lys Leu Lys Asp Pro Thr
            260                 265                 270

Phe Asp Ile Thr Ser Ser Val Thr Cys Ala Gln Gln Leu Ser Glu Ser
        275                 280                 285

Pro Leu Leu Ala Ala Leu Ala Ser Gly Tyr Phe Trp Arg Phe Ile Lys
    290                 295                 300

Pro Lys Leu Asn Glu Thr Ala Asp Lys Asp Asp Ile Tyr Trp Val Ser
305                 310                 315                 320

Val Tyr Val Asn Gly Tyr Ala Lys Gln Ala Asn Pro Tyr Tyr Pro Asn
                325                 330                 335

Arg Asp Lys Glu Pro Asn His Met Lys Glu Arg Val Gln Met Leu Ala
            340                 345                 350

Val Thr Lys Lys Ala His Gly Ile Val
        355                 360

<210>  74
<211>  355
<212>  PRT
<213>  artificial

<220>
<223>  synthetic sequence

<400>  74

Lys Gly Arg Gly Lys Gln Gly Gly Lys Val Arg Ala Lys Ala Lys Thr
1               5                   10                  15

Arg Ser Ser Lys Arg Lys Lys Arg Lys Lys Arg Lys Lys Asn Ser Glu
            20                  25                  30

Lys Asn Ala Ser Ile Ile Met Ser Ile Gln Arg Thr Leu Ala Ser Leu
        35                  40                  45

Ser Leu Tyr Gly Gly Arg Ile Asp Gly Leu Phe Gly Glu Lys Cys Arg
    50                  55                  60

Gly Ala Ile Ile Leu Met Leu Asn Lys Val Tyr Pro Asn Phe Ser Thr
65                  70                  75                  80

Asn Lys Leu Pro Ser Asn Thr Tyr Glu Ala Glu Ser Val Phe Thr Phe
                85                  90                  95

Leu Gln Thr Ala Leu Ala Gly Val Gly Leu Tyr Thr Ile Thr Ile Asp
            100                 105                 110

Gly Lys Trp Gly Gly Thr Ser Gln Gly Ala Ile Asp Ala Leu Val Lys
        115                 120                 125

Ser Tyr Arg Gln Ile Thr Glu Ala Glu Arg Ala Gly Ser Thr Leu Pro
    130                 135                 140

Leu Gly Leu Ala Thr Val Met Ser Lys His Met Ser Ile Glu Gln Leu
145                 150                 155                 160

Arg Ala Met Leu Pro Thr Asp Arg Gln Gly Tyr Ala Glu Val Tyr Ile
                165                 170                 175

Asp Pro Leu Asn Glu Thr Met Asp Ile Phe Glu Ile Asn Thr Pro Leu
            180                 185                 190

Arg Ile Ala His Phe Met Ala Gln Ile Leu His Glu Thr Ala Cys Phe
        195                 200                 205

Lys Tyr Thr Glu Glu Leu Ala Ser Gly Lys Ala Tyr Glu Gly Arg Ala
    210                 215                 220

Asp Leu Gly Asn Thr Arg Pro Gly Asp Gly Pro Leu Phe Lys Gly Arg
225                 230                 235                 240

Gly Leu Leu Gln Ile Thr Gly Arg Leu Asn Tyr Val Lys Cys Gln Val
                245                 250                 255

Tyr Leu Arg Glu Lys Leu Lys Asp Pro Thr Phe Asp Ile Thr Ser Ser
            260                 265                 270

Val Thr Cys Ala Gln Gln Leu Ser Glu Ser Pro Leu Leu Ala Ala Leu
        275                 280                 285

Ala Ser Gly Tyr Phe Trp Arg Phe Ile Lys Pro Lys Leu Asn Glu Thr
    290                 295                 300

Ala Asp Lys Asp Asp Ile Tyr Trp Val Ser Val Tyr Val Asn Gly Tyr
305                 310                 315                 320

Ala Lys Gln Ala Asn Pro Tyr Tyr Pro Asn Arg Asp Lys Glu Pro Asn
                325                 330                 335

His Met Lys Glu Arg Val Gln Met Leu Ala Val Thr Lys Lys Ala His
            340                 345                 350

Gly Ile Val
        355

<210>  75
<211>  361
<212>  PRT
<213>  artificial

<220>
<223>  synthetic sequence

<400>  75

Ile Trp Leu Thr Ala Leu Lys Phe Leu Gly Lys His Ala Ala Lys Lys
1               5                   10                  15

Leu Ala Lys Gln Gln Leu Ser Lys Leu Lys Arg Lys Lys Arg Lys Lys
            20                  25                  30

Arg Lys Lys Asn Ser Glu Lys Asn Ala Ser Ile Ile Met Ser Ile Gln
        35                  40                  45

Arg Thr Leu Ala Ser Leu Ser Leu Tyr Gly Gly Arg Ile Asp Gly Leu
    50                  55                  60

Phe Gly Glu Lys Cys Arg Gly Ala Ile Ile Leu Met Leu Asn Lys Val
65                  70                  75                  80

Tyr Pro Asn Phe Ser Thr Asn Lys Leu Pro Ser Asn Thr Tyr Glu Ala
                85                  90                  95

Glu Ser Val Phe Thr Phe Leu Gln Thr Ala Leu Ala Gly Val Gly Leu
            100                 105                 110

Tyr Thr Ile Thr Ile Asp Gly Lys Trp Gly Gly Thr Ser Gln Gly Ala
        115                 120                 125

Ile Asp Ala Leu Val Lys Ser Tyr Arg Gln Ile Thr Glu Ala Glu Arg
    130                 135                 140

Ala Gly Ser Thr Leu Pro Leu Gly Leu Ala Thr Val Met Ser Lys His
145                 150                 155                 160

Met Ser Ile Glu Gln Leu Arg Ala Met Leu Pro Thr Asp Arg Gln Gly
                165                 170                 175

Tyr Ala Glu Val Tyr Ile Asp Pro Leu Asn Glu Thr Met Asp Ile Phe
            180                 185                 190

Glu Ile Asn Thr Pro Leu Arg Ile Ala His Phe Met Ala Gln Ile Leu
        195                 200                 205

His Glu Thr Ala Cys Phe Lys Tyr Thr Glu Glu Leu Ala Ser Gly Lys
    210                 215                 220

Ala Tyr Glu Gly Arg Ala Asp Leu Gly Asn Thr Arg Pro Gly Asp Gly

225                 230                 235                 240

Pro Leu Phe Lys Gly Arg Gly Leu Leu Gln Ile Thr Gly Arg Leu Asn
                245                 250                 255

Tyr Val Lys Cys Gln Val Tyr Leu Arg Glu Lys Leu Lys Asp Pro Thr
            260                 265                 270

Phe Asp Ile Thr Ser Ser Val Thr Cys Ala Gln Gln Leu Ser Glu Ser
        275                 280                 285

Pro Leu Leu Ala Ala Leu Ala Ser Gly Tyr Phe Trp Arg Phe Ile Lys
    290                 295                 300

Pro Lys Leu Asn Glu Thr Ala Asp Lys Asp Asp Ile Tyr Trp Val Ser
305                 310                 315                 320

Val Tyr Val Asn Gly Tyr Ala Lys Gln Ala Asn Pro Tyr Tyr Pro Asn
                325                 330                 335

Arg Asp Lys Glu Pro Asn His Met Lys Glu Arg Val Gln Met Leu Ala
            340                 345                 350

Val Thr Lys Lys Ala His Gly Ile Val
        355                 360

<210>  76
<211>  341
<212>  PRT
<213>  artificial

<220>
<223>  synthetic sequence

<400>  76

Lys Arg Lys Lys Arg Lys Lys Arg Lys Phe Phe Val Ala Pro Lys Asn
1               5                   10                  15

Ser Glu Lys Asn Ala Ser Ile Ile Met Ser Ile Gln Arg Thr Leu Ala
            20                  25                  30

Ser Leu Ser Leu Tyr Gly Gly Arg Ile Asp Gly Leu Phe Gly Glu Lys
        35                  40                  45

Cys Arg Gly Ala Ile Ile Leu Met Leu Asn Lys Val Tyr Pro Asn Phe
    50                  55                  60

Ser Thr Asn Lys Leu Pro Ser Asn Thr Tyr Glu Ala Glu Ser Val Phe
65                  70                  75                  80

Thr Phe Leu Gln Thr Ala Leu Ala Gly Val Gly Leu Tyr Thr Ile Thr
                85                  90                  95

Ile Asp Gly Lys Trp Gly Gly Thr Ser Gln Gly Ala Ile Asp Ala Leu
            100                 105                 110

Val Lys Ser Tyr Arg Gln Ile Thr Glu Ala Glu Arg Ala Gly Ser Thr
        115                 120                 125

Leu Pro Leu Gly Leu Ala Thr Val Met Ser Lys His Met Ser Ile Glu
    130                 135                 140

Gln Leu Arg Ala Met Leu Pro Thr Asp Arg Gln Gly Tyr Ala Glu Val
145                 150                 155                 160

Tyr Ile Asp Pro Leu Asn Glu Thr Met Asp Ile Phe Glu Ile Asn Thr
                165                 170                 175

Pro Leu Arg Ile Ala His Phe Met Ala Gln Ile Leu His Glu Thr Ala
            180                 185                 190

Cys Phe Lys Tyr Thr Glu Glu Leu Ala Ser Gly Lys Ala Tyr Glu Gly
        195                 200                 205

Arg Ala Asp Leu Gly Asn Thr Arg Pro Gly Asp Gly Pro Leu Phe Lys
    210                 215                 220

Gly Arg Gly Leu Leu Gln Ile Thr Gly Arg Leu Asn Tyr Val Lys Cys
225                 230                 235                 240

Gln Val Tyr Leu Arg Glu Lys Leu Lys Asp Pro Thr Phe Asp Ile Thr
                245                 250                 255

Ser Ser Val Thr Cys Ala Gln Gln Leu Ser Glu Ser Pro Leu Leu Ala
            260                 265                 270

Ala Leu Ala Ser Gly Tyr Phe Trp Arg Phe Ile Lys Pro Lys Leu Asn
        275                 280                 285

Glu Thr Ala Asp Lys Asp Asp Ile Tyr Trp Val Ser Val Tyr Val Asn
    290                 295                 300

Gly Tyr Ala Lys Gln Ala Asn Pro Tyr Tyr Pro Asn Arg Asp Lys Glu
305                 310                 315                 320

Pro Asn His Met Lys Glu Arg Val Gln Met Leu Ala Val Thr Lys Lys
                325                 330                 335

Ala His Gly Ile Val
            340

<210>  77
<211>  349
<212>  PRT

<213>  artificial

<220>
<223>  synthetic sequence

<400>  77

Lys Arg Lys Lys Arg Lys Lys Arg Lys Pro Asn Arg Ala Lys Arg Val
1               5                   10                  15

Ile Thr Thr Phe Arg Thr Lys Asn Ser Glu Lys Asn Ala Ser Ile Ile
            20                  25                  30

Met Ser Ile Gln Arg Thr Leu Ala Ser Leu Ser Leu Tyr Gly Gly Arg
        35                  40                  45

Ile Asp Gly Leu Phe Gly Glu Lys Cys Arg Gly Ala Ile Ile Leu Met
    50                  55                  60

Leu Asn Lys Val Tyr Pro Asn Phe Ser Thr Asn Lys Leu Pro Ser Asn
65                  70                  75                  80

Thr Tyr Glu Ala Glu Ser Val Phe Thr Phe Leu Gln Thr Ala Leu Ala
                85                  90                  95

Gly Val Gly Leu Tyr Thr Ile Thr Ile Asp Gly Lys Trp Gly Gly Thr
            100                 105                 110

Ser Gln Gly Ala Ile Asp Ala Leu Val Lys Ser Tyr Arg Gln Ile Thr
        115                 120                 125

Glu Ala Glu Arg Ala Gly Ser Thr Leu Pro Leu Gly Leu Ala Thr Val
    130                 135                 140

Met Ser Lys His Met Ser Ile Glu Gln Leu Arg Ala Met Leu Pro Thr
145                 150                 155                 160

Asp Arg Gln Gly Tyr Ala Glu Val Tyr Ile Asp Pro Leu Asn Glu Thr
                165                 170                 175

Met Asp Ile Phe Glu Ile Asn Thr Pro Leu Arg Ile Ala His Phe Met
            180                 185                 190

Ala Gln Ile Leu His Glu Thr Ala Cys Phe Lys Tyr Thr Glu Glu Leu
        195                 200                 205

Ala Ser Gly Lys Ala Tyr Glu Gly Arg Ala Asp Leu Gly Asn Thr Arg
    210                 215                 220

Pro Gly Asp Gly Pro Leu Phe Lys Gly Arg Gly Leu Leu Gln Ile Thr
225                 230                 235                 240

Gly Arg Leu Asn Tyr Val Lys Cys Gln Val Tyr Leu Arg Glu Lys Leu

245                 250                 255

Lys Asp Pro Thr Phe Asp Ile Thr Ser Ser Val Thr Cys Ala Gln Gln
            260                 265                 270

Leu Ser Glu Ser Pro Leu Leu Ala Ala Leu Ala Ser Gly Tyr Phe Trp
        275                 280                 285

Arg Phe Ile Lys Pro Lys Leu Asn Glu Thr Ala Asp Lys Asp Asp Ile
    290                 295                 300

Tyr Trp Val Ser Val Tyr Val Asn Gly Tyr Ala Lys Gln Ala Asn Pro
305                 310                 315                 320

Tyr Tyr Pro Asn Arg Asp Lys Glu Pro Asn His Met Lys Glu Arg Val
                325                 330                 335

Gln Met Leu Ala Val Thr Lys Lys Ala His Gly Ile Val
            340                 345

<210>  78
<211>  39
<212>  PRT
<213>  Buforin I Toad

<400>  78

Ala Gly Arg Gly Lys Gln Gly Gly Lys Val Arg Ala Lys Ala Lys Thr
1               5                   10                  15

Arg Ser Ser Arg Ala Gly Leu Gln Phe Pro Val Gly Arg Val His Arg
            20                  25                  30

Leu Leu Arg Lys Gly Asn Tyr
        35

<210>  79
<211>  34
<212>  PRT
<213>  Dermaseptin 1 Frog

<400>  79

Ala Leu Trp Lys Thr Met Leu Lys Lys Leu Gly Thr Met Ala Leu His
1               5                   10                  15

Ala Gly Lys Ala Ala Leu Gly Ala Ala Ala Asp Thr Ile Ser Gln Gly
            20                  25                  30

Thr Gln

<210>  80
<211>  12
<212>  PRT

<213>  Bactenecin 1 Cow

<400>  80

Arg Leu Cys Arg Ile Val Val Ile Arg Val Cys Arg
1               5                   10


<210>  81
<211>  21
<212>  PRT
<213>  Thanatin Insect

<400>  81

Gly Ser Lys Lys Pro Val Pro Ile Ile Tyr Cys Asn Arg Arg Thr Gly
1               5                   10                  15


Lys Cys Gln Arg Met
            20


<210>  82
<211>  19
<212>  PRT
<213>  Brevinin 1T Rana frogs

<400>  82

Val Asn Pro Ile Ile Leu Gly Val Leu Pro Lys Val Cys Leu Ile Thr
1               5                   10                  15


Lys Lys Cys



<210>  83
<211>  26
<212>  PRT
<213>  Ranateurin 1 Rana frog

<400>  83

Ser Met Leu Ser Val Leu Lys Asn Leu Gly Lys Val Gly Leu Gly Phe
1               5                   10                  15


Val Ala Cys Lys Ile Asn Ile Lys Gln Cys
            20                  25


<210>  84
<211>  46
<212>  PRT
<213>  Esculentin 1 Rana frogs

<400>  84

Gly Ile Phe Ser Lys Leu Gly Arg Lys Lys Ile Lys Asn Leu Leu Ile
1               5                   10                  15


Ser Gly Leu Lys Asn Val Gly Lys Glu Val Gly Met Asp Val Val Arg
            20                  25                  30

Thr Gly Ile Lys Ile Ala Gly Cys Lys Ile Lys Gly Glu Cys
        35                  40                  45

<210>  85
<211>  17
<212>  PRT
<213>  Tachyplesin Horseshoe crab

<400>  85

Arg Trp Cys Phe Arg Val Cys Tyr Arg Gly Ile Cys Tyr Arg Lys Cys
1               5                   10                  15

Arg

<210>  86
<211>  25
<212>  PRT
<213>  Androctonin Scorpion

<400>  86

Arg Ser Val Cys Arg Gln Ile Lys Ile Cys Arg Arg Arg Gly Gly Cys
1               5                   10                  15

Tyr Tyr Lys Cys Thr Asn Arg Pro Tyr
            20                  25

<210>  87
<211>  30
<212>  PRT
<213>  alpha-defensin human

<400>  87

Asp Cys Tyr Cys Arg Ile Pro Ala Cys Ile Ala Gly Glu Arg Arg Tyr
1               5                   10                  15

Gly Thr Cys Ile Tyr Gln Gly Arg Leu Trp Ala Phe Cys Cys
            20                  25                  30

<210>  88
<211>  38
<212>  PRT
<213>  beta-defensin cow

<400>  88

Asn Pro Val Ser Cys Val Arg Asn Lys Gly Ile Cys Val Pro Ile Arg
1               5                   10                  15

Cys Pro Gly Ser Met Lys Gln Ile Gly Thr Cys Val Gly Arg Ala Val
            20                  25                  30

Lys Cys Cys Arg Lys Lys
        35

<210>  89
<211>  18
<212>  PRT
<213>  theta-defensin monkey

<400>  89

Gly Phe Cys Arg Cys Leu Cys Arg Arg Gly Val Cys Arg Cys Ile Cys
1               5                   10                  15

Thr Arg

<210>  90
<211>  40
<212>  PRT
<213>  defensin (sapecin A) insect

<400>  90

Ala Thr Cys Asp Leu Leu Ser Gly Thr Gly Ile Asn His Ser Ala Cys
1               5                   10                  15

Ala Ala His Cys Leu Leu Arg Gly Asn Arg Gly Gly Tyr Cys Asn Gly
            20                  25                  30

Lys Ala Val Cys Val Cys Arg Asn
        35                  40

<210>  91
<211>  46
<212>  PRT
<213>  Thionin (crambin) plant

<400>  91

Thr Thr Cys Cys Pro Ser Ile Val Ala Arg Ser Asn Phe Asn Val Cys
1               5                   10                  15

Arg Ile Pro Gly Thr Pro Glu Ala Ile Cys Ala Thr Tyr Thr Gly Cys
            20                  25                  30

Ile Ile Ile Pro Gly Ala Thr Cys Pro Gly Asp Tyr Ala Asn
        35                  40                  45

<210>  92
<211>  50
<212>  PRT
<213>  defensin from radish

<400>  92

Gln Lys Leu Cys Gln Arg Pro Ser Gly Thr Trp Ser Gly Val Cys Gly
1               5                   10                  15

Asn Asn Asn Ala Cys Lys Asn Gln Cys Ile Arg Leu Glu Lys Ala Arg
            20                  25                  30

His Gly Ser Cys Asn Tyr Val Phe Pro Ala His Cys Ile Cys Tyr Phe
        35                  40                  45

Pro Cys
    50

<210>  93
<211>  44
<212>  PRT
<213>  Drosomycin Drosophila

<400>  93

Asp Cys Leu Ser Gly Arg Tyr Lys Gly Pro Cys Ala Val Trp Asp Asn
1               5                   10                  15

Glu Thr Cys Arg Arg Val Cys Lys Glu Glu Gly Arg Ser Ser Gly His
            20                  25                  30

Cys Ser Pro Ser Leu Lys Cys Trp Cys Glu Gly Cys
        35                  40

<210>  94
<211>  25
<212>  PRT
<213>  Hepcidin Human

<400>  94

Asp Thr His Phe Pro Ile Cys Ile Phe Cys Cys Gly Cys Cys His Arg
1               5                   10                  15

Ser Lys Cys Gly Met Cys Cys Lys Thr
            20                  25

<210>  95
<211>  44
<212>  PRT
<213>  Bac 5 Cow

<400>  95

Arg Phe Arg Pro Pro Ile Arg Arg Pro Pro Ile Arg Pro Pro Phe Tyr
1               5                   10                  15

Pro Pro Phe Arg Pro Pro Ile Arg Pro Pro Ile Phe Pro Pro Ile Arg
            20                  25                  30

Pro Pro Phe Arg Pro Pro Leu Gly Arg Pro Phe Pro
        35                  40

<210>  96
<211>  39
<212>  PRT
<213>  PR-39 Pig

<400>  96

Arg Arg Arg Pro Arg Pro Pro Tyr Leu Pro Arg Pro Arg Pro Pro Pro
1               5                   10                  15

Phe Phe Pro Pro Arg Leu Pro Pro Arg Ile Pro Pro Gly Phe Pro Pro
            20                  25                  30

Arg Phe Pro Pro Arg Phe Pro
        35

<210>  97
<211>  20
<212>  PRT
<213>  Pyrrhocoricin Insect

<400>  97

Val Asp Lys Gly Ser Tyr Leu Pro Arg Pro Thr Pro Pro Arg Pro Ile
1               5                   10                  15

Tyr Asn Arg Asn
            20

<210>  98
<211>  24
<212>  PRT
<213>  Histatin 5 human

<400>  98

Asp Ser His Ala Lys Arg His His Gly Tyr Lys Arg Lys Phe His Glu
1               5                   10                  15

Lys His His Ser His Arg Gly Tyr
            20

<210>  99
<211>  39
<212>  DNA
<213>  artificial

<220>
<223>  synthetic sequence

<400>  99
ttggaatggg gagcccgaac cgtgcaaaac gtgtaatca                              39

<210>  100
<211>  39
<212>  DNA
<213>  artificial

<220>
<223>  synthetic sequence

<400>  100
gtttggagga gccggtacgg aaggtggtga ttacacgtt                              39

<210>  101
<211>  39

<212>  DNA
<213>  artificial

<220>
<223>  synthetic sequence

<400>  101
ttatgggctt cttcatcccg gcagtaatcc tgccctcca                              39


<210>  102
<211>  46
<212>  DNA
<213>  artificial

<220>
<223>  synthetic sequence

<400>  102
gtttggagga gcccggtacg atcaggaatg cgatggaggg caggat                      46


<210>  103
<211>  47
<212>  DNA
<213>  artificial

<220>
<223>  synthetic sequence

<400>  103
ttatgggcaa accgggctgg ctgatcaaaa ggcactggta ttcaaga                     47


<210>  104
<211>  57
<212>  DNA
<213>  artificial

<220>
<223>  synthetic sequence

<400>  104
gtttggagga gcctgccagt ctcttcagcg gacgacggat cagtttcttg aatacca          57


<210>  105
<211>  55
<212>  DNA
<213>  artificial

<220>
<223>  synthetic sequence

<400>  105
ggaatgggga gcatctggct gaccgcactg aaattcctcg gcaaacacgc cgcaa            55


<210>  106
<211>  57
<212>  DNA
<213>  artificial

<220>
<223>  synthetic sequence

<400>  106
gtttggagga gcccagtttg gataattgct gttttgccag tttctttgcg gcgtgtt          57

<210>  107
<211>  48
<212>  DNA
<213>  artificial

<220>
<223>  synthetic sequence

<400>  107
ttggaatggg gagcaaaggc cgtggcaagc agggaggcaa agtacgtg                    48


<210>  108
<211>  48
<212>  DNA
<213>  artificial

<220>
<223>  synthetic sequence

<400>  108
gtttggagga gcctgaggaa cgggtctttg cttttgcacg tactttgc                    48


<210>  109
<211>  39
<212>  DNA
<213>  artificial

<220>
<223>  synthetic sequence

<400>  109
ttggaatggg gagcccgaac cgtgcaaaac gtgtaatca                              39


<210>  110
<211>  42
<212>  DNA
<213>  artificial

<220>
<223>  synthetic sequence

<400>  110
tatttttgga ggagccggta cggaaggtgg tgattacacg tt                          42


<210>  111
<211>  39
<212>  DNA
<213>  artificial

<220>
<223>  synthetic sequence

<400>  111
ttatgggctt cttcatcccg gcagtaatcc tgccctcca                              39


<210>  112
<211>  53
<212>  DNA
<213>  artificial

<220>

<223>  synthetic sequence

<400>  112
tatttttgga tctgccgccc ggtacgatca ggaatgcgat ggagggcagg att          53


<210>  113
<211>  47
<212>  DNA
<213>  artificial

<220>
<223>  synthetic sequence

<400>  113
ttatgggcaa accgggctgg ctgatcaaaa ggcactggta ttcaaga                 47


<210>  114
<211>  64
<212>  DNA
<213>  artificial

<220>
<223>  synthetic sequence

<400>  114
tatttttgga tctgccgcct gccagtctct tcagcggacg acggatcagt ttcttgaata    60

ccag                                                                 64


<210>  115
<211>  55
<212>  DNA
<213>  artificial

<220>
<223>  synthetic sequence

<400>  115
ggaatgggga gcatctggct gaccgcactg aaattcctcg gcaaacacgc cgcaa         55


<210>  116
<211>  60
<212>  DNA
<213>  artificial

<220>
<223>  synthetic sequence

<400>  116
tatttttgga ggagcccagt ttggataatt gctgttttgc cagtttcttt gcggcgtgtt    60


<210>  117
<211>  48
<212>  DNA
<213>  artificial

<220>
<223>  synthetic sequence

<400>  117
ttggaatggg gagcaaaggc cgtggcaagc agggaggcaa agtacgtg                 48

<210>  118
<211>  51
<212>  DNA
<213>  artificial

<220>
<223>  synthetic sequence

<400>  118
tatttttgga ggagcctgag gaacgggtct ttgcttttgc acgtactttg c          51


<210>  119
<211>  60
<212>  DNA
<213>  artificial

<220>
<223>  synthetic sequence

<400>  119
ggaatgggga gctcctccaa acgcaagaaa cgtaagaaac gcaaaaaaaa tagcgagaat     60


<210>  120
<211>  24
<212>  DNA
<213>  artificial

<220>
<223>  synthetic sequence

<400>  120
aactattccg agtgctttct ttgt                                        24


<210>  121
<211>  57
<212>  DNA
<213>  artificial

<220>
<223>  synthetic sequence

<400>  121
atgggatcca aacgcaagaa acgtaagaaa cgcaaaggct cctccccgaa ccgtgca       57


<210>  122
<211>  57
<212>  DNA
<213>  artificial

<220>
<223>  synthetic sequence

<400>  122
atgggatcca aacgcaagaa acgtaagaaa cgcaaagcct cctccttctt cgtagca       57


<210>  123
<211>  33
<212>  DNA
<213>  artificial

<220>
<223>  synthetic sequence

<400>  123
ggaatgggga gctcctccaa aaatagcgag aag                                    33


<210>  124
<211>  24
<212>  DNA
<213>  artificial

<220>
<223>  synthetic sequence

<400>  124
aactattccg tgtgctttct ttgt                                              24


<210>  125
<211>  48
<212>  DNA
<213>  artificial

<220>
<223>  synthetic sequence

<400>  125
atgggatcct tcttcgtagc accgggctcc tccaaaaata gcgagaag                    48


<210>  126
<211>  24
<212>  DNA
<213>  artificial

<220>
<223>  synthetic sequence

<400>  126
aactattccg tgtgctttct ttgt                                              24


<210>  127
<211>  298
<212>  PRT
<213>  artificial

<220>
<223>  synthetic sequence

<400>  127

Met Lys Arg Lys Lys Arg Lys Lys Arg Lys Arg Gly Leu Arg Arg Leu
1               5                   10                  15


Gly Arg Lys Ile Ala His Gly Val Lys Lys Tyr Gly Pro Thr Val Leu
            20                  25                  30


Arg Ile Ile Arg Ile Ala Gly Lys Val Leu Arg Lys Gly Asp Arg Gly
        35                  40                  45


Asp Glu Val Cys Gln Leu Gln Thr Leu Leu Asn Leu Cys Gly Tyr Asp
    50                  55                  60


Val Gly Lys Pro Asp Gly Ile Phe Gly Asn Asn Thr Phe Asn Gln Val
65                  70                  75                  80

Val Lys Phe Gln Lys Asp Asn Cys Leu Asp Ser Asp Gly Ile Val Gly
                85                  90                  95

Lys Asn Thr Trp Ala Glu Leu Phe Ser Lys Tyr Ser Pro Pro Ile Pro
            100                 105                 110

Tyr Lys Thr Ile Pro Met Pro Thr Ala Asn Lys Ser Arg Ala Ala Ala
        115                 120                 125

Thr Pro Val Met Asn Ala Val Glu Asn Ala Thr Gly Val Arg Ser Gln
    130                 135                 140

Leu Leu Leu Thr Phe Ala Ser Ile Glu Ser Ala Phe Asp Tyr Glu Ile
145                 150                 155                 160

Lys Ala Lys Thr Ser Ser Ala Thr Gly Trp Phe Gln Phe Leu Thr Gly
                165                 170                 175

Thr Trp Lys Thr Met Ile Glu Asn Tyr Gly Met Lys Tyr Gly Val Leu
            180                 185                 190

Thr Asp Pro Thr Gly Ala Leu Arg Lys Asp Pro Arg Ile Ser Ala Leu
        195                 200                 205

Met Gly Ala Glu Leu Ile Lys Glu Asn Met Asn Ile Leu Arg Pro Val
    210                 215                 220

Leu Lys Arg Glu Pro Thr Asp Thr Asp Leu Tyr Leu Ala His Phe Phe
225                 230                 235                 240

Gly Pro Gly Ala Ala Arg Arg Phe Leu Thr Thr Gly Gln Asn Glu Leu
                245                 250                 255

Ala Ala Thr His Phe Pro Lys Glu Ala Gln Ala Asn Pro Ser Ile Phe
            260                 265                 270

Tyr Asn Lys Asp Gly Ser Pro Lys Thr Ile Gln Glu Val Tyr Asn Leu
        275                 280                 285

Met Asp Gly Lys Val Ala Ala His Arg Lys
    290                 295

<210>  128
<211>  622
<212>  PRT
<213>  artificial

<220>
<223>  synthetic sequence

<400>  128

Met Arg Gly Leu Arg Arg Leu Gly Arg Lys Ile Ala His Gly Val Lys
1               5                   10                  15

Lys Tyr Gly Pro Thr Val Leu Arg Ile Ile Arg Ile Ala Gly Lys Arg
            20                  25                  30

Lys Lys Arg Lys Lys Arg Lys Asn Phe Arg Thr Lys Asn Gly Tyr Arg
        35                  40                  45

Asp Leu Gln Ala Leu Val Lys Glu Leu Gly Leu Tyr Thr Gly Gln Ile
    50                  55                  60

Asp Gly Val Trp Gly Lys Gly Thr Ser Ser Ser Thr Glu Thr Leu Leu
65                  70                  75                  80

Arg Gly Tyr Ala Glu Val Val Gly Lys Asn Thr Gly Gly Ile Gly Leu
                85                  90                  95

Pro Thr Thr Ser Asp Ala Ser Gly Tyr Asn Val Ile Thr Ala Leu Gln
            100                 105                 110

Arg Asn Leu Ala Phe Leu Gly Leu Tyr Ser Leu Thr Val Asp Gly Ile
        115                 120                 125

Trp Gly Asn Gly Thr Leu Ser Gly Leu Asp Lys Ala Phe Glu Val Tyr
    130                 135                 140

Lys Glu Arg Tyr Arg Thr Pro Thr Tyr Asp Ile Ala Trp Ser Gly Lys
145                 150                 155                 160

Val Ser Pro Ala Phe Thr Ala Lys Val Lys Asp Trp Cys Gly Val His
                165                 170                 175

Val Pro Asn His Arg Ala Pro His Trp Leu Met Ala Cys Met Ala Phe
            180                 185                 190

Glu Thr Gly Gln Thr Phe Ser Pro Ser Ile Lys Asn Ala Ala Gly Ser
        195                 200                 205

Glu Ala Tyr Gly Leu Ile Gln Phe Met Ser Pro Ala Ala Asn Asp Leu
    210                 215                 220

Asn Val Pro Leu Ser Val Ile Arg Ser Met Asp Gln Leu Thr Gln Leu
225                 230                 235                 240

Asp Leu Val Phe Lys Tyr Phe Glu Met Trp Met Lys Arg Gly Lys Arg
                245                 250                 255

Tyr Thr Gln Leu Glu Asp Phe Tyr Leu Thr Ile Phe His Pro Ala Ser
            260                 265                 270

Val Gly Lys Lys Ala Asp Glu Val Leu Phe Leu Gln Gly Ser Lys Ala
        275                 280                 285

Tyr Leu Gln Asn Lys Gly Phe Asp Val Asp Lys Asp Gly Lys Ile Thr
    290                 295                 300

Leu Gly Glu Ile Ser Ser Thr Leu Tyr Thr Thr Tyr Tyr Lys Gly Leu
305                 310                 315                 320

Leu Pro Glu Asn Arg His Val Ile Ser Tyr Met Asn Phe Arg Thr Lys
                325                 330                 335

Asn Gly Tyr Arg Asp Leu Gln Ala Leu Val Lys Glu Leu Gly Leu Tyr
            340                 345                 350

Thr Gly Gln Ile Asp Gly Val Trp Gly Lys Gly Thr Ser Ser Ser Thr
        355                 360                 365

Glu Thr Leu Leu Arg Gly Tyr Ala Glu Val Val Gly Lys Asn Thr Gly
    370                 375                 380

Gly Ile Gly Leu Pro Thr Thr Ser Asp Ala Ser Gly Tyr Asn Val Ile
385                 390                 395                 400

Thr Ala Leu Gln Arg Asn Leu Ala Phe Leu Gly Leu Tyr Ser Leu Thr
                405                 410                 415

Val Asp Gly Ile Trp Gly Asn Gly Thr Leu Ser Gly Leu Asp Lys Ala
            420                 425                 430

Phe Glu Val Tyr Lys Glu Arg Tyr Arg Thr Pro Thr Tyr Asp Ile Ala
        435                 440                 445

Trp Ser Gly Lys Val Ser Pro Ala Phe Thr Ala Lys Val Lys Asp Trp
    450                 455                 460

Cys Gly Val His Val Pro Asn His Arg Ala Pro His Trp Leu Met Ala
465                 470                 475                 480

Cys Met Ala Phe Glu Thr Gly Gln Thr Phe Ser Pro Ser Ile Lys Asn
                485                 490                 495

Ala Ala Gly Ser Glu Ala Tyr Gly Leu Ile Gln Phe Met Ser Pro Ala
            500                 505                 510

Ala Asn Asp Leu Asn Val Pro Leu Ser Val Ile Arg Ser Met Asp Gln
        515                 520                 525

Leu Thr Gln Leu Asp Leu Val Phe Lys Tyr Phe Glu Met Trp Met Lys
    530                 535                 540

Arg Gly Lys Arg Tyr Thr Gln Leu Glu Asp Phe Tyr Leu Thr Ile Phe
545                 550                 555                 560

His Pro Ala Ser Val Gly Lys Lys Ala Asp Glu Val Leu Phe Leu Gln
                565                 570                 575

Gly Ser Lys Ala Tyr Leu Gln Asn Lys Gly Phe Asp Val Asp Lys Asp
            580                 585                 590

Gly Lys Ile Thr Leu Gly Glu Ile Ser Ser Thr Leu Tyr Thr Thr Tyr
        595                 600                 605

Tyr Lys Gly Leu Leu Pro Glu Asn Arg His Val Ile Ser Tyr
    610                 615                 620

<210>  129
<211>  318
<212>  PRT
<213>  artificial

<220>
<223>  synthetic sequence

<400>  129

Met Arg Gly Leu Arg Arg Leu Gly Arg Lys Ile Ala His Gly Val Lys
1               5                   10                  15

Lys Tyr Gly Pro Thr Val Leu Arg Ile Ile Arg Ile Ala Gly Arg Gly
            20                  25                  30

Leu Arg Arg Leu Gly Arg Lys Ile Ala His Gly Val Lys Lys Tyr Gly
        35                  40                  45

Pro Thr Val Leu Arg Ile Ile Arg Ile Ala Gly Lys Val Leu Arg Lys
    50                  55                  60

Gly Asp Arg Gly Asp Glu Val Cys Gln Leu Gln Thr Leu Leu Asn Leu
65                  70                  75                  80

Cys Gly Tyr Asp Val Gly Lys Pro Asp Gly Ile Phe Gly Asn Asn Thr
                85                  90                  95

Phe Asn Gln Val Val Lys Phe Gln Lys Asp Asn Cys Leu Asp Ser Asp
            100                 105                 110

Gly Ile Val Gly Lys Asn Thr Trp Ala Glu Leu Phe Ser Lys Tyr Ser
        115                 120                 125

Pro Pro Ile Pro Tyr Lys Thr Ile Pro Met Pro Thr Ala Asn Lys Ser
    130                 135                 140

Arg Ala Ala Ala Thr Pro Val Met Asn Ala Val Glu Asn Ala Thr Gly
145                 150                 155                 160

Val Arg Ser Gln Leu Leu Leu Thr Phe Ala Ser Ile Glu Ser Ala Phe
                165                 170                 175

Asp Tyr Glu Ile Lys Ala Lys Thr Ser Ser Ala Thr Gly Trp Phe Gln
            180                 185                 190

Phe Leu Thr Gly Thr Trp Lys Thr Met Ile Glu Asn Tyr Gly Met Lys
        195                 200                 205

Tyr Gly Val Leu Thr Asp Pro Thr Gly Ala Leu Arg Lys Asp Pro Arg
    210                 215                 220

Ile Ser Ala Leu Met Gly Ala Glu Leu Ile Lys Glu Asn Met Asn Ile
225                 230                 235                 240

Leu Arg Pro Val Leu Lys Arg Glu Pro Thr Asp Thr Asp Leu Tyr Leu
                245                 250                 255

Ala His Phe Phe Gly Pro Gly Ala Ala Arg Arg Phe Leu Thr Thr Gly
            260                 265                 270

Gln Asn Glu Leu Ala Ala Thr His Phe Pro Lys Glu Ala Gln Ala Asn
        275                 280                 285

Pro Ser Ile Phe Tyr Asn Lys Asp Gly Ser Pro Lys Thr Ile Gln Glu
    290                 295                 300

Val Tyr Asn Leu Met Asp Gly Lys Val Ala Ala His Arg Lys
305                 310                 315

<210> 130
<211> 274
<212> PRT
<213> artificial

<220>
<223> synthetic sequence

<400> 130

<210>  131
<211>  313
<212>  PRT
<213>  artificial

<220>
<223>  synthetic sequence

<400>  131

Met Gly Trp Leu Lys Lys Ile Gly Lys Lys Ile Glu Arg Val Gly Gln
1               5                   10                  15

His Thr Arg Asp Ala Thr Ile Gln Gly Leu Gly Ile Ala Gln Gln Ala
            20                  25                  30

Ala Asn Val Ala Ala Thr Ala Arg Arg Gly Leu Arg Arg Leu Gly Arg
        35                  40                  45

Lys Ile Ala His Gly Val Lys Lys Tyr Gly Pro Thr Val Leu Arg Ile
    50                  55                  60

Ile Arg Ile Ala Gly Ala His Glu His Ser Ala Gln Trp Leu Asn Asn
65                  70                  75                  80

Tyr Lys Lys Gly Tyr Gly Tyr Gly Pro Tyr Pro Leu Gly Ile Asn Gly
                85                  90                  95

Gly Met His Tyr Gly Val Asp Phe Phe Met Asn Ile Gly Thr Pro Val
            100                 105                 110

Lys Ala Ile Ser Ser Gly Lys Ile Val Glu Ala Gly Trp Ser Asn Tyr
        115                 120                 125

Gly Gly Gly Asn Gln Ile Gly Leu Ile Glu Asn Asp Gly Val His Arg
    130                 135                 140

Gln Trp Tyr Met His Leu Ser Lys Tyr Asn Val Lys Val Gly Asp Tyr
145                 150                 155                 160

Val Lys Ala Gly Gln Ile Ile Gly Trp Ser Gly Ser Thr Gly Tyr Ser
                165                 170                 175

Thr Ala Pro His Leu His Phe Gln Arg Met Val Asn Ser Phe Ser Asn
            180                 185                 190

Ser Thr Ala Gln Asp Pro Met Pro Phe Leu Lys Ser Ala Gly Tyr Gly
        195                 200                 205

Lys Ala Gly Gly Thr Val Thr Pro Thr Pro Asn Thr Gly Trp Lys Thr
    210                 215                 220

Asn Lys Tyr Gly Thr Leu Tyr Lys Ser Glu Ser Ala Ser Phe Thr Pro
225                 230                 235                 240

Asn Thr Asp Ile Ile Thr Arg Thr Thr Gly Pro Phe Arg Ser Met Pro
                245                 250                 255

Gln Ser Gly Val Leu Lys Ala Gly Gln Thr Ile His Tyr Asp Glu Val
            260                 265                 270

Met Lys Gln Asp Gly His Val Trp Val Gly Tyr Thr Gly Asn Ser Gly
        275                 280                 285

Gln Arg Ile Tyr Leu Pro Val Arg Thr Trp Asn Lys Ser Thr Asn Thr
    290                 295                 300

Leu Gly Val Leu Trp Gly Thr Ile Lys
305                 310

<210>  132
<211>  313
<212>  PRT
<213>  artificial

<220>
<223>  synthetic sequence

<400>  132

Met Ala His Glu His Ser Ala Gln Trp Leu Asn Asn Tyr Lys Lys Gly
1               5                   10                  15

Tyr Gly Tyr Gly Pro Tyr Pro Leu Gly Ile Asn Gly Gly Met His Tyr
            20                  25                  30

Gly Val Asp Phe Phe Met Asn Ile Gly Thr Pro Val Lys Ala Ile Ser
        35                  40                  45

Ser Gly Lys Ile Val Glu Ala Gly Trp Ser Asn Tyr Gly Gly Gly Asn
    50                  55                  60

Gln Ile Gly Leu Ile Glu Asn Asp Gly Val His Arg Gln Trp Tyr Met
65                  70                  75                  80

His Leu Ser Lys Tyr Asn Val Lys Val Gly Asp Tyr Val Lys Ala Gly
                85                  90                  95

Gln Ile Ile Gly Trp Ser Gly Ser Thr Gly Tyr Ser Thr Ala Pro His
            100                 105                 110

Leu His Phe Gln Arg Met Val Asn Ser Phe Ser Asn Ser Thr Ala Gln
        115                 120                 125

Asp Pro Met Pro Phe Leu Lys Ser Ala Gly Tyr Gly Lys Ala Gly Gly
    130                 135                 140

Thr Val Thr Pro Thr Pro Asn Thr Gly Trp Lys Thr Asn Lys Tyr Gly
145                 150                 155                 160

Thr Leu Tyr Lys Ser Glu Ser Ala Ser Phe Thr Pro Asn Thr Asp Ile
                165                 170                 175

Ile Thr Arg Thr Thr Gly Pro Phe Arg Ser Met Pro Gln Ser Gly Val
            180                 185                 190

Leu Lys Ala Gly Gln Thr Ile His Tyr Asp Glu Val Met Lys Gln Asp
        195                 200                 205

Gly His Val Trp Val Gly Tyr Thr Gly Asn Ser Gly Gln Arg Ile Tyr
    210                 215                 220

Leu Pro Val Arg Thr Trp Asn Lys Ser Thr Asn Thr Leu Gly Val Leu
225                 230                 235                 240

Trp Gly Thr Ile Lys Gly Trp Leu Lys Lys Ile Gly Lys Lys Ile Glu
                245                 250                 255

Arg Val Gly Gln His Thr Arg Asp Ala Thr Ile Gln Gly Leu Gly Ile
            260                 265                 270

Ala Gln Gln Ala Ala Asn Val Ala Ala Thr Ala Arg Arg Gly Leu Arg
        275                 280                 285

Arg Leu Gly Arg Lys Ile Ala His Gly Val Lys Lys Tyr Gly Pro Thr
    290                 295                 300

Val Leu Arg Ile Ile Arg Ile Ala Gly
305                 310

<210>  133
<211>  263
<212>  PRT
<213>  artificial

<220>
<223>  synthetic sequence

<400>  133

Met Gly Ile Gly Ala Val Leu Lys Val Leu Thr Thr Gly Leu Pro Ala
1               5                   10                  15

Leu Ile Ser Trp Ile Lys Arg Lys Arg Gln Gln Arg Gly Leu Arg Arg
            20                  25                  30

Leu Gly Arg Lys Ile Ala His Gly Val Lys Lys Tyr Gly Pro Thr Val
        35                  40                  45

Leu Arg Ile Ile Arg Ile Ala Gly Ala Ile Ser Lys Lys Lys Val Gly
    50                  55                  60

Gly Val Gly Gly Val Ile Ala Ala Ile Ile Ala Ala Val Phe Ala Val
65                  70                  75                  80

Glu Gly Gly Tyr Val Asn Asp Pro Lys Asp Pro Gly Gly Glu Thr Asn
                85                  90                  95

His Gly Val Thr Ile Gln Val Ala Gln Lys His Lys Gln Glu Leu Glu
            100                 105                 110

Ser Met Tyr Asn Trp Asp Gly Ser Met Lys Asn Leu Thr Gln Glu Met
        115                 120                 125

Ala Ser Ser Ile Tyr Tyr Asn Asp Tyr Ile Leu Lys Pro Gly Phe Val
    130                 135                 140

Lys Phe Ala Asp Val Ser Pro Ala Val Thr Glu Lys Leu Val Asp Ala
145                 150                 155                 160

Gly Val Asn Thr Gly Pro Ala Arg Pro Ser Arg Trp Leu Gln Glu Ser
                165                 170                 175

Leu Asn Ala Phe Ser Arg Asn Gly Lys Asp Tyr Pro Lys Ile Gln Val
            180                 185                 190

Asp Gly Lys Val Gly Ser Gly Thr Leu Ser Ala Tyr Lys Ser Leu Gln
        195                 200                 205

Asn Lys Arg Gly Lys Val Glu Ala Cys Lys Leu Ile Leu Lys Ser Leu
    210                 215                 220

Asp Gly Lys Gln Leu Asn Tyr Tyr Leu Ser Leu Asn Met Pro Glu Tyr
225                 230                 235                 240

Thr Thr Gly Trp Ile Ala Asn Arg Ile Gly Asn Val Pro Leu Glu Arg
                245                 250                 255

Cys Asn Glu Asp Ile Val Asn
            260

<210>  134
<211>  400
<212>  PRT
<213>  artificial

<220>
<223>  synthetic sequence

<400>  134

<210>  135
<211>  385
<212>  PRT
<213>  artificial

<220>
<223>  synthetic sequence

<400>  135
   Met Gly Ile Gly Lys Phe Leu His Ser Ala Lys Lys Phe Gly Lys Ala
   1               5                   10                  15
   Phe Val Gly Glu Ile Met Asn Ser Thr Arg Ser Ser Arg Ala Gly Leu
               20                  25                  30
   Gln Phe Pro Val Gly Arg Val His Arg Leu Leu Arg Lys Val Lys Tyr
           35                  40                  45
   Thr Val Glu Asn Lys Ile Ile Ala Gly Leu Pro Lys Gly Lys Leu Lys
       50                  55                  60
   Gly Ala Asn Phe Val Ile Ala His Glu Thr Ala Asn Ser Lys Ser Thr
   65                  70                  75                  80
   Ile Asp Asn Glu Val Ser Tyr Met Thr Arg Asn Trp Lys Asn Ala Phe
                   85                  90                  95
   Val Thr His Phe Val Gly Gly Gly Gly Arg Val Val Gln Val Ala Asn
               100                 105                 110
   Val Asn Tyr Val Ser Trp Gly Ala Gly Gln Tyr Ala Asn Ser Tyr Ser
           115                 120                 125
   Tyr Ala Gln Val Glu Leu Cys Arg Thr Ser Asn Ala Thr Thr Phe Lys
       130                 135                 140
   Lys Asp Tyr Glu Val Tyr Cys Gln Leu Leu Val Asp Leu Ala Lys Lys
   145                 150                 155                 160
   Ala Gly Ile Pro Ile Thr Leu Asp Ser Gly Ser Lys Thr Ser Asp Lys
                   165                 170                 175
   Gly Ile Lys Ser His Lys Trp Val Ala Asp Lys Leu Gly Gly Thr Thr
               180                 185                 190
   His Gln Asp Pro Tyr Ala Tyr Leu Ser Ser Trp Gly Ile Ser Lys Ala
           195                 200                 205
   Gln Phe Ala Ser Asp Leu Ala Lys Val Ser Gly Gly Gly Asn Thr Gly
       210                 215                 220
   Thr Ala Pro Ala Lys Pro Ser Thr Pro Ala Pro Lys Pro Ser Thr Pro
   225                 230                 235                 240
   Ser Thr Asn Leu Asp Lys Leu Gly Leu Val Asp Tyr Met Asn Ala Lys
                   245                 250                 255
   Lys Met Asp Ser Ser Tyr Ser Asn Arg Asp Lys Leu Ala Lys Gln Tyr
               260                 265                 270
   Gly Ile Ala Asn Tyr Ser Gly Thr Ala Ser Gln Asn Thr Thr Leu Leu
           275                 280                 285
   Ser Lys Ile Lys Gly Gly Ala Pro Lys Pro Ser Thr Pro Ala Pro Lys
       290                 295                 300
   Pro Ser Thr Ser Thr Ala Lys Lys Ile Tyr Phe Pro Pro Asn Lys Gly
   305                 310                 315                 320
   Asn Trp Ser Val Tyr Pro Thr Asn Lys Ala Pro Val Lys Ala Asn Ala
                   325                 330                 335
   Ile Gly Ala Ile Asn Pro Thr Lys Phe Gly Gly Leu Thr Tyr Thr Ile
               340                 345                 350
   Gln Lys Asp Arg Gly Asn Gly Val Tyr Glu Ile Gln Thr Asp Gln Phe
           355                 360                 365
   Gly Arg Val Gln Val Tyr Gly Ala Pro Ser Thr Gly Ala Val Ile Lys
       370                 375                 380
   Lys
   385

## Description

The present invention relates to a fusion protein composed of an enzyme having the activity of degrading the cell wall of Gram-negative bacteria and/or Gram-positive bacteria and at least two peptide stretches fused to the enzyme at the N- and/or C-terminus, wherein the peptide stretches are distinct and selected from the group of synthetic amphiphatic peptide, synthetic cationic peptide, synthetic polycationic peptide, synthetic hydrophobic peptide, or naturally occurring antimicrobial peptide, like sushi peptide and defensin. Moreover, the present invention relates to nucleic acid molecules encoding said fusion protein, vectors comprising said nucleic acid molecules and host cells comprising either said nucleic acid molecules or said vectors. In addition, the present invention relates to said fusion protein for use as a medicament, diagnostic means, cosmetic substance, a disinfectant or a food additive. The present invention also relates to the treatment or prevention of Gram-postive and/or Gram-negative bacterial contamination of foodstuff, of food processing equipment, of food processing plants, of surfaces coming into contact with foodstuff, of medical devices, of surfaces in hospitals and surgeries.

Gram-negative bacteria possess an outer membrane, with its characteristic asymmetric bilayer as a hallmark. The outer membrane bilayer consists of an inner monolayer containing phospholipids (primarily phosphatidyl ethanolamine) and an outer monolayer that is mainly composed of a single glycolipid, lipopolysaccharide (LPS). There is an immense diversity of LPS structures in the bacterial kingdom and the LPS structure may be modified in response to prevailing environmental conditions. The stability of the LPS layer and interaction between different LPS molecules is mainly achieved by the electrostatic interaction of divalent ions (Mg²⁺, Ca²⁺) with the anionic components of the LPS molecule (phosphate groups in the lipid A and the inner core and carboxyl groups of KDO). Furthermore, the dense and ordered packing of the hydrophobic moiety of lipid A, favored by the absence of unsaturated fatty acids, forms a rigid structure with high viscosity. This makes it less permeable for lipophilic molecules and confers additional stability to the outer membrane (OM).

In contrast to Gram-negative bacteria, Gram-positive bacteria do not possess an outer membrane. The cytoplasmic membrane is surrounded by an up to 25 nm thick layer of peptidoglycan (which is only up to 5 nm for Gram-negative bacteria) which forms the cell wall. Main purpose of the cell wall of Gram-positives is to maintain bacterial shape and to counteract the internal bacterial cell pressure. Peptidoglycan, or murein, is a polymer consisting of sugars and amino acids. The sugar component consists of alternating residues of β-(1,4) linked N-acetylglucosamine and N-acetylmuramic acid residues compose the sugar components. A peptide chain of three to five amino acids is attached to the N-acetylmuramic acid. The peptide chain can be cross-linked to the peptide chain of another strand forming a 3D mesh-like layer. The peptide chain may contain D- and L- amino acid residues and the composition may vary for different bacteria.

Various types of agents having bactericidal or bacteriostatic activity are known, e.g. antibiotics, endolysins, antimicrobial peptides and defensins. Increasingly microbial resistance to antibiotics, however, is creating difficulties in treating more and more infections caused by bacteria. Particular difficulties arise with infections caused by Gram-negative bacteria like *Pseudomonas aeruginosa* and Enterobacteriaceae and with infections caused by Gram-positive bacteria like *Staphylococcus aureus, Enterococci, Streptococci, Listeria monocytogenes* and *Clostridium difficile,* especially with e.g. Methicillin-resistant *Staphylococcus aureus* and Vancomycin-resistant *Enterococci.*

Endolysins are peptidoglycan hydrolases encoded by bacteriophages (or bacterial viruses). They are synthesized during late gene expression in the lytic cycle of phage multiplication and mediate the release of progeny virions from infected cells through degradation of the bacterial peptidoglycan. They are either ß(1,4)-glycosylases , transglycosylases, amidases or endopeptidases. Antimicrobial application of endolysins was already suggested in 1991 by Gasson (GB2243611). Although the killing capacity of endolysins has been known for a long time, the use of these enzymes as antibacterials was ignored due to the success and dominance of antibiotics. Only after the appearance of multiple antibiotic resistant bacteria this concept of combating human pathogens with endolysins received interest. A compelling need to develop totally new classes of antibacterial agents emerged and endolysins used as 'enzybiotics' - a hybrid term of 'enzymes' and 'antibiotics' - seem to meet this need. In 2001, Fischetti and coworkers demonstrated for the first time the therapeutic potential of bacteriophage Cl endolysin towards group A streptococci (Nelson et al., 2001). Since then many publications have established endolysins as an attractive and complementary alternative to control bacterial infections, particularly by Gram positive bacteria. Subsequently different endolysins against other Gram positive pathogens such as *Streptococcus pneumoniae* (Loeffler et al., 2001), *Bacillus anthracis* (Schuch et al., 2002), S. *agalactiae* (Cheng et al., 2005) and *Staphylococcus aureus* (Rashel et al, 2007) have proven their efficacy as enzybiotics. Nowadays, the most important challenge of endolysin therapy lies in the insensitivity of Gram-negative bacteria towards the exogenous action of endolysins, since the outer membrane shields the access of endolysins from the peptidoglycan. This currently prevents the expansion of the range of effective endolysins to important Gram-negative pathogens. However, it is also known that endolysins can, under some conditions (e.g. high ionic strength), create stable protoplast, where the internal bacterial cell pressure is not sufficient to lead to a cell burst. Under these conditions the bacterial cell wall can regenerate and the bacteria will survive.

Antimicrobial peptides (AMPs) represent a wide range of short, cationic or amphiphatic, gene encoded peptide antibiotics that can be found in virtually every organism. Different AMPs display different properties, and many peptides in this class are being intensively researched not only as antibiotics, but also as templates for cell penetrating peptides. Despite sharing a few common features (e.g., cationicity, amphiphaticity and short size), AMP sequences vary greatly, and at least four structural groups (α-helical, β-sheet, extended and looped) have been proposed to accommodate the diversity of the observed AMP conformations. Likewise, several modes of action as antibiotics have been proposed, and it was shown e.g. that the primary target of many of these peptides is the cell membrane whereas for other peptides the primary target is cytoplasmic invasion and disruption of core metabolic functions. AMPs may become concentrated enough to exhibit cooperative activity despite the absence of specific target binding; for example, by forming a pore in the membrane, as is the case for most AMPs. However, this phenomenon has only been observed in model phospholipid bilayers, and in some cases, AMP concentrations in the membrane that were as high as one peptide molecule per six phospholipid molecules were required for these events to occur. These concentrations are close to, if not at, full membrane saturation. As the minimum inhibitory concentration (MIC) for AMPs are typically in the low micromolar range, scepticism has understandably arisen regarding the relevance of these thresholds and their importance *in vivo* (Melo et al., Nature reviews, Microbiology, 2009, 245).

Defensins are a large family of small, cationic or amphiphatic, cysteine- and arginine-rich antimicrobial peptides, found in both vertebrates and invertebrates. Defensins are divided into five groups according to the spacing pattern of cysteines: plant, invertebrate, α-, β-, and θ-defensins. The latter three are mostly found in mammals. α -defensins are proteins found in neutrophils and intestinal epithelia. β-defensins are the most widely distributed and are secreted by leukocytes and epithelial cells of many kinds. θ-defensins have been rarely found so far e.g. in leukocytes of rhesus macaques. Defensins are active against bacteria, fungi and many enveloped and nonenveloped viruses. However, the concentrations needed for efficient killing of bacteria are mostly high, i.e. in the µ-molar range. Activity of many peptides may be limited in presence of physiological salt conditions, divalent cations and serum. Depending on the content of hydrophobic amino acid residues defensins also show haemolytic activity.

Thus, there is a need for new antimicrobial agents.

This object is solved by the subject matter defined in the claims.

The following figures serve to illustrate the invention.

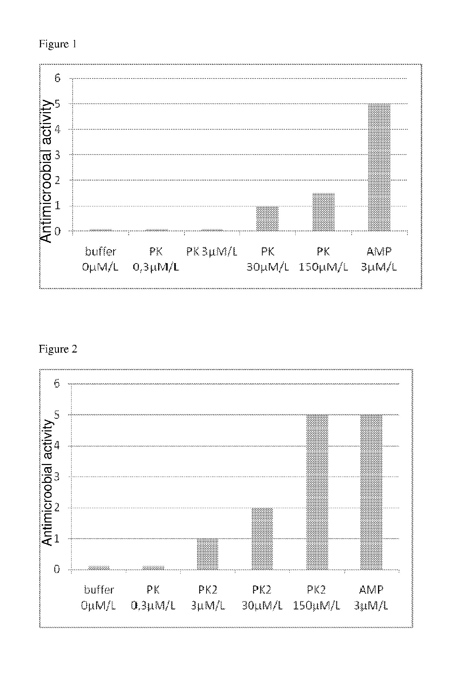

Figure 1 shows the antibacterial activities of a polycationic peptide named PK (SEQ ID NO: 23) at different concentrations compared to buffer (10 mM HEPES, 0.5 mM EDTA; pH 7.4) and an antimicrobial peptide (AMP = SMAP-29; SEQ NO: 45). The antibacterial activities are given as relative inactivation in logarithmic units on the y-axis.
Figure 2 shows the antibacterial activities shows the antibacterial activities of a polycationic peptide named PK2 (SEQ NO: 34) at different concentrations compared to buffer (10 mM HEPES, 0.5 mM EDTA; pH 7.4) and an antimicrobial peptide (AMP = SMAP-29, SEQ ID NO: 45). The antibacterial activities are given as relative inactivation in logarithmic units on the y-axis.

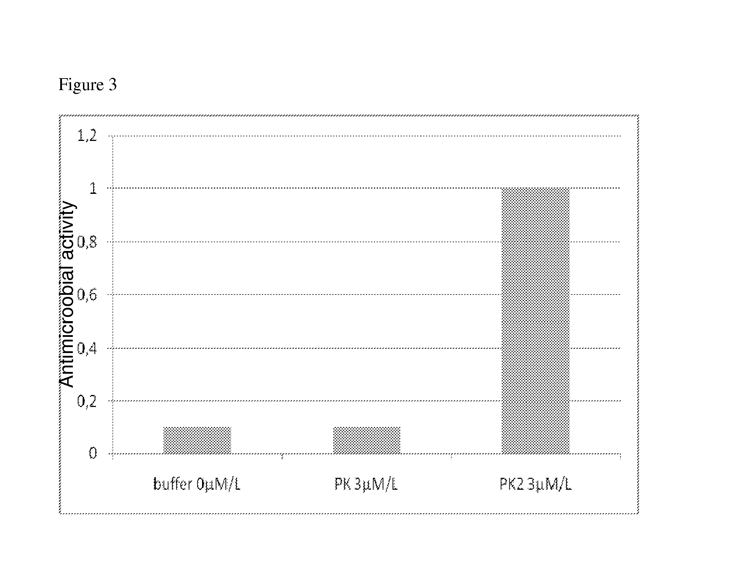

Figure 3 shows the antibacterial activities of a polycationic peptide named PK (SEQ ID NO: 23) and a polycationic peptide named PK2 (SEQ NO: 34) at a concentration of 3 µM/L compared to buffer (10 mM HEPES, 0.5 mM EDTA; pH 7.4). The antibacterial activities are given as relative inactivation in logarithmic units on the y-axis.

The term "protein" as used herein refers to a linear polymer of amino acid residues linked by peptide bonds in a specific sequence. The amino-acid residues of a protein may be modified by e.g. covalent attachments of various groups such as carbohydrates and phosphate. Other substances may be more loosely associated with the protein, such as heme or lipid, giving rise to the conjugated proteins which are also comprised by the term "protein" as used herein. The various ways in which the protein fold have been elucidated, in particular with regard to the presence of alpha helices and beta-pleated sheets. The term "protein" as used herein refers to all four classes of proteins being all-alpha, all-beta, alpha/beta and alpha plus beta. Moreover, the term "protein" refers to a complex, wherein the complex refers to a homomer.

The term "fusion protein" as used herein refers to an expression product resulting from the fusion of three nucleic acid sequences. Such a protein may be produced, e.g., in recombinant DNA expression systems. Moreover, the term "fusion protein" as used herein refers to a fusion of a first amino acid sequence as e.g. an endolysin, with a second and a third amino acid sequence. The second and third amino acid sequences are preferably peptide stretches, in particular selected from the group consisting of cationic, polycationic, hydrophobic, amphiphatic, sushi and antimicrobial peptides.. Preferably, said second and third amino acid sequence is foreign to and not substantially homologous with any domain of the first amino acid sequence. Moreover, the fusion protein of the present invention also refers to an expression product resulting from the fusion of at least three nucleic acid sequences. The fusion protein also refers to a fusion of a first amino acid sequence as e.g. an endolysin, with more than two additional amino acid sequences encoding peptide stretches.

The term "peptide stretch" as used herein refers to any kind of peptide linked to a protein such as an endolysin. In particular the term "peptide stretch" as used herein refers to a cationic peptide, a polycationic peptide, an amphiphatic peptide, a hydrophobic peptide, a sushi peptide and/or an antimicrobial peptide. However, a peptide stretch in the meaning of the present invention does not refer to His-tags, preferably His₅-tags, His₆-tags, His₇-tags, His₈-tags, His₉-tags, His₁₀-tags, His₁₁-tags, His₁₂-tags, His₁₆-tags and His₂₀-tags, Strep-tags, Avitags, Myc-tags, Gst-tags, JS-tags, cystein-tags, FLAG-tags or other tags known in the art, thioredoxin or maltose binding proteins (MBP). The term "tag" in contrast to the term "peptide stretch" as used herein refers to a peptide which can be useful to facilitate expression and/or affinity purification of a polypeptide, to immobilize a polypeptide to a surface or to serve as a marker or a label moiety for detection of a polypeptide e.g. by antibody binding in different ELISA assay formats as long as the function making the tag useful for one of the above listed facilitation is not caused by the positively charge of said peptide. However, the His₆-tag may, depending on the respective pH, also be positively charged, but is used as affinity purification tool as it binds to immobilized divalent cations and is not used as a peptide stretch according to the present invention.

The term "peptide" as used herein refers to short polypeptides consisting of from about 2 to about 100 amino acid residues, more preferably from about 4 to about 50 amino acid residues, more preferably from about 5 to about 30 amino acid residues, wherein the amino group of one amino acid residue is linked to the carboxyl group of another amino acid residue by a peptide bond. A peptide may have a specific function. A peptide can be a naturally occurring peptide or a synthetically designed and produced peptide. The peptide can be, for example, derived or removed from a native protein by enzymatic or chemical cleavage, or can be prepared using conventional peptide synthesis techniques (e.g., solid phase synthesis) or molecular biology techniques (see Sambrook, J. et al., Molecular Cloning: A Laboratory Manual, Cold Spring Harbor Press, Cold Spring Harbor, N.Y. (1989)). Preferred naturally occurring peptides are e.g. antimicrobial peptides, defensins, and sushi peptides. Preferred synthetically produced peptides are e.g. polycationic, amphipathic or hydrophobic peptides. A peptide in the meaning of the present invention does not refer to His-tags, Strep-tags, thioredoxin or maltose binding proteins (MBP) or the like, which are used to purify or locate proteins.

The term "endolysin" as used herein refers to an enzyme which is a peptidoglycan hydrolase naturally encoded by bacteriophages or bacterial viruses and which is suitable to hydrolyse bacterial cell walls. "Endolysins" comprise at least one "enzymatically active domain" (EAD) having at least one of the following activities: endopeptidase, N-acetyl-muramoyl-L-alanine-amidase (amidase), N-acetyl-muramidase, N-acetyl-glucosaminidase or transglycosylases. In addition, the endolysins may contain also regions which are enzymatically inactive, and bind to the cell wall of the host bacteria, the so-called CBDs (cell wall binding domains). The endolysin may contain two or more CBDs. Generally, the cell wall binding domain is able to bind different components on the surface of bacteria. Preferably, the cell wall binding domain is a peptidoglycan binding domain and binds to the bacteria's peptidoglycan structure. The different domains of an endolysin can be connected by a domain linker.

The term "domain linker" as used herein refers to an amino acid sequence functioning to connect single protein domains with one another. As a rule domain linkers form no or only few regular secondary structure like α-helices or ß-sheets and can occupy different conformations with the respective structural context. Methods to detect domain linker and properties of linker sequences are well known in the art as e.g. described in Bae et al., 2005, Bioinformatics, 21, 2264-2270 or George & Heringa, 2003, Protein Engineering, 15, 871-879.

The term "deletion" as used herein refers to the removal of 1, 2, 3, 4, 5 or more amino acid residues from the respective starting sequence.

The term "insertion" or "addition" as used herein refers to the insertion or addition of 1, 2, 3, 4, 5 or more amino acid residues to the respective starting sequence.

The term "substitution" as used herein refers to the exchange of an amino acid residue located at a certain position for a different one.

The term "cell wall" as used herein refers to all components that form the outer cell enclosure of the Gram-positive and Gram-negative bacteria and thus guarantee their integrity. In particular, the term "cell wall" as used herein refers to peptidoglycan, the outer membrane of the Gram-negative bacteria with the lipopolysaccharide, the bacterial cell membrane, but also to additional layers deposited on the peptidoglycan as e.g. capsules, outer protein layers or slimes.

The term "EAD" as used herein refers to the enzymatically active domain of an endolysin. The EAD is responsible for hydrolysing bacterial peptidoglycans. It exhibits at least one enzymatic activity of an endolysin. The EAD can also be composed of more than one enzymatically active module. The term "EAD" is used herein synonymously with the term "catalytic domain".

As used herein, the term "cationic peptide" refers to a synthetic peptide having positively charged amino acid residues. Preferably a cationic peptide has a pKa-value of 9.0 or greater. Typically, at least four of the amino acid residues of the cationic peptide can be positively charged, for example, lysine or arginine. "Positively charged" refers to the side chains of the amino acid residues which have a net positive charge at about physiological conditions. The term "cationic peptide" as used herein refers also to polycationic peptides.

The term "polycationic peptide" as used herein refers to a synthetically produced peptide composed of mostly positively charged amino acid residues, in particular lysine and/or arginine residues. A peptide is composed of mostly positively charged amino acid residues of at least about 20, 30, 40, 50, 60, 70, 75, 80, 85, 90, 95 or about 100 % of the amino acid residues are positively charged amino acid residues, in particular lysine and/or arginine residues. The amino acid residues being not positively charged amino acid residues can be neutrally charged amino acid residues and/or negatively charged amino acid residues and/or hydrophobic amino acid residues. Preferably the amino acid residues being not positively charged amino acid residues are neutrally charged amino acid residues, in particular serine and/or glycine.

The term, "antimicrobial peptide" (AMP) as used herein refers to any naturally occurring peptide that has microbicidal and/or microbistatic activity on for example bacteria, viruses, fungi, yeasts, mycoplasma and protozoa. Thus, the term "antimicrobial peptide" as used herein refers in particular to any peptide having anti-bacterial, anti-fungal, anti-mycotic, antiparasitic, anti-protozoal, anti-viral, anti-infectious, anti-infective and/or germicidal, algicidal, amoebicidal, microbicidal, bactericidal, fungicidal, parasiticidal, protozoacidal, protozoicidal properties.

The antimicrobial peptide may be a member of the RNAse A super family, a defensin, cathelicidin, granulysin, histatin, psoriasin, dermicidine or hepcidin. The antimicrobial peptide may be naturally occurring in insects, fish, plants, arachnids, vertebrates or mammals. Preferably the antimicrobial peptide may be naturally occurring in insects, fish, plants, arachnids, vertebrates or mammals. Preferably the antimicrobial peptide may be naturally occurring in radish, silk moth, wolf spider, frog, preferably in *Xenopus laevis, Rana* frogs, more preferably in *Rana catesbeiana,* toad, preferably Asian toad *Bufo bufo gargarizans,* fly, preferably in *Drosophila,* more preferably in *Drosophila melanogaster,* in *Aedes aegypti,* in honey bee, bumblebee, preferably in *Bombus pascuorum,* flesh fly, preferably in *Sarcophaga peregrine,* scorpion, horseshoe crab, catfish, preferably in *Parasilurus asotus,* cow, pig, sheep, porcine, bovine, monkey and human.

The term "sushi peptide" as used herein refers to complement control proteins (CCP) having short consensus repeats. The sushi module of sushi peptides functions as a protein-protein interaction domain in many different proteins. Peptides containing a Sushi domain have been shown to have antimicrobial activities. Preferably, sushi peptides are naturally occurring peptides.

The term "amphiphatic peptide" as used herein refers to synthetic peptides having both hydrophilic and hydrophobic functional groups. Preferably, the term "amphiphatic peptide" as used herein refers to a peptide having a defined arrangement of hydrophilic and hydrophobic groups e.g. amphiphatic peptides may be e.g. alpha helical, having predominantly non polar side chains along one side of the helix and polar residues along the remainder of its surface.

The term "hydrophobic group" as used herein refers to chemical groups such as amino acid side chains which are substantially water insoluble, but soluble in an oil phase, with the solubility in the oil phase being higher than that in water or in an aqueous phase. In water, amino acid residues having a hydrophobic side chain interact with one another to generate a nonaqueous environment. Examples of amino acid residues with hydrophobic side chains are valine, isoleucine, leucine, methionine, phenylalanine, tryptophan, cysteine, alanine, tyrosine, histidine, threonin, serine, proline and glycine residues.

The term "autolysins" refers to enzymes related to endolysins but encoded by bacteria and involved in e.g. cell division. An overview of autolysins is can be found in "Bacterial peptidoglycan (murein) hydrolases. Vollmer W, Joris B, Charlier P, Foster S. FEMS Microbiol Rev. 2008 Mar;32(2):259-86".

The term "bacteriocin" as used herein refers to protein-like, polypeptide-like or peptide-like substances which are able to inhibit the growth of other bacteria. Some bacteriocins are capable of degrading bacterial cell walls like Lysostaphin (degrading *Staphylococcus* cell walls), Mutanolysin (degrading *Streptococcus* cell walls) and Enterolysin (degrading *Enterococcus* cell walls). Preferably said inhibition is specifically by means of absorption of said other bacteria to specific receptors of the bacteriocin. In general, bacteriocins are produced by microorganisms. However, the term "bacteriocin" as used herein refers both to an isolated form procuded by a microorganism or to a synthetically produced form, and refers also to variants which substantially retain the activities of their parent bacteriocins, but whose sequences have been altered by insertion or deletion of one or more amino acid residues.

The present invention relates to new antibacterial agents against Gram-positive and/or Gram-negative bacteria, in particular to fusion proteins composed of an enzyme having the activity of degrading the cell wall of Gram-positive and/or Gram-negative bacteria and two peptide stretches. These two peptide stretches may be the same or distinct. These peptide stretches are linked to the N- or C-terminus or at both termini of the enzyme of the fusion protein according to the present invention. The skilled person understands that the fusion proteins according to the present invention comprises 2, 3, 4, 5 or more peptide stretches.

In a preferred embodiment the fusion proteins according to the present invention are composed of an enzyme having the activity of degrading the cell wall of Gram-positive and/or Gram-negative bacteria and two peptide stretches fused to the enzyme on the N-terminus. Preferably, wherein the two peptide stretches are distinct from each other.

In another preferred embodiment the fusion proteins according to the present invention are composed of an enzyme having the activity of degrading the cell wall of Gram-positive and/or Gram-negative bacteria and two peptide stretches fused to the enzyme on the C-terminus. Preferably, wherein the two peptide stretches are distinct from each other.

The inventors found out that the fusion proteins according to the present invention show different spectra in view of the species to be effected.

In one aspect of the present invention the enzyme having the activity of degrading the cell wall of Gram-positive and/or Gram-negative bacteria is an endolysin, autolysin or bacteriocin. The enzyme of the fusion protein according to the present invention is no lysozyme. In a preferred embodiment the enzyme of the fusion protein according to the present invention is encoded by bacteriophages or bacterial virus. In another preferred embodiment the enzyme of the fusion protein according to the present invention is an endolysin. In a more preferred embodiment the endolysin is encoded by bacteriophages or bacterial viruses.

Preferred fusion proteins according to the present invention are depicted in SEQ ID NO: 70 to 77 and 127 to 135 may comprise one or more additional amino acid residues on the N-terminus. Preferably the additional amino acid residue is methionine.

Preferably, the endolysin is encoded by bacteriophages specific for Gram-negative bacteria such as Gram-negative bacteria of bacterial groups, families, genera or species comprising strains pathogenic for humans or animals like *Enterobacteriaceae (Escherichia,* especially *E*. *coli, Salmonella, Shigella, Citrobacter, Edwardsiella, Enterobacter, Hafnia, Klebsiella, especially K. pneumoniae, Morganella, Proteus, Providencia, Serratia, Yersinia), Pseudomonadaceae (Pseudomonas, especially P. aeruginosa, Burkholderia, Stenotrophomonas, Shewanella, Sphingomonas, Comamonas), Neisseria, Moraxella, Vibrio, Aeromonas, Brucella, Francisella, Bordetella, Legionella, Bartonella, Coxiella, Haemophilus, Pasteurella, Mannheimia, Actinobacillus, Gardnerella, Spirochaetaceae (Treponema and Borrelia), Leptospiraceae, Campylobacter, Helicobacter, Spirillum, Streptobacillus, Bacteroidaceae (Bacteroides, Fusobacterium, Prevotella, Porphyromonas), Acinetobacter,* especially A. *baumanii.*

Preferably, the autolysin is encoded by Gram-negative bacteria such as Gram-negative bacteria of bacterial groups, families, genera or species comprising strains pathogenic for humans or animals as listed above.

The bacteriocin is preferably specific for Gram-negative bacteria as listed above, but may also be less specific.

The enzyme according to the present invention has cell wall degrading activity against Gram-negative bacteria of bacterial groups, families, genera or species comprising strains pathogenic for humans or animals as listed above.

In another preferred embodiment, the endolysin is encoded by bacteriophages specific for Gram-positive bacteria such as Gram-positive bacteria of bacterial groups, families, genera or species comprising strains pathogenic for humans or animals as listed in the following table.

**Table 1:**

| I. Phylum Actinobacteria | | |
|---|---|---|
| Class: | Actinobacteridae | |
| | Order | Actinomycetales |
| | | Families: |
| | | Actinomycineae: Actinomycetaceae (Actinomyces, Mobiluncus) |
| | | Corynebacterineae: Mycobacteriaceae (Mycobacterium), Nocardiaceae, |
| | | Corynebacteriaceae |
| | | Frankineae: Frankiaceae |
| | | Micrococcineae: Brevibacteriaceae |
| | | Propionibacteriaceae (Propionibacterium) |
| | | Order: Bifidobacteriales |
| | | Families: |
| | | Bifidobacteriaceae (Bifidobacterium, Falcivibrio, Gardnerella) |
| | | Other subclasses:Acidimicrobidae, Coriobacteridae, Rubrobacteridae, |
| | Sphaerobacteridae | |
| II. Phylum Firmicutes | | |
| Class: | Bacilli | |
| | Order: | Bacillales: |
| | | Families: |
| | | Bacillaceae (Bacillus), Listeriaceae (Listeria), Staphylococcaceae |
| | | (Staphylococcus, Gemella, Jeotgalicoccus) |
| | Order: | Lactobacillales: |
| | | Families: Enterococcaceae (Enterococcus), Lactobacillaceae |
| | | (Lactobacillus, Pediococcus), Leuconostocaceae (Leuconostoc), |
| | | Streptococcaceae (Lactococcus, Streptococcus) |
| Class: | | Clostridia |
| | Order: | Clostridiales (Clostridium, Peptostreptococcus, Selenomonas) |
| | Order: | Halanaerobiales |
| | Order: | Thermoanaerobacterales |
| | Class: | Tenericutes/Mollicutes |
| | Order: | Mycoplasmatales (Mycoplasma, Ureaplasma) |
| | Order: | Entomoplasmatales (Spiroplasma) |
| | Order: | Anaeroplasmatales (Erysipelothrix) |
| | Order: | Acholeplasmatales (Acholeplasma) |
| | Order: | Haloplasmatales (Haloplasma) |

In another preferred embodiment, the autolysin is encoded by Gram-positive bacteria such as Gram-positive bacteria of bacterial groups, families, genera or species comprising strains pathogenic for humans or animals as listed in table 1.

In another preferred embodiment, the bacteriocin is encoded by Gram-positive bacteria such as Gram-positive bacteria of bacterial groups, families, genera or species comprising strains pathogenic for humans or animals as listed in Table 1.

In a preferred embodiment, the enzyme according to the present invention has cell wall degrading activity against Gram-positive bacteria of bacterial groups, families, genera or species comprising strains pathogenic for humans or animals like *Listeria monocytogenes, Staphylococcus aureus, Enterococcus faecalis, Enterococcus faecium, Streptococcus pneumoniae, Streptococcus pyogenes, Streptococcus mutans, Streptococcus equi, Clostridium difficile, Clostridium botulinum, Clostridium tetani, Clostridium perfringens, Bacillus anthracis, Bacillus cereus, Propionibacterium acnes, Mycobacterium avium, Mycobacterium tuberculosis, Corynebacterium diphteriae, Mycoplasma pneumoniae, Actinomyces.*

Examples for the endolysin part are listed in the following table:

**Table 2:**

| phage | publication | Wild type endolysin | predicted function of the endolysin |
|---|---|---|---|
| ΦV₁₀ | Perry, L.L. and Applegate, B.M. | PhiV10p30 | chitinase |
| FELS-1 | McClelland, M. and Wilson, R.K. | STM0907.Fels0 | chitinase |
| ε15 | Kropinksi, A.M. and McConnel, M.R. | epsilon15p25 | chitinase |
| YUA | Ceyssens. P. (Laboratory for Gene technology) | YuA20 | lytic transglycosylase (C) / 1 transmembranair domain (N) |
| B3 | Braid, M.D. and Kitts, C.L. | ORF23 | lytic transglycosylase (C) / 2 transmembranair domains (N) |
| BCEPµ | Summer, E.J. and Young, R. | BcepMu22 | lytic transglycosylase (M) / 1 transmembranair domain (N) |
| F116 | Byrne, M. and Kropinski, A.M. | F116p62 | muraminidase (T4-like) |
| FELS-2 | McClelland, M. and Wilson, R.K. | STM271 S.S.Fels2 | muraminidase (T4-like) |
| ES18 | Casjens, S.R. and Hendrix, R.W. | gp76 | muraminidase (T4-like) |
| SETP3 | De Lappe, N and Cormican, M. | SPSV3_gp23 | muraminidase (T4-like) |
| OEC032 | Savalia, D and Severinov, K | phi32_17 | muraminidase (T4-like) |
| HK022 | Juhala , R and Hendrix, R.W. | HK022p54 | muraminidase (lambdalike) |
| HK97 | Juhala , R and Hendrix, R.W. | HK97p58 | muraminidase (lambdalike) |
| HK620 | Clark, A.J. and Dhillon, T.S. | HK620p36 | muraminidase (lambdalike) |
| E1 | Pickard, D. and Dougan, G | VIP0007 | muraminidase (lambdalike) |
| SF6 | Casjens, S and Clark, A.J. | Sf6p62 | muraminidase (lambdalike) |
| SFV | Allison, G.E. and Verma, N.K. | R (SfVp40) | muraminidase (lambdalike) |
| BCEPC6B | Summer, EJ and Young, R. | gp22 | muraminidase (lambdalike) |
| BCEPNAZGUL | Summer, EJ and Young, R. | Nazgul38 | muraminidase (lambdalike) |
| P2 | Christie, G.E. and Calender, R. | K (P2p09) | muraminidase (lambdalike) |
| WΦ | Christie, G.E. and Esposito, D. | K (Wphi09) | muraminidase (lambdalike) |
| RV5 | Kropinski, A.M. and Johnson | rv5_gp085 | muraminidase (lambdalike) |
| JS98 | Zuber, S and Denou, E. | EpJS98_gp116 | muraminidase (T4-like) |
| 13A | Savalia, D and Molineux, I. | gp3.5 | muramoyl-L-alanine amidase |
| BA14 | Savalia, D and Molineux, I. | gp3.5 | muramoyl-L-alanine amidase |
| ECODS1 | Savalia, D and Molineux, I. | gp3.5 | muramoyl-L-alanine amidase |
| K1F | Scholl, D and Merril, C | CKV1F_gp16 | muramoyl-L-alanine amidase |
| T3 | Pajunen, M.I. and Mollineux, I.J. | T3p18 | muramoyl-L-alanine amidase |
| GH-1 | Kropinski, A.M. and Kovalyova, I.V. | gh-1p12 | muramoyl-L-alanine amidase |
| K11 | Molineux, I. and Savalia, D. | gp3.5 | muramoyl-L-alanine amidase |
| ΦCTX | Nakayama, K and Hayashi, T. | ORF12 | PG-binding domain (N) / muramidase (C) |
| BCEP43 | Summer, EJ and Young, R. | Bcep43-27 | PG-binding domain (N) / muramidase (C) |
| BCEP781 | Summer, EJ and Young, R. | Bcep781-27 | PG-binding domain (N) / muramidase (C) |
| BCEP1 | Summer, EJ and Young, R. | Bcep1-28 | PG-binding domain (N) / muramidase (C) |
| BCEPNY3 | Summer, EJ and Young, R. | BcepNY3gene26 | PG-binding domain (N) / muramidase (C) |
| ΦE12-2 | DeShazer, D and Nierman, W.C. | gp45 | PG-binding domain (N) / muramidase (C) |
| Φ52237 | DeShazer, D and Nierman, W.C. | gp28 | PG-binding domain (N) / muramidase (C) |
| ΦP27 | Recktenwald, J and Schmidt, H. | P27p30 | endopeptidase |
| RB49 | Monod, C and Krisch, H.M. | RB49p102 | endopeptidase |
| Φ1 | Arbiol, C. and Comeau, A.M. | phi1-p102 | endopeptidase |
| T5 | Pankova, N.V. and Ksenzenko, V.N. | lys (T5.040) | endopeptidase |
| 201phi2-1 | Thomas et al., 2008 | | PG-binding domain (N) / unknown catalytic domain (C) |
| Aeh1 | Monod, C and Krisch, H.M. | Aeh1p339 | muraminidase (T4-like) |
| YYZ-2008 | Kropinski, A.M. | YYZgp45 | muraminidase (lambda-like) |

Also preferred is the endolysin part deriving from endolysins of the *Pseudomonas aeruginosa* phages ΦKZ and EL, of the *Pseudomonas putida* phage, of the *E. coli* phage N4, of the phage LUZ24, gp61 muramidase, STM0016 endolysin and PSP3 endolysin.

Further preferred endolysins are Listeria phage endolysins PlyA118, PlyA500, PlyPSA, PlyA511, PlyP35, PlyP40, Staphylococcal phage Phi 11 endolysin, Phi MR11 endolysin, LysK, *Clostridium perfringens* PlyS6, Ply3626, *Clostridium difficile:* CD27L endolysin, Streptococcus: B30 endolysin, phage Dp-1 Pal amidase, C1 endolysin, Cpl-1 endolysin, PlyGBS, Enterococccus: PlyV12, Bacillus anthracis: Phage gamma endolysin PlyG.

Preferred autolysins are described in: Bacterial peptidoglycan (murein) hydrolases. Vollmer W, Joris B, Charlier P, Foster S. FEMS Microbiol Rev. 2008 Mar;32(2):259-86. Epub 2008 Feb 11. Review. An example of a preferred autolysin is the AtlA Autolysine.

Preferred bacteriocins are Lysostaphin (degrading *Staphylococcus* cell walls), Mutanolysin (degrading *Streptococcus* cell walls) and Enterolysin (degrading *Enterococcus* cell walls).

Further examples for the endolysin part is selected from the group consisting of Cpl-1 according to SEQ ID NO: 1, Ply511 according to SEQ ID NO: 2, LysK according to SEQ ID NO: 3, Lysostaphin according to SEQ ID NO: 4, PA6-gp20 according to SEQ ID NO: 5, phiKZgp144 according to SEQ ID NO:6, ELgp188 according to SEQ ID NO:7, *Salmonella* endolysin according to SEQ ID NO:8, Enterobacteria phage T4 endolysin according to SEQ ID NO: 9, *Acinetobacter* baumanii endolysin according to SEQ ID NO:10, *E.coli* Phage K1F endolysin according to SEQ ID NO:11, OBPgpLYS according to SEQ ID NO:12, PSP3 *Salmonella* endolysin (PSP3gp10) according to SEQ ID NO:13, *E.coli* Phage P2 endolysin (P2gp09) according to SEQ ID NO:14, Salmonella typhimurium phage muramidase STM0016 according to SEQ ID NO:15, *E. coli* Phage N4 muramidase N4-gp61 according to SEQ ID NO:16 and N4-gp61 trunc. according to SEQ ID NO:17, KZ144 according to SEQ ID NO: 18.

In another preferred embodiment of the present invention the endolysins, autolysins and bacteriocins of the fusion protein according to the present invention comprise modifications and/or alterations of the amino acid sequences. Such alterations and/or modifications may comprise mutations such as deletions, insertions and additions, substitutions or combinations thereof and/or chemical changes of the amino acid residues, e.g. biotinylation, acetylation, pegylation, chemical changes of the amino-, SH- or carboxyl- groups. Said endolysins, autolysins and bacteriocins of the fusion protein according to the present invention exhibit the lytic activity of the respective wild-type endolysin, autolysin and bacteriocin. However, said activity can be the same, higher or lower as the activity of the respective wild-type endolysin, autolysin and bacteriocin. Said activity can be about 10, 20, 30, 40, 50, 60, 70, 80, 90, 100, 110, 120, 130, 140, 150, 160, 170, 180, 190 or about 200 % of the activity of the respective wild-type endolysin, autolysin and bacteriocin or even more. The activity can be measured by assays well known in the art by a person skilled in the art as e.g. the plate lysis assay or the liquid lysis assay which are e.g. described in Briers et al., J. Biochem. Biophys Methods 70: 531-533, (2007*)* or Donovan DM, Lardeo M, Foster-Frey J. FEMS Microbiol Lett. 2006 Dec; 265(1*)* or similar publications.

The peptide stretches of the fusion protein according to the present invention may be linked to the enzyme by additional amino acid residues e.g. due to cloning reasons. Preferably, said additional amino acid residues may be not recognized and/or cleaved by proteases. Preferably said peptide stretches may be linked to the enzyme by at least 1, 2, 3, 4, 5, 6, 7, 8, 9 or 10 additional amino acid residues. In a preferred embodiment the second peptide stretch is linked to the first peptide stretch which is fused to the N-terminus of the enzyme by the additional amino acid residues glycine, serine and serine (Gly-Ser-Ser).. Moreover, the second peptide stretch fused on the N-terminus of the first peptide stretch of the fusion protein according to the invention further comprises additional amino acids on its N-terminus. Preferably the peptide stretch comprises the amino acid methionine (Met), or methionine, glycine and serine (Met-Gly-Ser). In another preferred embodiment the first peptide stretch is linked to the N-terminus of the enzyme by the additional amino acid residues, in particular glycine and serine (Gly-Ser) and the second peptide stretch is linked to the N-terminus of the first peptide stretch by the additional amino acid residues, in particular glycine and serine (Gly-Ser). In another preferred embodiment the first peptide stretch is linked to the C-terminus of the enzyme by the additional amino acid residues, in particular glycine and serine (Gly-Ser) and the second peptide stretch is linked to the C-terminus of the first peptide stretch by the additional amino acid residues, in particular glycine and serine (Gly-Ser).

The peptide stretches of the fusion protein according to the present invention are preferably covalently bound to the enzyme. Preferably, said peptide stretches consist of at least 5, more preferably at least of 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or at least 100 amino acid residues. Especially preferred are peptide stretches comprising about 5 to about 100 amino acid residues, about 5 to about 50 or about 5 to about 30 amino acid residues. More preferred are peptide stretches comprising about 6 to about 42 amino acid residues, about 6 to about 39 amino acid residues, about 6 to about 38 amino acid residues, about 6 to about 31 amino acid residues, about 6 to about 25 amino acid residues, about 6 to about 24 amino acid residues, about 6 to about 22 amino acid residues, about 6 to about 21 amino acid residues, about 6 to about 20 amino acid residues, about 6 to about 19 amino acid residues, about 6 to about 16 amino acid residues, about 6 to about 14 amino acid residues, about 6 to about 12 amino acid residues, about 6 to about 10 amino acid residues or about 6 to about 9 amino acid residues.

Preferably, the peptide stretches are no tag such as a His-tag, Strep-tag, Avi-tag, Myc-tag, Gst-tag, JS-tag, cystein-tag, FLAG-tag or other tags known in the art and no thioredoxin or maltose binding proteins (MBP). However, the fusion protein according to the present invention may comprise in addition such tag or tags.

More preferably the peptide stretches have the function to lead the fusion protein through the outer membrane but may have activity or may have no or only low activity when administered without being fused to the enzyme. The function to lead the fusion protein through the outer membrane of Gram-negative bacteria is caused by the potential of the outer membrane or LPS disrupting or permeabilising or destabilizing activity of said peptide stretches. Such outer membrane or LPS disrupting or permeabilising or destabilizing activity of the peptide stretches may be determined in a method as follows: The bacteria cells to be treated are cultured in liquid medium or on agar plates. Then the bacteria cell concentration in the liquid medium is determined photometrically at OD600nm or the colonies on the agar plates are counted, respectively. Now, the bacteria cells in liquid medium or on the plates are treated with a fusion protein according to the invention. After incubation the bacteria cell concentration in the liquid medium is determined photometrically at OD600nm or the colonies on the agar plates are counted again. If the fusion protein exhibits such outer membrane or LPS disrupting or permeabilising or destabilizing activity, the bacteria cells are lysed due to the treatment with the fusion protein and thus, the bacteria cell concentration in the liquid medium or the number of the bacteria colonies on the agar plate is reduced. Thus, the reduction in bacteria cell concentration or in the number of bacteria colonies after treatment with fusion protein is indicative for an outer membrane or LPS disrupting or permeabilising or destabilizing activity of the fusion protein.

In a preferred embodiment the peptide stretches of the fusion protein according to the present invention are selected from the group of cationic peptides, polycationic peptides, hydrophobic peptides, antimicrobial peptides, sushi peptides and amphiphatic peptides. The two peptide stretches at the N- or C-terminus or at both termini of the fusion protein according to the invention may be two distinct cationic peptides or polycationic peptides or antimicrobial peptides or amphiphatic peptides or hydrophobic peptides. The two peptide stretches at the Nor C-terminus or at both termini of the fusion protein according to the invention may be any combination of a cationic peptide, a polycationic peptide, a hydrophobic peptide, an antimicrobial peptide, a sushi peptide and an amphiphatic peptide.

Especially preferred are cationic and/or polycationic peptide stretches comprising at least one motive according to SEQ ID NO: 19 (KRKKRK). In particular cationic peptide stretches comprising at least 2, 3, 4, 5, 6, 7, 8, 9, 10, 11, 12, 13, 14, 15, 16 or 17 motives according to SEQ ID NO: 19 (KRKKRK) are preferred. More preferred are cationic peptide stretches comprising at least one KRK motive (lys-arg-lys), preferable at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32 or 33 KRK motives.

In another preferred embodiment of the present invention the cationic peptide stretch comprises beside the positively charged amino acid residues, in particular lysine and/or arginine residues, neutrally charged amino acid residues, in particular glycine and/or serine residues. Preferred are cationic peptide stretches consisting of about 70 % to about 100 %, or about 80 % to about 95 %, or about 85 % to about 90 % positively charged amino acid residues, in particular lysine, arginine and/or histidine residues, more preferably lysine and/or arginine residues and of about 0 % to about 30 %, or about 5 % to about 20 %, or about 10 % to about 20 % neutrally charged amino acid residues, in particular glycine and/or serine residues. Preferred are polypeptide stretches consisting of about 4 % to about 8 % serine residues, of about 33 % to about 36 % arginine residues and of about 56 % to about 63 % lysine residues. Especially preferred are polypeptide stretches comprising at least one motive according to SEQ ID NO: 20 (KRXKR), wherein X is any other amino acid than lysine, arginine and histidine. Especially preferred are polypeptide stretches comprising at least one motive according to SEQ ID NO: 21 (KRSKR). More preferred are cationic stretches comprising at least about 2, 3, 4, 5, 6, 7, 8, 9, 10, 11, 12, 13, 14, 15, 16, 17, 18, 19 or about 20 motives according to SEQ ID NO: 20 (KRXKR) or SEQ NO: 21 (KRSKR).

Also preferred are polypeptide stretches consisting of about 9 to about 16 % glycine residues, of about 4 to about 11 % serine residues, of about 26 to about 32 % arginine residues and of about 47 to about 55 % lysine residues. Especially preferred are polypeptide stretches comprising at least one motive according to SEQ ID NO: 22 (KRGSG). More preferred are cationic stretches comprising at least about 2, 3, 4, 5, 6, 7, 8, 9, 10, 11, 12, 13, 14, 15, 16, 17, 18, 19 or about 20 motives according to SEQ ID NO: 22 (KRGSG).

In another preferred embodiment of the present invention the cationic peptide stretch comprises beside the positively charged amino acid residues, in particular lysine and/or arginine residues, hydrophobic amino acid residues, in particular valine, isoleucine, leucine, methionine, phenylalanine, tryptophan, cysteine, alanine, tyrosine, histidine, threonin, serine, proline and glycine residues, more preferably alanine, valine, leucine, isoleucine, phenylalanine, and/or tryptophan residues. Preferred are cationic peptide stretches consisting of about 70 % to about 100 %, or about 80 % to about 95 %, or about 85 % to about 90 % positively charged amino acid residues, in particular lysine and/or arginine residues and of about 0 % to about 30 %, or about 5 % to about 20 %, or about 10 % to about 20 % hydrophobic amino acid residues, valine, isoleucine, leucine, methionine, phenylalanine, tryptophan, cysteine, alanine, tyrosine, histidine, threonin, serine, proline and glycine residues, more preferably alanine, valine, leucine, isoleucine, phenylalanine, and/or tryptophan residues.

Examples for cationic and polycationic peptide stretches are listed in the following table:
Table 3:

| peptide stretch | length | SEQ ID NO: |
|---|---|---|
| KRKKRK | 6 | SEQ ID NO:19 |
| KRKKRKKRK | 9 | SEQ ID NO:23 |
| RRRRRRRRR | 9 | SEQ ID NO:24 |
| KKKKKKKK | 8 | SEQ ID NO:25 |
| KRKKRKKRKK | 10 | SEQ ID NO:26 |
| KRKKRKKRKKRK | 12 | SEQ ID NO:27 |
| KRKKRKKRKKRKKR | 14 | SEQ ID NO:28 |
| KKKKKKKKKKKKKKKK | 16 | SEQ ID NO:29 |
| KRKKRKKRKKRKKRKKRK | 18 | SEQ ID NO:30 |
| KRKKRKKRKKRKKRKKRKK | 19 | SEQ ID NO:31 |
| RRRRRRRRRRRRRRRRRRR | 19 | SEQ ID NO:32 |
| KKKKKKKKKKKKKKKKKKK | 19 | SEQ ID NO:33 |
| KRKKRKKRKRSKRKKRKKRK | 20 | SEQ ID NO:34 |
| KRKKRKKRKRSKRKKRKKRKK | 21 | SEQ ID NO:35 |
| KRKKRKKRKKRKKRKKRKKRK | 21 | SEQ ID NO:36 |
| KRKKRKKRKRGSGKRKKRKKRK | 22 | SEQ ID NO:37 |
| KRKKRKKRKRGSGSGKRKKRKKRK | 24 | SEQ ID NO:38 |
| KRKKRKKRKKRKKRKKRKKRKKRKK | 25 | SEQ ID NO:39 |
| KRKKRKKRKRSKRKKRKKRKRSKRKKRKKRK | 31 | SEQ ID NO:40 |
| KRKKRKKRKRGSGSGKRKKRKKRKGSGSGKRKKRKKRK | 38 | SEQ ID NO:41 |
| KRKKRKKRKKRKKRKKRKKRKKRKKRKKRKKRKKRKKRK | 39 | SEQ ID NO:42 |
| KRKKRKKRKRSKRKKRKKRKRSKRKKRKKRKRSKRKKRKKRK | 42 | SEQ ID NO:43 |

In a further aspect of the present invention at least one of the fused peptide stretches is an antimicrobial peptide, which comprises a positive net charge and around 50% hydrophobic amino acids. The antimicrobial peptides are amphiphatic, with a length of about 12 to about 50 amino acid residues. The antimicrobial peptides are naturally occurring in insects, fish, plants, arachnids, vertebrates or mammals. Preferably the antimicrobial peptide may be naturally occurring in radish, silk moth, wolf spider, frog, preferably in *Xenopus laevis, Rana* frogs, more preferably in *Rana catesbeiana,* toad, preferably Asian toad *Bufo bufo gargarizans,* fly, preferably in *Drosophila,* more preferably in *Drosophila melanogaster,* in *Aedes aegypti,* in honey bee, bumblebee, preferably in *Bombus pascuorum,* flesh fly, preferably in *Sarcophaga peregrine,* scorpion, horseshoe crab, catfish, preferably in *Parasilurus asotus,* cow, pig, sheep, porcine, bovine, monkey and human.

In another preferred embodiment of the present invention the antimicrobial peptide stretches consisting of about 0 % to about 5 %, or about 0 % to about 35 %, or about 10 % to about 35 % or about 15 % to about 45 %, or about 20 % to about 45 % positively charged amino acid residues, in particular lysine and/or arginine residues and of about 50 % to about 80 %, or about 60 % to about 80 %, or about 55 % to about 75 %, or about 70 % to about 90 hydrophobic amino acid residues, valine, isoleucine, leucine, methionine, phenylalanine, tryptophan, cysteine, alanine, tyrosine, histidine, threonin, serine, proline and glycine residues, more preferably alanine, valine, leucine, isoleucine, phenylalanine, and/or tryptophan residues.

In another preferred embodiment of the present invention the antimicrobial peptide stretches consisting of about 4 % to about 58 % positively charged amino acid residues, in particular lysine and/or arginine residues and of about 33 % to about 89 % hydrophobic amino acid residues, valine, isoleucine, leucine, methionine, phenylalanine, tryptophan, cysteine, alanine, tyrosine, histidine, threonin, serine, proline and glycine residues, more preferably alanine, valine, leucine, isoleucine, phenylalanine, and/or tryptophan residues.

Examples for antimicrobial peptides according to the present invention are listed in the following table.

**Table 4:**

| **Peptid** | **Sequenz** | |
|---|---|---|
| LL-37 | LLGDFFRKSKEKIGKEFKRIVQRIKDFLRNLVPRTES | SEQ ID NO:44 |
| SMAP-29 | RGLRRLGRKIAHGVKKYGPTVLRIIRIAG | SEQ ID NO:45 |
| Indolicidin | ILPWKWPWWPWRR | SEQ ID NO:46 |
| Protegrin | RGGRLCYCRRRFCVCVGR | SEQ ID NO:47 |
| Cecropin P1 | SWLSKTAKKLENSAKKRISEGIAIAIQGGPR | SEQ ID NO:48 |
| Magainin | GIGKFLHSAKKFGKAFVGEIMNS | SEQ ID NO:49 |
| Pleurocidin | GWGSFFKKAAHVGKHVGKAALTHYL | SEQ ID NO:50 |
| Cecropin A (A.aegypti) | GGLKKLGKKLEGAGKRVFNAAEKALPVVAGAKALRK | SEQ ID NO:51 |
| Cecropin A (D. melanogaster) | GWLKKIGKKIERVGQHTRDATIQGLGIPQQAANVAATA RG | SEQ ID NO:52 |
| Buforin II | TRSSRAGLQFPVGRVHRLLRK | SEQ ID NO:53 |
| Sarcotoxin IA | GWLKKIGKKIERVGQHTRDATIQGLGIAQQAANVAATA R | SEQ ID NO:54 |
| Apidaecin | ANRPVYIPPPRPPHPRL | SEQ ID NO:55 |
| Ascaphine 5 | GIKDWIKGAAKKLIKTVASHIANQ | SEQ ID NO:56 |
| Nigrocine 2 | GLLSKVLGVGKKVLCGVSGLVC | SEQ ID NO:57 |
| Pseudin 1 | GLNTLKKVFQGLHEAIKLINNHVQ | SEQ ID NO:58 |
| Ranalexin | FLGGLIVPAMICAVTKKC | SEQ ID NO:59 |
| Melittin | GIGAVLKVLTTGLPALISWIKRKRQQ | SEQ ID NO:60 |
| Lycotoxin 1 | IW LTALKFLGKHAAKKLAKQQLSKL | SEQ ID NO:61 |
| Parasin 1 | KGRGKQGGKVRAKAKTRSS | SEQ ID NO: 62 |
| Buforin I | AGRGKQGGKVRAKAKTRSSRAGLQFPVGRVHRLLRKGNY | SEQ ID NO:78 |
| Dermaseptin 1 | ALWKTMLKKLGTMALHAGKAALGAAADTISQGTQ | SEQ ID NO:79 |
| Bactenecin 1 | RLCRIVVIRVCR | SEQ ID NO:80 |
| Thanatin | GSKKPVPIIYCNRRTGKCQRM | SEQ ID NO:81 |
| Brevinin 1T | VNPIILGVLPKVCLITKKC | SEQ ID NO:82 |
| Ranateurin 1 | SMLSVLKNLGKVGLGFVACKINIKQC | SEQ ID NO:83 |
| Esculentin 1 | GIFSKLGRKKIKNLLISGLKNVGKEVGMDVVRTGIKIAGCKI KGEC | SEQ ID NO: 84 |
| Tachyplesin | RWCFRVCYRGICYRKCR | SEQ ID NO:85 |
| Androctonin | RSVCRCIKICRRRGGCYYKCTNRPY | SEQ ID NO:86 |
| alphadefensin | DCYCRIPACIAGERRYGTCIYQGRLWAFCC | SEQ ID NO:87 |
| beta-defensin | NPVSCVRNKGICVPIRCPGSMKQIGTCVGRAVKCCRKK | SEQ ID NO:88 |
| thetadefensin | GFCRCLCRRGVCRCICTR | SEQ ID NO:89 |
| defensin (sapecin A) | ATCDLLSGTGINHSACAAHCLLRGNRGGYCNGKAVCVCRN | SEQ ID NO:90 |
| Thionin (crambin) | TTCCPSIVARSNFNVCRIPGTPEAICATYTGCIIIPGATCPG DYAN | SEQ ID NO:91 |
| defensin from radish | QKLCQRPSGTWSGVCGNNNACKNQCIRLEKARHGSCNYVFPA HCICYFPC | SEQ ID NO:92 |
| Drosomvcin | DCLSGRYKGPCAVWDNETCRRVCKEEGRSSGHCSPSLKCWCE GC | SEQ ID NO:93 |
| Hepcidin | DTHFPICIFCCGCCHRSKCGMCCKT | SEQ ID NO:94 |
| Bac 5 | RFRPPIRRPPIRPPFYPPFRPPIRPPIFPPIRPPFRPPLGRP FP | SEQ ID NO:95 |
| PR-39 | RRRPRPPYLPRPRPPPFFPPRLPPRIPPGFPPRFPPRFP | SEQ ID NO:96 |
| Pyrrhocoricin | VDKGSYLPRPTPPRPIYNRN | SEQ ID NO:97 |
| Histatin 5 | DSHAKRHHGYKRKFHEKHHSHRGY | SEQ ID NO:98 |

In a further aspect of the present invention at least one of the fused peptide stretches is a sushi peptide which is described by Ding JL, Li P, Ho B Cell Mol Life Sci. 2008 Apr;65(7-8):1202-19. The Sushi peptides: structural characterization and mode of action against Gram-negative bacteria. Especially preferred is the sushi 1 peptide according to SEQ ID NO: 63.

Preferred sushi peptides are sushi peptides S 1 and S3 and multiples thereof; FASEB J. 2000 Sep;14(12):1801-13.

In a further aspect of the present invention at least one of the fused peptide stretches is a hydrophobic peptide, which comprises at least 90 % of the hydrophobic amino acid residues of valine, isoleucine, leucine, methionine, phenylalanine, tryptophan, cysteine, alanine, tyrosine, histidine, threonin, serine, proline and/or glycine. In another preferred embodiment the hydrophobic peptide fused to the fusion protein of the invention consist of about 90 % to about 95 %, or of about 90 to about 100%, or of about 95 to about 100 % of the hydrophobic amino acid residues of valine, isoleucine, leucine, methionine, phenylalanine, tryptophan, cysteine, alanine, tyrosine, histidine, threonin, serine, proline and/or glycine..

Preferred hydrophobic peptides are Walmagh1 having the amino acid sequence according to SEQ ID NO: 65and the hydrophobic peptide having the amino acid sequence Phe-Phe-Val-Ala-Pro (SEQ ID NO: 66)

In a further aspect of the present invention at least one of the fused peptide stretches is an amphiphatic peptide, which comprises one or more of the positively charged amino acid residues of lysine, arginine and/or histidine, combined to one or more of the hydrophobic amino acid residues of valine, isoleucine, leucine, methionine, phenylalanine, tryptophan, cysteine, alanine, tyrosine, histidine, threonin, serine, proline and/or glycine. Side chains of the amino acid residues are oriented in order that cationic and hydrophobic surfaces are clustered at opposite sides of the peptide. Preferably, more than about 30, 40, 50, 60 or 70% of the amino acids in said peptide are positively charged amino acids. Preferably, more than about 30, 40, 50, 60 or 70%, of the amino acid residues in said peptide are hydrophobic amino acid residues. Advantageously, the amphiphatic peptide is fused at the N-terminal and/or the C-terminal end of the enzyme having cell wall degrading activity, thus enhancing the amphiphaticity of the latter proteins.

In another embodiment of the invention, the amphiphatic peptide consists of at least 5, more preferably at least of 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 amino acid residues. In a preferred embodiment at least about 30, 40, 50, 60 or 70% of the said amino acid residues of the amphiphatic peptide are either arginine or lysine residues and/or at least about 30, 40, 50, 60 or 70% of the said amino acid residues of the amphiphatic peptide are of the hydrophobic amino acids valine, isoleucine, leucine, methionine, phenylalanine, tryptophan, cysteine, alanine, tyrosine, histidine, threonin, serine, proline and/or glycine.

In another preferred embodiment of the present invention the amphiphatic peptide stretch comprises beside the positively charged amino acid residues, in particular lysine and/or arginine residues, hydrophobic amino acid residues, in particular valine, isoleucine, leucine, methionine, phenylalanine, tryptophan, cysteine, alanine, tyrosine, histidine, threonin, serine, proline and glycine residues, more preferably alanine, valine, leucine, isoleucine, phenylalanine, and/or tryptophan residues. Preferred are amphiphatic peptide stretches consisting of about 10 % to about 50 %, or about 20 % to about 50 %, or about 30 % to about 45 % or about 5 % to about 30 % positively charged amino acid residues, in particular lysine and/or arginine residues and of about 50 % to about 85 %, or about 50 % to about 90 %, or about 55 % to about 90 %, or about 60 % to about 90 %, or about 65 % to about 90 % hydrophobic amino acid residues, valine, isoleucine, leucine, methionine, phenylalanine, tryptophan, cysteine, alanine, tyrosine, histidine, threonin, serine, proline and glycine residues, more preferably alanine, valine, leucine, isoleucine, phenylalanine, and/or tryptophan residues. In another preferred embodiment amphiphatic peptide stretches consisting of 12 % to about 50 % positively charged amino acid residues, in particular lysine and/or arginine residues and of about 50 % to about 85 hydrophobic amino acid residues, valine, isoleucine, leucine, methionine, phenylalanine, tryptophan, cysteine, alanine, tyrosine, histidine, threonin, serine, proline and glycine residues, more preferably alanine, valine, leucine, isoleucine, phenylalanine, and/or tryptophan residues.

Preferred amphiphatic peptides are, α4-helix of T4 lysozyme according to SEQ ID NO: 68 and WLBU2-Variant having the amino acid sequence according to SEQ ID NO: 64 and Walmagh 2 according to SEQ ID NO: 69.

In another preferred embodiment of the present invention the peptide stretches of the fusion protein according to the present invention comprise modifications and/or alterations of the amino acid sequences. Such alterations and/or modifications may comprise mutations such as deletions, insertions and additions, substitutions or combinations thereof and/or chemical changes of the amino acid residues, e.g. biotinylation, acetylation, peglyation, chemical changes of the amino-, SH- or carboxyl- groups.

In a preferred embodiment the two peptide stretches of the fusion protein on the N- or C-terminus or at both termini according to the present invention consist of a polycationic peptide combined with an amphiphatic peptide. Preferably, the polycationic peptide is fused to the N-terminus of the enzyme, wherein the amphiphatic peptide is fused to the N-terminus of the polycationic peptide. The amphiphatic peptide may be the α4-helix of T4-lysozyme according to SEQ ID NO: 68, WLBU2-Variant according to SEQ ID NO: 64 or Walmagh 2 according to SEQ ID NO: 69. In a preferred embodiment the polycationic peptide may be a peptide according to SEQ ID NO: 23 or a peptide according to SEQ ID NO: 34. In another preferred embodiment the amphiphatic peptide may be α4-helix of T4-lysozyme according to SEQ ID NO: 68 and the polycationic peptide may be a peptide according to SEQ ID NO: 23. In another preferred embodiment the amphiphatic peptide may be Walmagh 2 according to SEQ ID NO: 69 and the polycationic peptide may be a peptide according to SEQ ID NO: 23. In another preferred embodiment the amphiphatic peptide may be WLBU2-Variant according to SEQ ID NO: 64 and the polycationic peptide may be a peptide according to SEQ ID NO: 23.

In another preferred embodiment the amphiphatic peptide may be the α4-helix of T4-lysozyme according to SEQ ID NO: 68 and the polycationic peptide may be a peptide according to SEQ ID NO: 23, wherein the polycationic peptide is fused to the N-Terminus of the enzyme or to the N-Terminus of the amphiphatic peptide. In another preferred embodiment the hydrophobic peptide may be Walmagh 2 according to SEQ ID NO: 69 and the polycationic peptide may be a peptide according to SEQ ID NO: 23, wherein the polycationic peptide is fused to the N-Terminus of the enzyme or to the N-Terminus of the amphiphatic peptide. In another preferred embodiment the hydrophobic peptide may be WLBU2-Variant according to SEQ ID NO: 64 and the polycationic peptide may be a peptide according to SEQ ID NO: 23, wherein the polycationic peptide is fused to the N-Terminus of the enzyme or to the N-Terminus of the amphiphatic peptide

In a preferred embodiment the two peptide stretches of the fusion protein on the N- or C-terminus or at both termini according to the present invention consist of a polycationic peptide combined with a hydrophobic peptide. Preferably, the polycationic peptide is fused to the N-terminus of the enzyme, wherein the hydrophobic peptide is fused to the N-terminus of the polycationic peptide. In a preferred embodiment the hydrophobic peptide may be the pentapeptide according to SEQ ID NO: 66 or Walmagh 1 according to SEQ ID NO: 65. In a preferred embodiment the polycationic peptide may be a peptide according to SEQ ID NO: 23 or a peptide according to SEQ ID NO: 34. In another preferred embodiment the hydrophobic peptide may be the pentapeptide according to SEQ ID NO: 66 and the polycationic peptide may be a peptide according to SEQ ID NO: 23. In another preferred embodiment the hydrophobic peptide may be Walmagh 1 according to SEQ ID NO: 65 and the polycationic peptide may be a peptide according to SEQ ID NO: 23.

In another preferred embodiment the hydrophobic peptide may be the pentapeptide according to SEQ ID NO: 66 and the polycationic peptide may be a peptide according to SEQ ID NO: 23, wherein the polycationic peptide is fused to the N-Terminus of the enzyme or to the N-Terminus of the hydrophobic peptide. In another preferred embodiment the hydrophobic peptide may be Walmagh 1 according to SEQ ID NO: 65 and the polycationic peptide may be a peptide according to SEQ ID NO: 23, wherein the polycationic peptide is fused to the N-Terminus of the enzyme or to the N-Terminus of the hydrophobic peptide.

In another preferred embodiment the two peptide stretches of the fusion protein on the N- or C-terminus or at both termini according to the present invention consist of a polycationic peptide combined with an antimicrobial peptide. Preferably, the polycationic peptide is fused to the N-terminus of the enzyme, wherein the antimicrobial peptide is fused to the N-terminus of the polycationic peptide. Preferably, the antimicrobial peptide may be Parasin 1 according to SEQ ID NO: 62 or Lycotoxin 1 according to SEQ ID NO: 61. In a preferred embodiment the polycationic peptide may be a peptide according to SEQ ID NO: 23 or a peptide according to SEQ ID NO: 34. In another preferred embodiment the antimicrobial peptide may be Parasin 1 according to SEQ ID NO: 62 and the polycationic peptide may be a peptide according to SEQ ID NO: 23. In another preferred embodiment the hydrophobic peptide may be Lycotoxin 1 according to SEQ ID NO: 61 and the polycationic peptide may be a peptide according to SEQ ID NO: 23.

In another preferred embodiment the antimicrobial peptide may be Parasin 1 according to SEQ ID NO: 62 and the polycationic peptide may be a peptide according to SEQ ID NO: 23, wherein the polycationic peptide is fused to the N-Terminus of the enzyme or to the N-Terminus of the antimicrobial peptide. In another preferred embodiment the antimicrobial peptide may be Lycotoxin 1 according to SEQ ID NO: 61 and the polycationic peptide may be a peptide according to SEQ ID NO: 23, wherein the polycationic peptide is fused to the N-Terminus of the enzyme or to the N-Terminus of the antimicrobial peptide.

In a preferred embodiment of the present invention the fusion protein consists of two peptide stretches according to SEQ ID NO: 19 to 69 and 78 to 98 and an enzyme according to SEQ ID NO: 1 to 18. In a preferred embodiment of the present invention the fusion protein comprises two peptide stretches selected from the group of peptide stretches according to SEQ ID NO: 19 to 69 and 78 to 98 and an enzyme selected from the group of enzymes according to SEQ ID NO: 1 to 18.

Specific examples of fusion proteins according to the present invention are listed in the following table: [[SEquenzen ohne Linker]]

**Table 3:**

| Fusion protein | First peptide stretch (N-terminal unless otherwise indicated) | Second peptide stretch (N-terminal unless otherwise indicated) | Enzyme part |
|---|---|---|---|
| SEQ ID NO: 70 | Pentapeptide (SEQ ID NO: 66) | polycationic peptide (SEQ ID NO: 23) | OBPgpLys (SEQ ID NO: 12) |
| SEQ ID NO: 76 | polycationic peptide (SEQ ID NO: 23) | Pentapeptide (SEQ ID NO: 66) | OBPgpLys (SEQ ID NO: 12) |
| SEQ ID NO: 71 | α4-helix of T4-lysozyme (SEQ ID NO: 68) | polycationic peptide (SEQ ID NO: 23) | OBPgpLys (SEQ ID NO: 12) |
| SEQ ID NO: 77 | polycationic peptide (SEQ ID NO: 23) | α4-helix of T4-lysozyme(SEQ ID NO: 68) | OBPgpLys (SEQ ID NO: 12) |
| SEQ ID NO: 72 | Walmagh 1 (SEQ ID NO: 65) | polycationic peptide (SEQ ID NO: 23) | OBPgpLys (SEQ ID NO: 12) |
| SEQ ID NO: 73 | Walmagh 2 (SEQ ID NO: 69) | polycationic peptide (SEQ ID NO: 23) | OBPgpLys (SEQ ID NO: 12) |
| SEQ ID NO: 74 | Parasin 1 (SEQ ID NO: 62) | polycationic peptide (SEQ ID NO: 23) | OBPgpLys (SEQ ID NO: 12) |
| SEQ ID NO: 75 | Lycotoxin 1 (SEQ ID NO: 61) | polycationic peptide (SEQ ID NO: 23) | OBPgpLys (SEQ ID NO: 12) |
| SEQ ID NO: 127 | Polycationic peptide (SEQ ID NO: 23) | SMAP-29 (SEQ ID NO: 45) | KZ144 (SEQ ID NO: 18) |
| SEQ ID NO: 128 | SMAP-29 (SEQ ID NO: 45) | Polycationic peptide (SEQ ID NO: 23) | EL188 (SEQ ID NO: 7) |
| SEQ ID NO: 129 | SMAP-29 (SEQ ID NO: 45) | SMAP-29 (SEQ ID NO: 45) | KZ144 (SEQ ID NO: 18) |
| SEQ ID NO: 130 | SMAP-29 (SEQ ID NO: 45) | LL-37 (SEQ ID NO: 44) | N4gp61 (SEQ ID NO: 16) |
| SEQ ID NO: 131 | Sarcotoxin IA (SEQ NO: 54) | SMAP-29 (SEQ ID NO: 45) | Lysostaphin (SEQ ID NO: 4) |
| SEQ ID NO: 132 | (C-terminal of the enzyme) Sarcotoxin IA (SEQ ID NO: 54) | (C-terminal of the first peptide-stretch) SMAP-29 (SEQ ID NO: 45) | Lysostaphin (SEQ ID NO: 4) |
| SEQ ID NO: 133 | Melittin (SEQ ID NO: 60) | SMAP-29 (SEQ ID NO: 45) | N4gp61 (SEQ ID NO: 16) |
| SEQ ID NO: 134 | LL-37 (SEQ ID NO: 44) | Pseudin-1 (SEQ ID NO: 58) | Cp1-1 (SEQ ID NO: 1) |
| SEQ ID NO: 135 | Magainin (SEQ ID NO: 49) | Buforin II (SEQ ID NO: 53) | Ply511 (SEQ ID NO: 2) |

The fusion protein according to the present invention, and thus in particular the especially preferred fusion proteins according to SEQ ID NO: to 77 and 127 to 135, may additional comprise a methionine on the N-terminus.

The fusion protein according to the present invention, and thus in particular the especially preferred fusion proteins according to SEQ ID NO: 70 to 77 and 127 to 135 may additional comprise a tag e.g. for purification. Preferred is a His₆-tag, preferably at the C-terminus and/or the N-terminus of the fusion protein. Said tag can be linked to the fusion protein by additional amino acid residues e.g. due to cloning reasons. Preferably said tag can be linked to the fusion protein by at least 1, 2, 3, 4, 5, 6, 7, 8, 9 or 10 additional amino acid residues. Preferably said additional amino acid residues may not be recognized and/or cleaved by proteases.In a preferred embodiment the fusion protein comprises a His₆-tag at its C-terminus linked to the fusion protein by the additional amino acid residues lysine and glycine (Lys-Gly) or leucine and glutamic acid (Leu-Glu). Preferably, said additional amino acid residues may be not recognized or cleaved by proteases. In another preferred embodiment the fusion protein comprises a His₆-tag at its N-terminus linked to the fusion protein by the additional amino acid residues lysine and glycine (Lys-Gly) or leucine and glutamic acid (Leu-Glu). In another preferred embodiment the fusion protein comprises a His₆-tag at its N- and C-terminus linked to the fusion protein by the additional amino acid residues lysine and glycine (Lys-Gly) or leucine and glutamic acid (Leu-Glu).

In a more preferred embodiment the fusion protein comprises a His₆-tag at its C-terminus linked to the fusion protein by the additional amino acid residues leucine and glutamic acid (Leu-Glu) and the second peptide stretch of the fusion protein according to the invention is linked to the N-terminus of the first peptide stretch by the additional amino acid residues glycine and serine. In another preferred embodiment the fusion protein comprises a His₆-tag at its C-terminus linked to the fusion protein by the additional amino acid residues leucine and glutamic acid (Leu-Glu) and the second peptide stretch of the fusion protein according to the invention is linked to the N-terminus of the first peptide stretch by the additional amino acid residues glycine and serine (Gly-Ser) and the fusion protein comprises on the N-terminus the additional amino acid residues methionine (Met) or methionine and glycine (Met-Gly) or methionine, glycine and serine (Met-Gly-Ser). In another preferred embodiment the fusion protein comprises a His₆-tag at its C-terminus linked to the fusion protein by the additional amino acid residues leucine and glutamic acid (Leu-Glu) and the second peptide stretch of the fusion protein according to the invention is linked to the N-terminus of the first peptide stretch by the additional amino acid residues glycine and serine (Gly-Ser) and the first peptide stretch is fused to the N-terminus of the enzyme by the additional amino acid residues glycine and serine (Gly-Ser). In another preferred embodiment the fusion protein comprises a His₆-tag at its C-terminus linked to the fusion protein by the additional amino acid residues leucine and glutamic acid (Leu-Glu) and the second peptide stretch of the fusion protein according to the invention is linked to the N-terminus of the first peptide stretch by the additional amino acid residues glycine and serine (Gly-Ser) and the first peptide stretch is fused to the N-terminus of the enzyme by the additional amino acid residues glycine and serine (Gly-Ser) and the fusion protein comprises on the N-terminus the additional amino acid residues methionine (Met) or methionine and glycine (Met-Gly) or methionine, glycine and serine (Met-Gly-Ser).

Fusion proteins are constructed by linking at least three nucleic acid sequences using standard cloning techniques as described e.g. by Sambrook et al. 2001, Molecular Cloning: A Laboratory Manual. Such a protein may be produced, e.g., in recombinant DNA expression systems. Such fusion proteins according to the present invention can be obtained by fusing the nucleic acids for endolysin and the respective peptide stretches.

The fusion proteins according to the present invention may be fused or linked to other additional proteins. Example for this other additional protein is thioredoxin.

The present invention further relates to an isolated nucleic acid molecule encoding the fusion protein according to the present invention. The present invention further relates to a vector comprising the nucleic acid molecule according to the present invention. Said vector may provide for the constitutive or inducible expression of said fusion protein according to the present invention.

The invention also relates to a method for obtaining said fusion proteins from a microorganism, such as a genetically modified suitable host cell which expresses said fusion proteins. Said host cell may be a micro-organism such as bacteria or yeast or an animal cell as e.g. a mammalian cell, in particular a human cell. In one embodiment of the present invention the host cell is a *Pichia pastoris* cell. The host may be selected due to mere biotechnological reasons, e.g. yield, solubility, costs, etc. but may be also selected from a medical point of view, e.g. a non-pathological bacteria or yeast, human cells.

Another aspect of the present invention is related to a method for genetically transforming a suitable host cell in order to obtain the expression of the fusion proteins according to the invention wherein the host cell is genetically modified by the introduction of a genetic material encoding said fusion proteins into the host cell and obtain their translation and expression by genetic engineering methods well known by the man skilled in the art.

In a further aspect the present invention relates to a composition, preferably a pharmaceutical composition, comprising a fusion protein according to the present invention and/or a host transformed with a nucleic acid molecule or a vector comprising a nucleotide sequence encoding a fusion protein according to the present invention.

In a preferred embodiment of the present invention the composition comprises additionally agents permeabilizing the outer membrane of Gram-negative bacteria such metal chelators as e.g. EDTA, TRIS, lactic acid, lactoferrin, polymyxin, citric acid and/or other substances as described e.g. by Vaara (Agents that increase the permeability of the outer membrane. Vaara M. Microbiol. Rev. 1992 Sep; 56 (3):395-441). Also preferred are compositions comprising combinations of the above mentioned permeabilizing agents. Especially preferred is a composition comprising about 10 µM to about 100 mM EDTA, more preferably about 50 µM to about 10 mM EDTA, more preferably about 0.5 mM to about 10 mM EDTA, more preferably about 0.5 mM to about 2 mM EDTA, more preferably about 0.5 mM to 1 mM EDTA. However, also compositions comprising about 10 µM to about 0.5 mM EDTA are preferred. Also preferred is a composition comprising about 0.5 mM to about 2 mM EDTA, more preferably about 1 mM EDTA and additionally about 10 to about 100 mM TRIS.

The present invention also relates to a fusion protein according to the present invention and/or a host transformed with a nucleic acid comprising a nucleotide sequence encoding a fusion protein according to the present invention for use as a medicament. In a further aspect the present invention relates to the use of a fusion protein according to the present invention and/or a host transformed with a vector comprising a nucleic acid molecule comprising a nucleotide sequence encoding a modified, fusion protein according to the present invention in the manufacture of a medicament for the treatment and/or prevention of a disorder, disease or condition associated with Gram-positive and/or Gram-negative bacteria. In particular the treatment and/or prevention of the disorder, disease or condition may be caused by Gram-negative bacteria of bacterial groups, families, genera or species comprising strains pathogenic for humans or animals like *Enterobacteriaceae (Escherichia,* especially *E. coli, Salmonella, Shigella, Citrobacter, Edwardsiella, Enterobacter, Hafnia, Klebsiella, especially K. pneumoniae, Morganella, Proteus, Providencia, Serratia, Yersinia), Pseudomonadaceae (Pseudomonas, especially P. aeruginosa, Burkholderia, Stenotrophomonas, Shewanella, Sphingomonas, Comamonas), Neisseria, Moraxella, Vibrio, Aeromonas, Brucella, Francisella, Bordetella, Legionella, Bartonella, Coxiella, Haemophilus, Pasteurella, Mannheimia, Actinobacillus, Gardnerella, Spirochaetaceae (Treponema and Borrelia), Leptospiraceae, Campylobacter, Helicobacter, Spirillum, Streptobacillus, Bacteroidaceae (Bacteroides, Fusobacterium, Prevotella, Porphyromonas), Acinetobacter,* especially A. *baumanii.* In particular the treatment and/or prevention of the disorder, disease or condition may be caused by Gram-positive bacteria of bacterial groups, families, genera or species comprising strains pathogenic for humans or animals like *Listeria monocytogenes, Staphylococcus aureus, Enterococcus faecalis, Enterococcus faecium, Streptococcus pneumoniae, Streptococcus pyogenes, Streptococcus mutans, Streptococcus equi, Clostridium difficile, Clostridium botulinum, Clostridium tetani, Clostridium perfringens, Bacillus anthracis, Bacillus cereus, Propionibacterium acnes, Mycobacterium avium, Mycobacterium tuberculosis, Corynebacterium diphteriae, Mycoplasma pneumoniae, Actinomyces.*

The present invention further relates to a medicament comprising a fusion protein according to the present invention and/or a host transformed with a nucleic acid comprising a nucleotide sequence encoding a fusion protein according to the present invention.

In a further aspect the present invention relates to a method of treating a disorder, disease or condition in a subject in need of treatment and/or prevention, which method comprises administering to said subject an effective amount of a fusion protein according to the present invention and/or an effective amount of a host transformed with a nucleic acid comprising a nucleotide sequence encoding a fusion protein according to the present invention or a composition according to the present invention. The subject may be a human or an animal.

In particular said method of treatment may be for the treatment and/or prevention of infections of the skin, of soft tissues, the respiratory system, the lung, the digestive tract, the eye, the ear, the teeth, the nasopharynx, the mouth, the bones, the vagina, of wounds of bacteraemia and/or endocarditis caused by Gram-positive and/or Gram-negative bacteria, in particular by the Gram-positive and/or Gram-negative bacteria as listed above.

The dosage and route of administration used in a method of treatment (or prophylaxis) according to the present invention depends on the specific disease/site of infection to be treated. The route of administration may be for example oral, topical, nasopharyngeal, parenteral, intravenous, rectal or any other route of administration.

For application of a fusion protein according to the present invention and/or an effective amount of a host transformed with a nucleic acid comprising a nucleotide sequence encoding a fusion protein according to the present invention or a composition according to the present invention to a site of infection (or site endangered to be infected) a formulation may be used that protects the active compounds from environmental influences such as proteases, oxidation, immune response etc., until it reaches the site of infection. Therefore, the formulation may be capsule, dragee, pill, powder, suppository, emulsion, suspension, gel, lotion, cream, salve, injectable solution, syrup, spray, inhalant or any other medical reasonable galenic formulation. Preferably, the galenic formulation may comprise suitable carriers, stabilizers, flavourings, buffers or other suitable reagents. For example, for topical application the formulation may be a lotion, cream, gel, salve or plaster, for nasopharyngeal application the formulation may be saline solution to be applied via a spray to the nose. For oral administration in case of the treatment and/or prevention of a specific infection site e.g. in the intestine, it can be necessary to protect a fusion protein according to the present invention from the harsh digestive environment of the gastrointestinal tract until the site of infection is reached. Thus, bacteria as carrier, which survive the initial steps of digestion in the stomach and which secret later on a fusion protein according to the present invention into the intestinal environment can be used.

In a specific embodiment of the present invention the use of a fusion protein according to the present invention and/or a host transformed with a vector comprising a nucleic acid molecule comprising a nucleotide sequence encoding a fusion protein according to the present invention in the manufacture of a medicament for the treatment and/or prevention of a disorder, disease or condition caused by *Pseudomonas,* particularly by *Pseudomonas aeruginosa* in particular intestinal affections, in particular in infants, infections of the meninges, e.g. meningitis haemorrhagica, infections of the middle ear, the skin (Ecthyma gangraenosum), in particular burns, the urinary tract, rhinitis, bacteremic pneumonia, in particular wherein the patient is suffering from cystic fibrosis or hematologic malignancies such as leukemia, or with neutropenia from immunosuppressive therapy, septicemia, in particular because of long-term intravenous or urinary catheterization, invasive surgical procedures and severe burns, endocarditis, in particular wherein the patient is a intravenous drug user or a patient with complications from open heart surgery, highly destructive ocular infections, in particular after the use of contaminated ophthalmologic solutions or severe facial burns, osteochondritis, in particular as a result of severe trauma or puncture wounds through contaminated clothing.

In another specific embodiment of the present invention the disorder, disease or condition is caused by *Burkholderia pseudomallei,* in particular Whitmore's Disease, chronic pneumonia, septicemia, in particular wherein the patient has a traumatized skin lesion.

In another specific embodiment of the present invention the disorder, disease or condition is caused by *Salmonella thyphimurium* and *Salmonella enteritidis,* in particular acute gastroenteritis and local purulent processes, particularly osteomyelitis, endocarditis, cholecystitis and especially caused by *Salmonella thyphimurium* meningitis, in particular wherein the patient is less than two years old.

In another specific embodiment of the present invention the disorder, disease or condition is caused by *Salmonella typhi,* in particular typus.

In another specific embodiment of the present invention the disorder, disease or condition is caused by *Salmonell paratyphi,* in particular paratyphus.

In another specific embodiment of the present invention the disorder, disease or condition is caused by *Acinetobacter baumannii,* in particular bronchitis, pneumonia, wound infections and septicemia, in particular as a result of intravenous catheterization.

In another specific embodiment of the present invention the disorder, disease or condition is caused by *Escherichia coli,* in particular extra intestinal infections, particularly appendicitis, purulent cholecystitis, peritonitis, purulent meningitis and infection of the urinary tract, intraintestinal *E. coli* infections, particularly epidemic enteritis, and infectious disease similar to dysentery, septicemia, enterotoxemia, mastitis and dysentery.

In another specific embodiment of the present invention the disorder, disease or condition is caused by *Klebsiella pneumoniae,* in particular pneumonia, bacteremia, meningitis and infections of the urinary tract.

In a specific embodiment of the present invention the use of a fusion protein according to the present invention and/or a host transformed with a vector comprising a nucleic acid molecule comprising a nucleotide sequence encoding a fusion protein according to the present invention in the manufacture of a medicament for the treatment and/or prevention of a disorder, disease or condition caused by *Listeria monocytogenes,* in particular Granulomatosis infantiseptica (listeriosis of newborns), mononucleosis, conjunctivitis, meningitis, granulomatosis septica and the listeriosis of pregnant women.

In another specific embodiment of the present invention the disorder, disease or condition is caused by *Staphylococcus aureus,* in particular infections of the skin like pyoderma, particularly folliculitis, furuncle, carbuncle, abscesses of the sweat glands and pemphigus, and like scaled skin syndrome. The scaled skin syndrome can appear in three clinical pictures: dermatitis exfoliativa, impetigo bullosa and scarlatiniform erythroderma. Moreover the disorder, disease or condition caused by *Staphylococcus aureus* is *Staphylococcus* pneumonia, hospitalism, in particular surgical wound infections, mastitis puerperalis and enterokolitis, and food poisonings.

In another specific embodiment of the present invention the disorder, disease or condition is caused by *Streptococcus pyogenes,* in particular tonsillitis, pharyngitis, scarlet, erysipelas, rheumatic fever and acute glomerulonephritis.

In another specific embodiment of the present invention the disorder, disease or condition is caused by Streptococcus pneumoniae, in particular pneumonia, ulcus serpens corneae, otitis media, meningitis, peritonitis, mastoiditis and osteomyelitis.

In another specific embodiment of the present invention the disorder, disease or condition is caused by *Clostridium perfringens,* in particular gas gangrene, enteritis necroticans ulcerosa and food poisonings.

In another specific embodiment of the present invention the disorder, disease or condition is caused by *Clostridium botulinum,* in particular botulism.

In another specific embodiment of the present invention the disorder, disease or condition is caused by *Clostridium difficile,* in particular pseudomembranoes enterokolitis.

In another specific embodiment of the present invention the disorder, disease or condition is caused by *Bacillus anthracis,* in particular cutaneous anthrax, inhalation anthrax, and gastrointestinal anthrax.

In another specific embodiment of the present invention the disorder, disease or condition is caused by *Enterococcus faecalis* or *E. faecium,* like nosokomial infections, and endokarditis.

In another specific embodiment of the present invention the disorder, disease or condition is caused by *Bacillus cereus,* in particular food poisonings, bronchial pneumonia, septicaemia and meningitis.

In another specific embodiment of the present invention the disorder, disease or condition is caused by *Mycobacterium avium, Mycobacterium paratuberculosis* and *Mycobacterium tuberculosis,* in particular tuberculosis.

In another specific embodiment of the present invention the disorder, disease or condition is caused by *Mycoplasma pneumoniae,* in particular pneumonia, diseases of the upper respiratory tract and inflammations of the ear drum.

In another specific embodiment of the present invention the disorder, disease or condition is caused by *Actinomyces,* in particular actinomycosis in human, cattle, cat and dog.

In another specific embodiment of the present invention the disorder, disease or condition is caused by *Corynebacterium diphteriae,* in particular localized diphtheria of the tonsils, the nose, the nasopharynx or the middle ear, progressive diphtheria of the larynx, the trachea and the bronchi, toxic or maligne diphtheria, skin and wound diphtheria.

Preferably, a fusion protein according to the present invention is used for medical treatment, if the infection to be treated (or prevented) is caused by multiresistant bacterial strains, in particular by strains resistant against one or more of the following antibiotics: streptomycin, tetracycline, cephalothin, gentamicin, cefotaxime, cephalosporin, ceftazidime or imipenem. Furthermore, a fusion protein according to the present invention can be used in methods of treatment by administering it in combination with conventional antibacterial agents, such as antibiotics, lantibiotics, bacteriocins or endolysins, etc.

The present invention also relates to a pharmaceutical pack comprising one or more compartments, wherein at least one compartment comprises one or more fusion protein according to the present invention and/or one or more hosts transformed with a nucleic acid comprising a nucleotide sequence encoding a fusion protein according to the present invention or a composition according to the present invention.

In another aspect the present invention relates to a process of preparation of a pharmaceutical composition, said process comprising admixing one or more fusion protein according to the present invention and/or one or more hosts transformed with a nucleic acid comprising a nucleotide sequence encoding a fusion protein according to the present invention with a pharmaceutically acceptable diluent, excipient or carrier.

In an even further aspect the composition according to the present invention is a cosmetic composition. Several bacterial species can cause irritations on environmentally exposed surfaces of the patient's body such as the skin. In order to prevent such irritations or in order to eliminate minor manifestations of said bacterial pathogens, special cosmetic preparations may be employed, which comprise sufficient amounts of the fusion protein according to the present invention in order to degrade already existing or freshly settling pathogenic Gram-negative bacteria.

In a further aspect the present invention relates to the fusion protein according to the present invention for use as diagnostic means in medicinal, food or feed or environmental diagnostics, in particular as a diagnostic means for the diagnostic of bacteria infection caused in particular by Gram-positive and/or Gram-negative bacteria. In this respect the fusion protein according to the present invention may be used as a tool to specifically degrade pathogenic bacteria, in particular Gram-positive and/or Gram-negative pathogenic bacteria. The degradation of the bacterial cells by the fusion protein according to the present invention can be supported by the addition of detergents like Triton X-100 or other additives which weaken the bacterial cell envelope like polymyxin B. Specific cell degradation is needed as an initial step for subsequent specific detection of bacteria using nucleic acid based methods like PCR, nucleic acid hybridization or NASBA (Nucleic Acid Sequence Based Amplification), immunological methods like IMS, immunofluorescence or ELISA techniques, or other methods relying on the cellular content of the bacterial cells like enzymatic assays using proteins specific for distinct bacterial groups or species (e.g. β-galactosidase for enterobacteria, coagulase for coagulase positive strains).

In a further aspect the present invention relates to the use of the fusion protein according to the present invention for the treatment, removal, reduction or prevention of Gram-positive and/or Gram-negative bacterial contamination of foodstuff, of food processing equipment, of food processing plants, of surfaces coming into contact with foodstuff such as shelves and food deposit areas and in all other situations, where pathogenic, facultative pathogenic or other undesirable bacteria can potentially infest food material, of medical devices and of all kind of surfaces in hospitals and surgeries.

In particular, a fusion protein of the present invention may be used prophylactically as sanitizing agent. Said sanitizing agent may be used before or after surgery, or for example during hemodialysis. Moreover, premature infants and immunocompromised persons, or those subjects with need for prosthetic devices may be treated with a fusion protein according to the present invention. Said treatment may be either prophylactically or during acute infection. In the same context, nosocomial infections, especially by antibiotic resistant strains like *Pseudomonas aeruginosa* (FQRP), Acinetobacter species and Enterobacteriaceae such as *E.coli, Salmonella, Shigella, Citrobacter, Edwardsiella, Enterobacter, Hafnia, Klebsiella, Morganella, Proteus, Providencia, Serratia, Yersinia* species, *Methicillin-resistant Staphylococcus aureus, Vancomycin-resistant Enterococcus faecalis, Vancomycin-resistant Enterococcus faecium, Streptococcus pneumoniae, Propionibacterium acnes, multidrug-resistant Mycobacterium tuberculosis,* may be treated prophylactically or during acute phase with a fusion protein of the present invention. Therefore, a fusion protein according to the present invention may be used as a disinfectant also in combination with other ingredients useful in a disinfecting solution like detergents, tensids, solvents, antibiotics, lanthibiotics, or bacteriocins.

For the use of the fusion protein according to the present invention as a disinfectant e.g. in hospital, dental surgery, veterinary, kitchen or bathroom, the fusion protein can be prepared in a composition in form of e.g. a fluid, a powder, a gel, or an ingredient of a wet wipe or a disinfection sheet product. Said composition may additionally comprise suitable carrier, additives, diluting agents and/or excipients for its respective use and form, respectively, - but also agents that support the antimicrobial activity like EDTA or agents enhance the antimicrobial activity of the fusion proteins. The fusion protein may also be used with common disinfectant agents like, Alcohols, Aldehydes, Oxidizing agents, Phenolics, Quaternary ammonium compounds or UV-light. For disinfecting for example surfaces, objects and/or devices the fusion protein can be applied on said surfaces, objects and/or devices. The application may occur for instance by wetting the disinfecting composition with any means such as a cloth or rag, by spraying, pouring. The fusion proteins may be used in varying concentration depending on the respective application and the "reaction time" intended to obtain full antimicrobial activity.

In a further aspect the present invention relates to the use of the fusion protein according to the present invention as a food additive.

Another aspect of the present invention is that the invention can be used like a tool box, i.e. any peptide stretch and antimicrobial peptide disclosed above may be fused to any endolysin, autolysin or bacteriocin disclosed herein. Thus, it is possible to combine the respective peptide stretch, which enables the binding of the fusion protein to the respective bacteria and the endolysin, autolysin or bacteriocin, which inhibit the growth of the respective bacteria. Consequently, it is possible to construct a suitable fusion protein for any bacteria which should be eliminated.

Further scope of applicability of the present invention will become apparent from the detailed description given hereinafter, however, it should be understood that the detailed description and specific examples, while indicating preferred embodiments of the invention, are given by way of illustration only, since various changes and modifications within the spirit and scope of the invention will become apparent to those skilled in the art from this detailed description. It is to be understood that both the foregoing general description and the following detailed description are exemplary and explanatory only and are not restrictive of the invention, as claimed.

The following examples explain the present invention but are not considered to be limiting. Unless indicated differently, molecular biological standard methods were used, as e.g., described by Sambrock et al., 1989, Molecular Cloning: A Laboratory Manual, 2nd edition, Cold Spring Harbor Laboratory Press, Cold Spring Harbor, New York.

### Example 1: N-terminal fusion of antibacterial peptide tags to PK-modified endolysin (PK-OBPgpLys) of Pseudomonas putida phase OBP

PK-OBPgpLys, the modified endolysin variant of *P. putida* phage OBP with an N-terminal polycationic peptide according to SEQ ID NO: 23, was N-terminally fused to a set of natural antibacterial peptide tags (Table) to investigate its anti Gram-negative activity.

**Table 4: List of antibacterial peptide tags which were fused to PK-OBPgpLYS**

| Tag | Description + size | Amino acid sequence | Reference |
|---|---|---|---|
| α4-helix of T4-lysozyme | Amphiphatic helix (13 aa) | PNRAKRVITTFRT (SEQ ID NO: 68) | Matthews* |
| Pentapeptide (designed) | Hydrophobic (5 aa) | FFVAP (SEQ ID NO: 66) | Briers *et al.,* 2008 |
| Walmagh1 (designed) | Hydrophobic (18 aa) | GFFIPAVILPSIAFLIVP (SEQ ID NO: 65) | Walmagh1 |
| Walmagh2 (designed) | Amphiphatic helix (25 aa) | GKPGWLIKKALVFKKLIRRPLKRLA (SEQ ID NO:69) | Walmagh2 |
| Parasin 1 | Alpha-helical peptide (19 aa) | KGRGKQGGKVRAKAKTRSS (SEQ ID NO: 62) | Park, Y *et al.,* 1998** |
| Lycotoxin 1 | Amphiphatic helix (25 aa) | IWLTALKFLGKHAAKKLAKQQLSKL (SEQ ID NO:61) | Yan & Adams, 1988*** |

| | | | |
|---|---|---|---|
| ***Matthews, B.W. and Remington, S.J. (1974). The three dimensional structure of the lysozyme from bacteriophage T4. Proc. Natl. Acad. Sci. USA, 71: 4178-4182 ***In* Yup Park, Chan Bae Park, Mi Sun Kim, Sun Chang Kim (1998). Parasin I, an antimicrobial peptide derived from histone H2A in the cat¢sh, Parasilurus asotus. FEBS Letters 437 258-262 *****Yan, L and Adams, M.A. (1998). Lycotoxins, Antimicrobial Peptides from Venom of the Wolf Spider, Lycosa carolinensis J. Biol. Chem, 273:2059-2066*.* | | | |

### Methodology of tag modification of PK-OBPgpLys

To obtain the α4-, Walmagh 1-, Walmagh 2-, Lycotoxin1- and Parasinl-PK-OBPgplys ORF's, the corresponding peptide stretches (created by hybridization of primer pairs, see Table 5) were fused to the ORF which encodes for PK-OBPgpLYS using an adapted version of the Ligation Independent Cloning (LIC) technique. First, an unique Ec1136II restriction site (in bold) was inserted upstream of the PK modified OBPgpLys ORF by a tail PCR, with a specific designed 5' primer encoding the restriction site (5' GGAATGGGGAGCTCCTCCAAACGCAAGAAACGTAAGAAACGCAAAAAAAATAG CGAGAAT 3'; SEQ ID NO:119) and the standard OBPgpLys reverse primer (5' AACTATTCCGAGTGCTTTCTTTGT 3'; SEQ ID NO:120) on purified genomic DNA of phage OBP. This extended fragment was then ligated into the pEXP5CT/TOPO® expression vector (Invitrogen, Carlsbad, CA, USA) following the TA cloning protocol of the manufacturer. Pure plasmid was cut once (Ec1136II) and the hybridized peptide cassettes were inserted into the cut plasmid without a ligation step (LIC).

To fuse the PK-peptide stretch in front of the single tagged α4- and Pentapeptide-OBPgpLys ORF's a tail PCR with an extended 5' primer encoding this PK peptide stretch (for PK- α4-OBPgpLys the 5' primer is 5' ATGGGATCCAAACGCAAGAAACGTAAGAAACGCAAAGGCTCCTCCCCGAACCGT GCA 3'; SEQ ID NO:121) and for PK-Pentapeptide-OBPgpLys the 5' primer is 5' ATGGGATCCAAACGCAAGAAACGTAAGAAACGCAAAGCCTCCTCCTTCTTCGTA GCA 3' (SEQ ID NO:122) and the standard 3' OBPgpLys reverse primer was applied on purified pEXP5-CT/α4-OBPgpLys and pEXP5-CT/ Pentapeptide-OBPgpLys plasmid DNA, respectively. Obtained PCR fragments were then recloned in the pEXP5CT/TOPO® expression vector. Correct insertion of the fragments in the expression vector was verified by sequencing analysis before introducing the construct into a suitable Escherichia coli BL21(DE3)pLysS expression strain.

**Table 5: Used primer pairs for hybridization of antibacterial peptide tags for N-terminal fusion to ORF encoding PK-OBPgpLYS**

| **Tag** | **forward primer** | **reverse primer** |
|---|---|---|
| α4-helix of T4-lysozyme | 5'TTGGAATGGGGAGCCCGAACCGTGCAAAACGTGTA ATCA 3' SEQ ID NO: 99 | 5’GTTTGGAGGAGCCGGTACGGAAGGTGGTGATTACACG TT 3’ SEQ ID NO: 100 |
| Walmagh1 (designed) | 5'TTATGGGCTTCTTCATCCCGGCAGTAATCCTGCCCTC CA 3' SEQ ID NO: 101 | 5'GTTTGGAGGAGCCCGGTACGATCAGGAATGCGATGGA GGGCAGGAT 3' SEQ ID NO: 102 |
| Walmagh 2 (designed) | 5'TTATGGGCAAACCGGGCTGGCTGATCAAAAGGCACT GGTATTCAAGA 3' SEQ ID NO: 103 | 5'GTTTGGAGGAGCCTGCCAGTCTCTTCAGCGGACGACG GATCAGTTTCTTGAATACCA 3' SEQ ID NO: 104 |
| Lycotoxin1 | 5'GGAATGGGGAGCATCTGGCTGACCGCACTGAAATT CCTCGGCAAACACGCCGCAA 3' SEQ ID NO: 105 | 5'GTTTGGAGGAGCCCAGTTTGGATAATTGCTGTTTTGCC AGTTTCTTTGCGGCGTGTT 3' SEQ ID NO: 106 |
| Parasin1 | 5'TTGGAATGGGGAGCAAAGGCCGTGGCAAGCAGGG AGGCAAAGTACGTG 3' SEQ ID NO: 107 | 5'GTTTGGAGGAGCCTGAGGAACGGGTCTTTGCTTTTGCA CGTACTTTGC 3'  SEQ ID NO: 108 |

### Large scale recombinant expression of modified PK-OBPgpLYS fusion variants

Standard expression is performed in Lysogeny Broth (LB) in exponentially growing cells (OD₆₀₀ₙₘ= 0.6) induced with 1mM isopropyl-beta-D-thiogalactopyranoside. Expression parameters like temperature, time and expression strain varied on a protein specific basis in order to optimize the soluble expression levels of the modified endolysins (see Table 6).

For purification, cells from an expression culture (500-600 ml) are harvested (4500 rpm, 30 min, 4°C) and resuspended in 1/25 volumes of lysis buffer (10 mM imidazole, 20 mM NaH₂PO₄, 0.5 M NaCl, pH 7.4). This suspension is frozen/thawed three times prior to sonication (8 x 30 s, amplitude 40% on a Vibra CellTM, Sonics, Dandurry, CT, USA) and filtered through 0.45 and 0.22 µm Durapore membrane filters (Millipore, Billerica, MA, USA). Purification of the His-tagged fusion protein was performed by a one-step protocol employing Ni²⁺ - affinity chromatography (HisTrap HP 1 ml column, GE Healthcare, Buckinghamshire, UK) according to the manufacturer's instructions. The Ni²⁺ affinity chromatography is performed in 4 subsequent steps, all on room temperature:
1. *Equilibration* of the *Histrap HP 1 ml* column (GE Healthcare) with 10 column volumes of Washing Buffer (60 mM imidazole, 0.5 mM NaCl and 20 mM NaH₂PO₄-NaOH on pH 7.4) at a flow rate of 0.5 ml/min.
2. *Loading* of the total lysate (with wanted endolysin) on the *Histrap HP 1 ml* column at a flow rate of 0.5 ml/min.
3. Washing of the column with 15 column volumes of Washing Buffer at a flow rate of 1 ml/min.
4. Elution of bounded endolysin from the column with 10 column volumes of Elution Buffer (500 mM imidazole, 5 mM NaCl and 20 mM NaH₂P0₄-NaOH on pH 7.4) at a flow rate of 0.5 ml/min

The wash buffer included a low imidazole concentration which varied on protein specific base to ensure higher purity of the protein (see Table 6). The total yields of recombinant proteins per liter *E. coli* expression culture is also shown in Table 6. The values were determined by spectrophotometric measurement of the protein concentration and the total volume of the purified stock solution at a wavelength of 280 nm. Purified stock solutions were at least 95 % pure as determined visually on SDS-PAGE gels.

**Table 6: Expression parameters and obtained protein yields per liter expression culture of N-terminal modified PK-OBPgpLys variants**

| **Modified endolysin** | **Temperature/ time** | **Expression strain** | **Protein yield (in mg/l)** | **Imidazole (in mM)** |
|---|---|---|---|---|
| **α4-PK-OBPgpLys** | 16°C/overnight | *E.coli* BL21 RIL | 0.56 | 60 |
| **PK-α4-OBPgpLys** | 16°C/overnight | *E.coli* BL21 RIL | 7.61 | 60 |
| **Walmagh 1-PK-OBPgpLys** | 16°C/overnight | *E.coli* BL21 RIL | 0.81 | 60 |
| **Walmagh 2-PK-OBPgpLys** | 16°C/overnight | *E.coli* BL21 RIL | 1.78 | 60 |
| **Lycotoxin1-PK-OBPgpLys** | 16°C/overnight | E.coli BL21 RIL | 0.23 | 60 |
| **Parasin1-PK-OBPgpLys** | 16°C/overnight | *E.coli* BL21 RIL | 8.08 | 60 |
| **PK-pentapeptide-OBPgpLys** | 16°C/overnight | *E.coli BL21 RIL* | 45.09 | 60 |

### In vitro antibacterial activity and host range of modified PK-OBPgpLys fusion variants

Exponential growing Gram-negative bacterial cells (OD600nm = 0.6) were 100-fold diluted to a final density of about 10⁶ cells/ml in 5 mM HEPES pH 7.4 and incubated for 30 minutes at room temperature without shaking with the different modified OBPgpLYS variants (in Elution Buffer in an end concentration of 1500 nM). After incubation cell suspensions and as a control untreated bacterial cells without any added modified OBPgpLYS variant were diluted three times (respectively 10⁵-10⁴-10³ cells/ml) in 1x concentrated PBS buffer and 100 µl of each dilution was plated out on LB-medium. The bacteria colonies on the agar plates were counted after an overnight incubation on 37°C. Based on the counted cell numbers the antibacterial activity as the relative inactivation in logarithmic units (=log10N₀/Nᵢ with No = number of untreated cells (of the control) and Nᵢ = number of treated cells, both counted after incubation) is calculated (Table 7). The reduction in the number of bacteria colonies after treatment with fusion protein is indicative for an antimicrobial activity of the different modified OBPgpLYS variants, because the bacterial cells are lysed due to the treatment with the fusion proteins. All samples were replicated in threefold. Averages +/- standard deviations are represented.

**Table 7: In vitro antibacterial activity of different N-terminal modified PK-OBPgpLYS fusion variants on a range of exponential growing Gram-negative species without (A) and with (B) extra addition of the outer membrane permeabilizer EDTA (0.5 mM). Initial density is 10⁶ cells/ml and incubation proceeds for 30 minutes without shaking at RT. Average log reduction values are shown together with standard deviation (stdev).**

| **A** | | | | | | | | |
|---|---|---|---|---|---|---|---|---|
| **Fusionprotein** | ***P. aeruginosa* PAO1p** | | ***P. putida G1*** | | ***E. coli XL1 blue*** | | ***S. typhimurium LT**2* | |
| | average | stdev | average | stdev | average | stdev | average | stdev |
| **α4-PK-OBPgpLys** | 0.59 | 0.02 | 0.02 | 0.13 | 0.55 | 0.06 | 0.22 | 0.03 |
| **PK-α4-OBPgpLys** | 1.10 | 0.05 | 0.01 | 0.04 | 0.54 | 0.17 | 0.23 | 0.04 |
| **Walmagh1-PK-OBPgpLYS** | 0.20 | 0.09 | 0.29 | 0.22 | 1.46 | 0.08 | 0.12 | 0.03 |
| **Walmagh2-PK-OBPgplys** | 0.57 | 0.10 | 0.00 | 0.04 | 1.68 | 0.12 | 0.17 | 0.02 |
| **Lycotoxin1-PK-OBPgpLYS** | 0.08 | 0.05 | 0.56 | 0.10 | 0.41 | 0.04 | 0.17 | 0.07 |
| **Parasin1-PK-OBPgpLys PK-Pentapeptide-** | 0.75 | 0.03 | 0.16 | 0.04 | 0.70 | 0.09 | 0.27 | 0.11 |
| **OBPgplys** | 0.95 | 0.08 | 0.80 | 0.04 | 1.69 | 0.17 | 0.22 | 0.06 |
| **B** | | | | | | | | |
| **Fusionprotein** | ***P. aeruginosa* PAO1p** | | ***P. putida G1*** | | ***E. coli XL1 blue*** | | ***S. typhimurium* LT.2** | |
| | average | stdev | average | stdev | average | stdev | average | stdev |
| EDTA α4-PK-OBPgpLys + | 1.82 | +/-0.17 | 0.65 | +/-0.35 | 0.85 | +/-0.16 | 0.08 | +/-0.01 |
| EDTA PK-α4-OBPgpLys + | 5.23 | +/-0.03 | 2.98 | +/-0.16 | 0.58 | +/-0.13 | 0.35 | +/-0.11 |
| EDTA | 5.07 | +/-0.21 | 2.97 | +/-0.09 | 0.86 | +/-0.12 | 0.29 | +/-0.06 |
| Walmagh1-PK-OBPgpLYS+ EDTA | 3.66 | +/-0.12 | 3.22 | +/-0.21 | 1.70 | +/-0.07 | 0.17 | +/-0.07 |
| Walmagh2-PK-OBPgplys +EDTA | 3.91 | +/-0.09 | 2.60 | +/-0.12 | 1.86 | +/-0.10 | 0.25 | +/-0.04 |
| Lycotoxin1-PK-OBPgpLYS + EDTA | 3.73 | +/-0.37 | 2.26 | +/-0.10 | 0.90 | +/-0.24 | 0.29 | +/-0.06 |
| Parasin1-PK-OBPgpLys + EDTA | 4.92 | +/-0.03 | 2.32 | +/-0.08 | 1.00 | +/-0.14 | 0.28 | +/-0.05 |
| PK-Pentapept-OBPgpLys + EDTA | 4.00 | +/-0.06 | 3.24 | +/-0.26 | 2.12 | +/-0.33 | 0.42 | +/-0.05 |

### Example 2: Antimicrobial activity of fusion proteins of endolysin (PK-OBPgpLys) of Pseudomonas putida phage OBP with two peptide stretches in comparison to fusion protein of endolysin (PK-OBPgpLys) of Pseudomonas putida phage OBP with only one peptide stretch

### Methodology of production of OBPgpLys with one peptide stretch

Except for the pentapeptide tag, all antibacterial peptide tags were fused to the ORF which encodes for the OBPgpLYS derivative using an adapted version of the Ligation Independent Cloning (LIC) as e.g. described in Berrow *et al.* 2007. Here fore, an unique Ec1136II restriction site was inserted in front of the WT endolysin gene by a tail PCR with a specific designed 5' primer (5'-GGAATGGGGAGCTCCTCCAAAAATAGCGAGAAG-3'; SEQ ID NO: 123) and the standard OBPgpLys derivative reverse primer (5'-AACTATTCCGTGTGCTTTCTTTGT -3'; SEQ ID NO: 124) on pure genomic DNA of phage OBP. This extended fragment was then ligated in the pEXP5CT/TOPO® expression vector (Invitrogen, Carlsbad, CA, USA) by following the TA cloning protocol of the manufacturer. Pure plasmid was cutted once in an Ec1136II restriction digest and hybridized peptide cassettes (created by hybridization of primer pairs, see Table 8) were inserted into the cutted plasmid without a necessary ligation step (LIC). For the N-terminal pentapeptide tag fusion a tail PCR with an extended 5' primer which encodes for this pentapeptide (5'-ATGGGATCCTTCTTCGTAGCA CCGGGCTCCTCCAAAAATAGCGAGAAG-3'; SEQ ID NO: 125) and the standard OBPgpLys derivative reverse primer (5'-AACTATTCCGTGTGCTTTCTTTGT-3'; SEQ ID NO:126) was applied on phage OBP genomic DNA. Correct insertion of the fragments in the expression vector was verified by sequencing analysis before introducing the construct into a suitable Escherichia coli BL21(DE3)pLysS expression strain.

**Table 8: Used primer pairs for hybridization of antibacterial peptide tags to ORF encoding the OBPgpLys derivative**

| | Tag | forward primer | reverse primer | |
|---|---|---|---|---|
| | α4-helix of T4-lysozyme | 5'TTGGAATGGGGAGCCCGAACCGTGCAAAACG TGTAATCA 3' SEQ ID NO: 109 | 5'TATTTTTGGAGGAGCCGGTACGGAAGGTGGTGAT TACACGTT 3'; SEQ ID NO:110 | |
| | Walmagh1 (designed) | 5’TTATGGGCTTCTTCATCCCGGCAGTAATCCTGC CCTCCA 3’ SEQ ID NO:111 | 5'TATTTTTGGATCTGCCGCCCGGTACGATCAGGAAT GCGATGGAGGGCAGGATT 3'; SEQ ID NO:112 | |
| | Walmagh 2 (designed) | 5'TTATGGGCAAACCGGGCTGGCTGATCAAAAG GCACTGGTATTCAAGA 3' SEQ ID NO:113 | 5'TATTTTTGGATCTGCCGCCTGCCAGTCTCTTCAGC GGACGACGGATCAGTTTCTTGAATACCAG 3'; SEQ ID NO:114 | |
| | Parasin 1 | 5'TTGGAATGGGGAGCAAAGGCCGTGGCAAGCA GGGAGGCAAAGTACGTG 3' SEQ ID NO:117 | 5'TATTTTTGGAGGAGCCTGAGGAACGGGTCTTTGCT TTTGCACGTACTTTGC 3'; SEQ ID NO:118 | |
| | Lycotoxin 1 | 5'GGAATGGGGAGCATCTGGCTGACCGCACTGA AATTCCTCGGCAAACACGCCGCAA 3' SEQ ID NO:115 | 5’TATTTTTGGAGGAGCCCAGTTTGGATAATTGCTGT TTTGCCAGTTTCTTTGCGGCGTGTT 3´; SEQ ID NO:116 | |

The production of OBPgpLys with two peptide stretches is described above in Example 1. Moreover, the purification of the fusion proteins and the antimicrobial activity assay was performed as described already in Example 1. The results for the antimicrobial activity assay are given in Table 9.

**Table 9 In vitro antibacterial activity of OBPgpLYS fusion variants with one or two peptide stretches on a range of exponential growing Gram-negative species with extra addition of the outer membrane permeabilizer EDTA (0.5 mM). Initial density is 10⁶ cells/ml and incubation proceeds for 30 minutes without shaking at RT. Average log reduction values are shown together with standard deviation (stdev).**

| Fusion protein | *P. aeruginosa* PAO1p | *P. putida* G1 | *E. coli* Xl-1 1 | *Salmonella typhimurium* LT2 |
|---|---|---|---|---|
| α4-OBPgpLys | ++ | ++ | ++ | + |
| α4-PK-OBPgpLys | +++ | ++ | + | + |
| PK-α4-OBPgpLys | +++ | ++ | ++ | + |
| Pentapeptide-OBPgpLys | ++ | +++ | ++ | + |
| PK-Pentapeptide-OBPgplys | +++ | +++ | ++ | + |
| Walmagh2-OBPgpLys | ++ | ++ | ++ | + |
| Walmagh2-PK-OBPgplys | +++ | ++ | ++ | + |
| Parasin1-OBPgpLys | +++ | +++ | +++ | ++ |
| Parasin1-PK-OBPgpLys | +++ | ++ | ++ | + |
| Lycotoxin1-OBPgpLys | ++ | +++ | ++ | + |
| Lycotoxin1-PK-OBPgpLys | +++ | ++ | ++ | + |
| OBPgpLYS | + | + | + | + |

### Example 3: Antimicrobial activity of different peptide stretches

Exponential *P. aeruginosa* PAO1p cells (Burn wound isolate, Queen Astrid Hospital, Brussels; Pirnay JP et al. (2003), http://www.ncbi.nlm.nih.gov/pubmed/12624051?ordinalpos=3&itool=EntrezSystem2.PEntre z.Pubmed.Pubmed_ResultsPanel.Pubmed_DefaultReportPanel.Pubmed_RVDocSum J Clin Microbiol., 41(3):1192-1202) were washed in 10 mM HEPES, pH 7.4 and diluted 1:10 in 10 mM HEPES, 0.5 mM EDTA, pH 7.4. Bacteria were incubated at room temperature with the respective peptide, polycationic peptide named PK according to SEQ ID NO: 23, the polycationic peptide PK2 according to SEQ ID NO: 34 or the antimicrobial peptide named SMAP-29 according to SEQ ID NO: 45 in buffer (15 mM HEPES, 250 mM NaCl, 250 µM EDTA; pH 7.4) in the concentrations.0.3 µmol/L, 3 µmol/L,, 30 µmol/L and 150 µmol/L for the polycationic peptides and 3 µmol/L for the antimicrobial peptide. After 30 minutes cell suspensions were serial diluted (1:10) and plated on LB. Additionally, a negative control was plated using buffer (15 mM HEPES, 250 mM NaCl, 250 µM EDTA; pH 7.4). The bacteria colonies on the agar plates were counted after an overnight incubation at 37°C. Based on the counted cell numbers the antibacterial activity as the relative inactivation in logarithmic units (= log10N₀/Nᵢ with No = number of untreated cells (of the control) and Nᵢ = number of treated cells, both counted after incubation) is calculated. The reduction in the number of bacteria colonies after treatment with a peptide stretch is indicative for an antimicrobial activity of the peptide stretch, because the bacterial cells are lysed due to the treatment with the peptide stretch. All samples were replicated in four fold.

Both polycationic peptides PK and PK2 showed increasing antimicrobial activity against P. *aeruginosa* dependent on the used concentration of the peptide. PK2 showed higher antimicrobial activity against *P. aeruginosa* than PK. Moreover, PK2 used in a concentration of 150 µmol/L showed same antimicrobial activity against *P. aeruginosa* as SMAP-29 used in a concentration of 3 µmol/L. For both PK and PK2 more peptide has to be used to achieve the same antimicrobial activity against *P. aeruginosa* as SMAP-29. Usage of equimolar concentrations of PK or PK2 (3 µM/L) show no or only minor antimicrobial effects compared to AMP or Artilysin.

## Claims

1. A fusion protein composed of an enzyme having the activity of degrading the cell wall of Gram-negative bacteria and/or Gram-positive bacteria and at least two peptide stretches fused to the enzyme at the N- and/or C-terminus, wherein the peptide stretches are distinct and selected from the group of synthetic amphiphatic peptide, synthetic cationic peptide, synthetic polycationic peptide, synthetic hydrophobic peptide, or naturally occurring antimicrobial peptide, like sushi peptide and defensin.

2. The fusion protein according to claim 1, wherein the enzyme is an endolysin, autolysin or bacteriocin.

3. The fusion protein according to claim 1 or 2, wherein the synthetic peptide or naturally occurring peptide has a length of 6 to 39 amino acid residues.

4. The fusion protein according to any of the preceding claims, wherein the Gram-negative bacteria are selected from the group consisting of
Enterobacteriaceae,
in particular Escherichia, Salmonella, Shigella, Citrobacter, Edwardsiella, Enterobacter, Hafnia, Klebsiella, Morganella, Proteus, Providencia, Serratia, and Yersinia,
Pseudomonadaceae,
in particular Pseudomonas, Burkholderia, Stenotrophomonas, Shewanella, Sphingomonas and Comamonas,
Neisseria, Moraxella, Vibrio, Aeromonas, Brucella, Francisella, Bordetella, Legionella, Bartonella, Coxiella, Haemophilus, Pasteurella, Mannheimia, Actinobacillus, Gardnerella, Spirochaetaceae,
in particular Treponema and Borrelia,
Leptospiraceae, Campylobacter, Helicobacter, Spirillum, Streptobacillus, Bacteroidaceae,
in particular Bacteroides, Fusobacterium, Prevotella and Porphyromonas, and Acinetobacter,
in particular A. baumanii; and
wherein the Gram-positive bacteria are selected from the group consisting of *Listeria monocytogenes, Staphylococcus aureus, Enterococcus faecalis, Enterococcus faecium, Streptococcus pneumoniae, Streptococcus pyogenes, Streptococcus mutans, Streptococcus equi, Clostridium difficile, Clostridium botulinum, Clostridium tetani, Clostridium pefringens, Bacillus anthracis, Bacillus cereus, Propionibacterium acnes, Mycobacterium avium, Mycobacterium tuberculosis, Corynebacterium diphteriae, Mycoplasma pneumoniae, Actinomyces.*

5. The fusion protein according to any of the preceding claims, wherein the enzyme exhibits an amino acid sequence according to SEQ ID NO: 1 to 18.

6. The fusion protein according to any of the preceding claims, wherein the antimicrobial peptide exhibits an amino acid sequence according to SEQ ID NO: 44 to 62 and 78 to 98, or wherein the sushi peptide exhibits an amino acid sequence according to SEQ ID NO: 63; or wherein the hydrophobic peptide exhibits an amino acid sequence according to SEQ ID NO: 65 and/or 66; or wherein the amphiphatic peptide exhibits an amino acid sequence according to SEQ ID NO: 64, 68 and/or 69.

7. The fusion protein according to any of the preceding claims, wherein said fusion protein exhibits an amino acid sequence according to SEQ ID NO: 70 to 77 and 127 to 135.

8. An isolated nucleic acid molecule encoding a fusion protein according to any of claims 1 to 7.

9. A vector comprising the nucleic acid molecule according to claim 8.

10. A host cell comprising the nucleic acid molecule according to claim 8 or the vector according to claim 9.

11. A host cell according to claim 10, wherein the cell is a bacterial cell or a yeast cell.

12. The fusion protein according to any one of claims 1 to 7 for use as a medicament, diagnostic means, cosmetic substance, a disinfectant or food additive.

13. The use of the fusion protein according to any one of claims 1 to 7 for the treatment or prevention of Gram-negative and/or Gram-positive bacterial contamination of foodstuff, of food processing equipment, of food processing plants, of surfaces coming into contact with foodstuff, of medical devices, of surfaces in hospitals and surgeries.

14. The use of the fusion protein according to any one of claims 1 to 7 as a diagnostic means in medicinal, food or feed or environmental diagnostics.
